(19) Europäisches Patentamt
European Patent Office
Office européen des brevets

(11) EP 3 294 747 B1

(12) **EUROPEAN PATENT SPECIFICATION**

(45) Date of publication and mention of the grant of the patent:
**24.02.2021 Bulletin 2021/08**

(21) Application number: **15823770.1**

(22) Date of filing: **02.12.2015**

(51) Int Cl.:
*C07F 15/00* (2006.01)
*B01J 31/28* (2006.01)
*C08F 32/04* (2006.01)
*C08F 32/08* (2006.01)
*C08F 4/00* (2006.01)
*B01J 31/22* (2006.01)
*C07C 6/04* (2006.01)
*C07C 67/333* (2006.01)
*C08G 61/08* (2006.01)

(86) International application number:
**PCT/IB2015/059287**

(87) International publication number:
**WO 2016/092424 (16.06.2016 Gazette 2016/24)**

(54) **RUTHENIUM COMPLEXES, METHOD OF PRODUCING THEM, AND THEIR USE**

RUTHENIUMKOMPLEXE, VERFAHREN ZUR HERSTELLUNG DAVON UND DEREN VERWENDUNG

COMPLEXES DE RUTHÉNIUM, LEUR PROCÉDÉ DE PRÉPARATION, ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2014 PL 41032914**

(43) Date of publication of application:
**21.03.2018 Bulletin 2018/12**

(73) Proprietor: **Uniwersytet Warszawski**
**00-927 Warszawa (PL)**

(72) Inventors:
- **GRELA, Karol**
  **01-471 Warszawa (PL)**
- **SMOLE , Micha**
  **42-242 R dziny (PL)**

(74) Representative: **Dargiewicz, Joanna et al**
**JD&P Patent Attorneys**
**Joanna Dargiewicz & Partners**
**Ul. Mysliborska 93A/50**
**03-185 Warszawa (PL)**

(56) References cited:
**US-A1- 2003 144 437**

- **ANNA KAJETANOWICZ ET AL: "Metathesis of renewable raw materials-influence of ligands in the indenylidene type catalysts on self-metathesis of methyl oleate and cross-metathesis of methyl oleate with (Z)-2-butene-1,4-diol diacetate", GREEN CHEMISTRY, vol. 16, no. 3, 10 December 2013 (2013-12-10), page 1579, XP055261184, GB ISSN: 1463-9262, DOI: 10.1039/c3gc42194e -& Anna Kajetanowicz ET AL: "Supporting Information", , 10 December 2013 (2013-12-10), XP055261254, Retrieved from the Internet: URL:http://www.rsc.org/suppdata/gc/c3/c3gc42194e/c3gc42194e.pdf [retrieved on 2016-03-29]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 294 747 B1

## Description

The present invention relates to novel ruthenium complexes, a method of producing them, and their use as precatalysts and/or catalysts in the olefin metathesis reactions. This invention is applicable in the wide understood organic synthesis reactions using olefin cross-metathesis (CM), ring closing metathesis (RCM), ring closure enyne metathesis (RCEYM), diastereo ring-rearrangement metathesis (DRRM), ring opening metathesis polymerization (ROMP), and acyclic diene metathesis polymerization (ADMET).

In recent years, applications of olefin metathesis in organic synthesis have achieved significant progress [Grela, K.; edt. Olefin Metathesis: Theory and Practice, 2014, John Wiley & Sons, Inc., 608 pages].

The state of the art encompasses several ruthenium carbene complexes acting as a precatalysts and/or catalysts, both possess a high activity in various types of olefin metathesis reactions as well as have a broad functional group tolerance. This combination of properties determines the usefulness of such precatalysts and/or catalysts in organic synthesis. Well-known applications of ruthenium complexes in organic synthesis include Grubbs' complexes (**Gru-I, Gru-II, Gru-III** and **Gru-III'**), Hoveyda complexes (**Hov-I** and **Hov-II**), and indenylidene complexes (**Ind-I, Ind-II** and **Ind-III**), 1$^{st}$, 2$^{nd}$ and 3$^{rd}$ generation [Grela et al.. Chem. Rev. 2009, 109, 3708-3742; Grubbs et al.. Chem. Rev. 2010, 110, 1746-1787; Lemcoff, et al.. Dalton Trans. 2012, 41, 32-43; A. Hoveyda, J. Org. Chem. 2014, 79, 4763-4792; Nolan et al.. Chem. Commun. 2014, 50, 10355-10375].

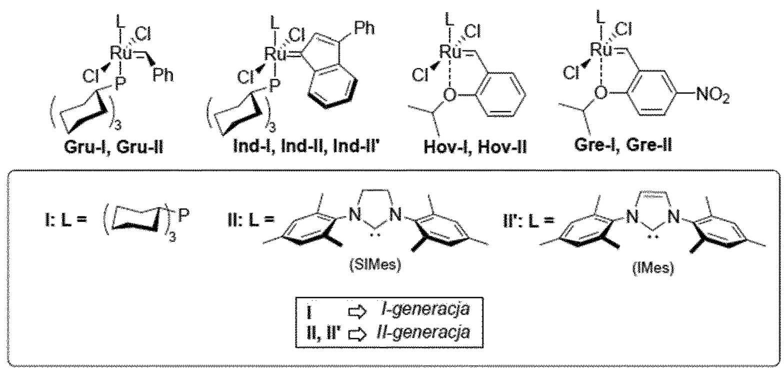


From the practical point of view, application of olefin metathesis reaction, particularly on an industrial scale, highly demands that these ruthenium complexes exhibit a prolonged stability at elevated temperatures and may be stored, purified and applied in commercially available solvents without need of inert gas atmosphere. In some applications, it is important that depending on the reaction conditions such a latent precatalysts and/or catalysts have a delayed initiation and after initiation, they promote the reaction in a controlled manner.

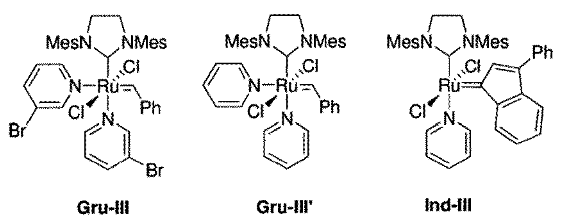


Mes= 2,4,6-trimetylofenyl

There are many known ruthenium complexes active in olefin metathesis [for examples, see review articles: Chem. Rev. 2010, 110, 1746; Chem. Rev. 2009, 109, 3708]. It has been demonstrated that benzylidene ruthenium complexes without phosphine ligands, but having two pyridine ligands: **Gru-III** i **Gru-III'** are characterized by much higher rate of initiation and propagation in many metathesis reactions, including ring opening metathesis polymerization (ROMP). Similarly, European researchers have introduced indenylidene complex **Ind-III,** that contains only one molecule of pyridine, as a convenient precatalyst for olefin metathesis also having a high activity in ROMP polymerization.

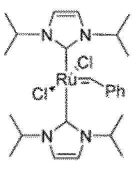

bis-1

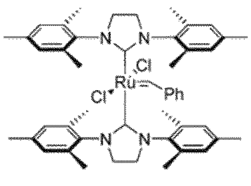

bis-2

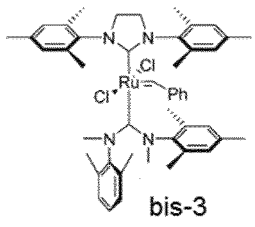

bis-3

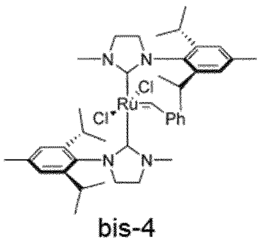

bis-4

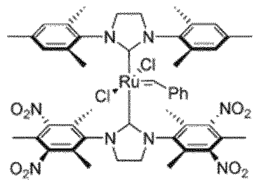

bis-5

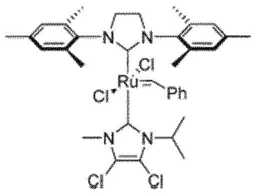

bis-6

In the state of the art, there are also known precatalysts and/or catalysts for olefin metathesis reactions in which the two neutral ligands are *N*-heterocyclic carbenes. Ruthenium benzylidene complexes bearing two identical symmetrical NHC ligands have been obtained for the first time by Herrmann et al.. in 1998 [EP1087838B1; Angew. Chem. Int. Ed. 1998, 37, 2490-2493; Organometallics 2003, 22, 1986-1988]. Subsequently, ruthenium benzylidene complexes active in olefin metathesis bearing two identical unsymmetrical NHC ligands, that possess two different substituents on the nitrogen atoms [Organometallics 2007, 26, 1052-1056; Organometallics 2012, 31, 2476-2481]. In the art, there are also known benzylidene precatalysts and/or catalysts containing two NHC ligands inseparably connected via hydrocarbon or aromatic bridge [J. Organomet. Chem. 2006, 691, 5204-5210; Organometallics 2012, 31, 580-587]. Next, there are also known benzylidene precatalysts and/or catalysts bearing two different NHC ligands *[*Angew. Chem. Int. Ed. 2009, 48, 5191-5194; Organometallics 2009, 29, 250-256; Chem. Eur. J. 2010, 16, 3983-3993; J. Organomet. Chem. 2010, 695, 2418-2422]. In the art, there are also known indenylidene ruthenium complexes bearing two different NHC ligands [Organometallics 2010, 29, 2761-2766; Organometallics 2010, 29, 3007-3011; J. Organomet. Chem. 2012, 710, 68-74].

In the state of the art, there are known Grubbs and Hoveyda type ruthenium complexes active in olefin metathesis bearing unsymmetrical *N,N'*-disubstituted NHC ligands [Organometallics 2006, 25, 25-28; Adv. Synth. Catal. 2007, 349, 1692-1700; Organometallics 2007, 26, 2469-2472; Chem. Eur. J. 2008, 14, 7545-75561. Recent reports in the literature have revealed unprecedented *Z*-selectivity in olefin cross metathesis performed in the presence of Hoveyda type complexes bearing unsymmetrical *N,N'*-disubstituted NHC ligands *[*J. Am. Chem. Soc. 2011, 133, 8525-8527], and high selectivity in ethenolysis reactions [J. Am. Chem. Soc. 2011, 133, 7490-7496].

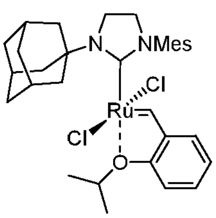

Cat 1

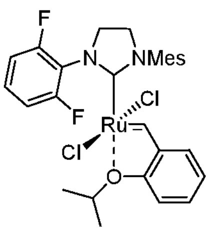

Cat 2

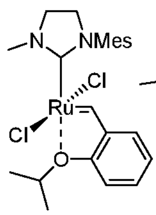

Cat 3

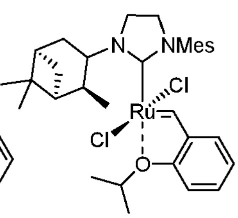

Cat 4

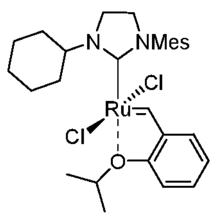

Cat 5

Anna Kajetanowicz et al. in "Metathesis of renewable raw materials-influence of ligands in the indenylidene type catalysts on self-metathesis of methyl oleate and cross-metathesis of methyl oleate with (Z)-2-butene-1,4-diol acetate", Green Chemistry, vol. 16, no. 3, 10 December 2013 (2013-12-10), page 1579, GB, ISSN: 1463-9262, DOI: 10.1039/c3gc42194e & Anna Kajetanowicz et al. "Supporting information", 10 December 2013, studied the influence of different N-heterocyclic carbene (NHC) and other neutral ligands on outcome of the cross-metathesis reaction of methyl oleate with (*Z*)-2-butene-1,4-diol acetate for a series of indenylidene type Ru catalysts.

Bell Andrew at al. in US2013/144437 A1 discloses a process for converting a less active or slower to initiate catalyst system to a higher activity catalyst system wherein the process comprises contacting a protected N-heterocyclic carbene with a metathesis catalyst and an olefin in the presence of energy.

A long, complicated, and costly synthesis pathways of ruthenium complexes without phosphine and having

one or two NHC ligands that require the use of expensive reagents such as silver salts, strong bases, dry and deoxygenated solvents; they all represent obstacles for the synthesis of ruthenium complexes, and the widespread usage of olefin metathesis in the industry. In addition, there are only a few known complexes that are stable and resistant to high temperatures, as well as such precatalysts that its activation leading to catalysts may be controlled during the reaction at elevated temperature. Research for new possibly application of olefin metathesis reaction in the industry is an important cause for continuous search of new ruthenium complexes having modified catalytic properties. In particular, ruthenium complexes with controlled activity during the reaction at elevated temperature.

Unexpectedly, it was found that novel ruthenium complexes defined by the general formula 1:

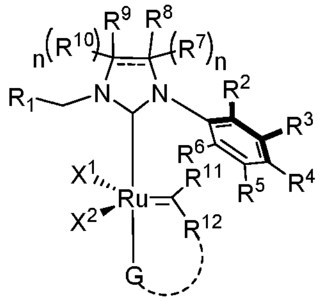

**1**

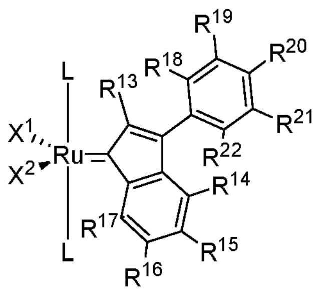

**1a**

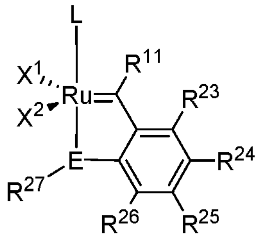

**1b**

L = 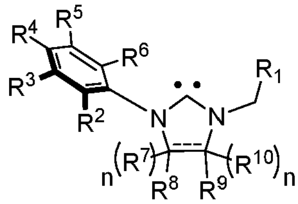

having in their structure indenylidene alkylidene, bearing two neutral NHC carbene ligands lacking phosphine ligand type, or Hoveyda type having one NHC ligand and chelating benzylidene typical for olefin metathesis precatalyst, that exhibits a significantly higher thermal stability both in solution as well as in the solid state, and at the same time effectively catalyze metathesis reactions at the elevated temperatures.

Unexpectedly, it has been found that catalysts represented by the structure of formula 1 are activated in a slow and controlled manner at high temperatures, as compared to commercially available ruthenium complexes. Complexes of general Formula 1 combine features of the "latent catalyst" and high stability features of II-generation catalysts.

In the laboratory scale, the reaction rate is of moderate importance for the overall process, whereas in the industrial scale when reactions are performed in large volume, the reaction rate is a key parameter. Delayed or slowed down initiation of olefin metathesis precatalysts is a desirable feature of a ruthenium complexes, because it allows preparation of substrate-precatalyst mixture at room temperature, controlled handling of such a mixture, its transport and introduction into the reactor or the mold, where the reaction takes place. The chemical reaction is started only at the certain high temperature, which initiate precatalyst. This is critical feature for the use of these precatalysts in organic synthesis in the large scale. Precatalysts described by the invention prevent the uncontrolled chemical reaction, that increases the safety of industrial installations and employees. Valid control of the reaction allows easy delivery and/or receiving of energy that is required for the reaction performance, and the discharge of resulting gas products.

Unexpectedly, it was found that the synthesis of indenylidene ruthenium complexes bearing two NHC ligands represented by Formula 1 is considerably shorter, less expensive and easier to apply on an industrial scale than the prior art syntheses of ruthenium precatalysts and/or catalysts, because they does not require the use of a silver salt, and it involves a one-step modification of commercially available complex Umicore **M1™** (Ind-I).

Complexes according to the invention, represented by general Formula 1, find usage in a wide range of reaction. These complexes may performed with a good result either numerous ring-closing metathesis reactions, homometathesis, enyne metathesis (RCEYM), cross-metathesis, including ethenolysis, diastereo ring rearrangements metathesis (DRRM), as well as ring opening polymerization (ROMP), and acyclic diene polymerization (ADMET).

The subject of the present invention is compound defined by the general Formula **1a**

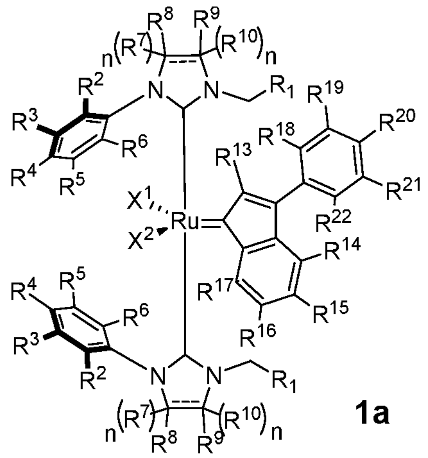

**1a**

in which:

$X^1$ and $X^2$ are an anionic ligand independently selected from group encompassing halogen anion, the groups - CN, -SCN, -OR', -SR', -O(C=O)R', -O($SO_2$)R', and -OSi(R')$_3$, where R' is $C_1$-$C_{12}$ alkyl, $C_3$-$C_{12}$ cycloalkyl, $C_2$-$C_{12}$ alkenyl, or $C_5$-$C_{20}$ aryl, which optionally may be substituted with at least one $C_1$-$C_{12}$ alkyl, $C_1$-$C_{12}$ perfluoroalkyl,

$C_1$-$C_{12}$ alkoxyl, $C_5$-$C_{24}$ aryloxyl, $C_5$-$C_{20}$ heteroaryloxyl, or halogen atom;

$R^1$ is heteroaryl group;

$R^2$, $R^3$, $R^4$, $R^5$, and $R^6$ independently of one another are hydrogen atom, $C_1$-$C_{25}$ alkyl group, $C_1$-$C_{25}$ alkoxyl group, or $C_2$-$C_{25}$ alkenyl group, wherein the groups $R^2$, $R^3$, $R^4$, $R^5$ and $R^6$ may be mutually connected into ring that form substituted or unsubstituted $C_4$-$C_{10}$ cyclic or $C_4$-$C_{12}$ polycyclic structure;

$R^7$, $R^8$, $R^9$, and $R^{10}$ independently of one another are hydrogen atom, or $C_1$-$C_{25}$ alkyl group, $R^7$ and/or $R^8$ may be connected with $R^9$ and/or $R^{10}$, to form a cycle;

n is 0 or 1;

$R^{13}$, $R^{14}$, $R^{15}$, $R^{16}$, $R^{17}$, $R^{18}$, $R^{19}$, $R^{20}$, $R^{21}$, and $R^{22}$ independently of one another are hydrogen atom, halogen atom, $C_1$-$C_{25}$ alkyl, $C_1$-$C_{25}$ alkylamino, $C_1$-$C_{25}$ alkylammonium, $C_1$-$C_{25}$ perfluoroalkyl, $C_2$-$C_{25}$ alkenyl, $C_3$-$C_7$ cycloalkyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryl, $C_5$-$C_{20}$ heteroaryl, $C_3$-$C_{12}$ heterocycle, 3-12 membered heterocycle, a thioether (-SR'), an ester (-COOR'), an amide (-CONR'$_2$), a sulphone (-$SO_2$R'), a sulphonamide (-$SO_2$NR'$_2$), or a ketone (-COR'), in which R' group is $C_1$-$C_5$ alkyl, $C_1$-$C_5$ perfluoroalkyl, $C_5$-$C_{25}$ aryl, or $C_5$-$C_{25}$ perfluoroaryl.

In preferable embodiment of the present invention, compound **1a** has a structure defined by the general Formula **1a**
in which:

$R^1$ is heteroaryl selected from a group encompassing furan, thiophene, benzothiophene, benzofuran;

$R^2$, $R^3$, $R^4$, $R^5$, and $R^6$ are independently hydrogen atom, methyl, isopropyl, halogen atom;

$R^7$, $R^8$, $R^9$, and $R^{10}$ are independently hydrogen atom or methyl group;

n is 0 or 1;

$X^1$, and $X^2$ are halogen atom;

$R^{13}$, $R^{14}$, $R^{15}$, $R^{16}$, $R^{17}$, $R^{18}$, $R^{19}$, $R^{20}$, $R^{21}$, and $R^{22}$ are independently hydrogen atom, halogen atom, $C_1$-$C_{25}$ alkyl, $C_1$-$C_{25}$ perfluoroalkyl, $C_1$-$C_{25}$ alkoxyl.

Another subject of the present invention is compound defined by the general Formula **1b**

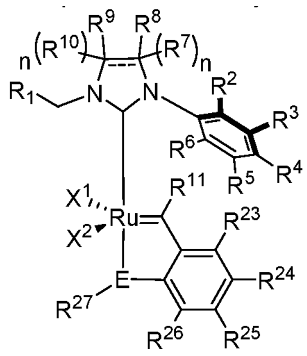

**1b**

in which:

$X^1$ and $X^2$ are an anionic ligand independently selected from group encompassing halogen anion, the groups - CN, -SCN, -OR', -SR', -O(C=O)R', -O($SO_2$)R', and -OSi(R')$_3$, where R' is $C_1$-$C_{12}$ alkyl, $C_3$-$C_{12}$ cycloalkyl, $C_2$-$C_{12}$ alkenyl, or $C_5$-$C_{20}$ aryl, which optionally may be substituted with at least one $C_1$-$C_{12}$ alkyl, $C_1$-$C_{12}$ perfluoroalkyl,

$C_1$-$C_{12}$ alkoxyl, $C_5$-$C_{24}$ aryloxyl, $C_5$-$C_{20}$ heteroaryloxyl, or halogen atom;

$R^1$ is heteroaryl group;

$R^2$, $R^3$, $R^4$, $R^5$, and $R^6$ independently of one another are hydrogen atom, $C_1$-$C_{25}$ alkyl group, $C_1$-$C_{25}$ alkoxyl group, or $C_2$-$C_{25}$ alkenyl group, wherein the groups $R^2$, $R^3$, $R^4$, $R^5$ and $R^6$ may be mutually connected into ring that form substituted or unsubstituted $C_4$-$C_{10}$ cyclic or $C_4$-$C_{12}$ polycyclic structure;

$R^7$, $R^8$, $R^9$, and $R^{10}$ independently of one another are hydrogen atom, or $C_1$-$C_{25}$ alkyl group, $R^7$ and/or $R^8$ may be connected with $R^9$ and/or $R^{10}$, to form a cycle;

n is 0 or 1;

$R^{11}$ is hydrogen atom;

$R^{23}$ , $R^{24}$, $R^{25}$, and $R^{26}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro (-$NO_2$) group, cynaide (-CN) group, carboxyl (-COOH) group, an ester (-COOR') group, an amide (-CONR'$_2$) group, an imide (-CONR'COR') group, an amine (-NR'$_2$) group, an ammonium (-$N^+$R'$_3$) group, an amide (-NR'COR') group, a sulphonamide (-NR'$SO_2$R') group, a sulphone (-$SO_2$R') group, a formyl (-CHO) group, a sulphonamide (-$SO_2$NR'$_2$) group, a ketone (-COR') group, in which substituent R' denotes: a $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, whereas $R^{23}$, $R^{24}$, $R^{25}$, and $R^{26}$ are preferentially hydrogen atom;

$R^{27}$ is hydrogen atom, $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, Cyanide (-CN) group, carboxyl (-COOH) group, an ester (-COOR') group, an amide (-CONR'$_2$) group, a sulphone (-$SO_2$R') group, a formyl (-CHO) group, a sulphonamide (-$SO_2$NR'$_2$) group, a ketone (-COR') group, in which substituent R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

E is heteroatom selected from atoms encompassing oxygen, sulphur, nitrogen, phosphorus.

In preferable embodiment of the present invention, compound **1b** has a structure defined by the general Formula **1b**
in which:

$R^1$ is heteroaryl selected from a group encompassing furan, thiophene, benzothiophene, benzofuran;

$R^2$, $R^3$, $R^4$, $R^5$, and $R^6$ are independently hydrogen atom, methyl, isopropyl, halogen atom;

$R^7$, $R^8$, $R^9$, and $R^{10}$ are independently hydrogen atom or methyl;

n is 0 or 1;

$X^1$, and $X^2$ are halogen atom;

$R^{11}$ is a hydrogen atom;

$R^{23}$ , $R^{24}$, $R^{25}$, and $R^{26}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro ($-NO_2$) group, cyanide (-CN) group, carboxyl (-COOH) group, an ester (-COOR') group, an amide ($-CONR'_2$) group, an imide (-CONR'COR') group, an amine ($-NR'_2$) group, an ammonium ($-N^+R'_3$) group, an amide (-NR'COR') group, a sulphonamide ($-NR'SO_2R'$) group, a sulphone ($-SO_2R'$) group, a formyl (-CHO) group, a sulphonamide ($-SO_2NR'_2$) group, a ketone (-COR') group, in which substituents R' are $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, 3-12 membered heterocycle, wherein $R^{23}$, $R^{24}$, $R^{25}$, and $R^{26}$ are preferably hydrogen atom;

$R^{27}$ is $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, Cyanide (-CN) group, carboxyl (-COOH) group, an ester (-COOR') group, an amide ($-CONR'_2$) group, a sulphone ($-SO_2R'$) group, a formyl (-CHO) group, a sulphonamide ($-SO_2NR'_2$) group, a ketone (-COR') group, in which substituent R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

E is heteroatom selected from atoms encompassing oxygen or sulphur atom.

In another preferable embodiment of the present invention, its subject is a compound having a structure defined by the general Formula selected from among **1aa, 1ab, 1ac, 1ba,** and **1bb:**

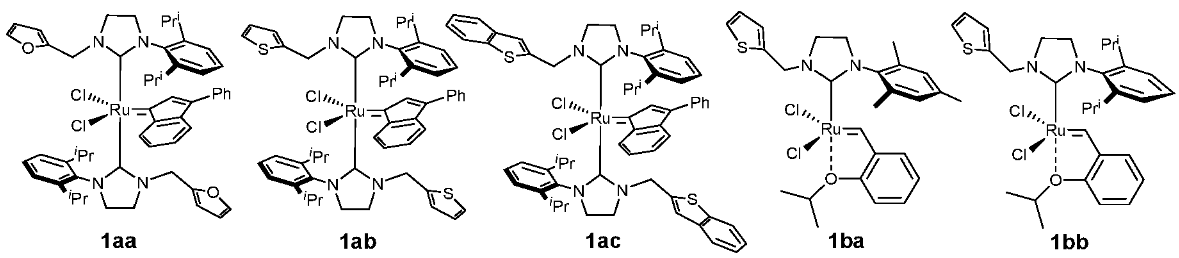

**1aa** **1ab** **1ac** **1ba** **1bb**

The subject of present invention is also a method of producing compound defined by the general Formula 1a or 1b, which encompasses reactions of alkylidene ruthenium complex defined by general Formula 2

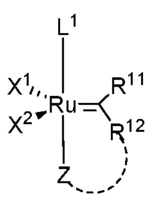

**2**

in which:

$R^{11}$, and $R^{12}$ are independently hydrogen atom, halogen atom, $C_1$-$C_{25}$ alkyl, $C_1$-$C_{25}$ perfluoroalkyl, $C_2$-$C_{25}$ alkene,

$C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle,

wherein groups $R^{11}$ and $R^{12}$ may be mutually connected into ring selected from among $C_3$-$C_7$ cycloalkyl, $C_3$-$C_{25}$ cycloalkenyl, $C_3$-$C_{25}$ cycloalkynyl, $C_5$-$C_{24}$ aryl, $C_5$-$C_{20}$ heteroaryl, $C_5$-$C_{24}$ perfluoroaryl, which may be independently substituted by at least one and/or more substituents selected from group encompassing hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocyle;

wherein groups $R^{11}$, and $R^{12}$ are preferentially hydrogen atom or aryl independently substituted by an ether group (-OR'), a thioether group (-SR'), a sulphoxide group (-S(O)R'), a sulphonium group ($-S^+R'_2$), a sulphone group ($-SO_2R'$), a sulphonamide group ($-SO_2NR'_2$), an amino group ($-NR'_2$), an ammonium group ($-N^+R'_3$), a nitro group ($-NO_2$), cyanide group (-CN), a phosphonate group ($-P(O)(OR)_2$), a phosphinate group (-P(O)R'(OR')), a phosphonite group ($-P(OR')_2$), a phosphine group ($-PR'_2$), a phosphine oxide group ($-P(O)R'_2$), a phosphonium group ($-P^+R'_3$), carboxyl group (-COOH), an ester group (-COOR'), an amide group ($-CONR'_2$), a formyl group (-CHO), a ketone group (-COR'), where each R' independently is $C_1$-$C_5$ alkyl, $C_1$-$C_5$ perfluoroalkyl, $C_5$-$C_{24}$ aryl, $C_7$-$C_{24}$ aralkyl, $C_5$-$C_{24}$ perfluoroaryl;

$L^1$ is neutral ligand selected from group encompassing pyridine or substituted pyridine, $P(R')_3$, $P(OR')_3$, $O(R')_2$, $N(R')_3$, in which each R' is independently a $C_1$-$C_{12}$ alkyl, $C_3$-$C_{12}$ cycloalkyl, $C_5$-$C_{20}$ aryl, $C_7$-$C_{24}$ aralkyl, $C_5$-$C_{24}$ perfluoroaryl, a 5-12 membered heteroaryl;

Z is selected from such as ligand $L^1$, and then substituents $R^{11}$, and $R^{12}$ are mutually connected to form ring selected from group encompassing a $C_3$-$C_7$ cycloalkyl, $C_3$-$C_{25}$ cycloalkenyl, $C_3$-$C_{25}$ cycloalkynyl, $C_5$-$C_{24}$ aryl, $C_5$-$C_{20}$ heteroaryl, $C_5$-$C_{24}$ perfluoroaryl, 3-12 membered heterocycle, which may be independently substituted by at least one substituent selected from group encompassing hydrogen atom, a halogen, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle, and then dashed line between Z and $R^{12}$ does not mean a chemical bond; heteroatom selected from group encompassing oxygen, nitrogen, sulphur, or phosphorus atom, optionally substituted by group selected from such as hydrogen atom, $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, cyanide (-CN) group, carboxyl (-COOH) group, an ester (-COOR') group, an amide ($-CONR'_2$) group, a sulphone ($-SO_2R'$) group, a formyl (-CHO) group, a sulphonamide ($-SO_2NR'_2$) group, a ketone (-COR') group, in which group R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, and then dashed line between heteroatom and $R^{12}$ means a chemical bond between a heteroatom and $R^{12}$ substituent, which is aryl optionally substituted by 1, 2, 3, or 4 substituents independently selected from group encompassing hydrogen atom, halogen atom, $C_1$-$C_{25}$ alkyl, $C_1$-$C_{25}$ perfluoroalkyl, $C_2$-$C_{25}$ alken, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro ($-NO_2$) group, cyanide (-CN) group, carboxyl (-COOH) group, an ester (-COOR') group, an amide ($-CONR'_2$) group, an imide (-CONR'COR') group, an amine ($-NR'_2$) group, an ammonium ($-N^+R'_3$) group, an amide (-NR'COR') group, a sulphonamide ($-NR'SO_2R'$) group, a sulphone ($-SO_2R'$) group, a formyl (-CHO) group, a sulphonamide ($-SO_2NR'_2$) group, a ketone (-COR') group, in which R' has the following meaning: a $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

$X^1$, and $X^2$ are an anionic ligand selected independently from group encompassing halide anions, a -CN, - SCN, -OR', -SR', -O(C=O)R', $-O(SO_2)R'$, $-OSi(R')_3$ group, where R' is $C_1$-$C_{12}$ alkyl, $C_3$-$C_{12}$ cycloalkyl, $C_2$-$C_{12}$ alkenyl, or $C_5$-$C_{20}$ aryl, which is optionally substituted at least one $C_1$-$C_{12}$ alkyl, $C_1$-$C_{12}$ perfluoroalkyl, $C_1$-$C_{12}$ alkoxyl, $C_5$-$C_{24}$ aryloxyl, $C_5$-$C_{20}$ heteroaryloxyl or halogen atom;

is subjected to a reaction with a carbene defined by the general Formula **3a**

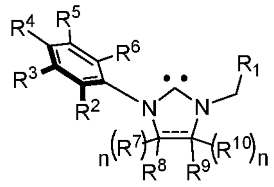

**3a**

in which:

$R^1$ is heteroaryl group;

$R^2$, $R^3$, $R^4$, $R^5$, and $R^6$ are independently hydrogen atom, $C_1$-$C_{25}$ alkyl, $C_1$-$C_{25}$ alkoxyl, or alkenyl $C_2$-$C_{25}$,

wherein substituents $R^2$, $R^3$, $R^4$, $R^5$, and $R^6$ may be mutually connected into ring that form substituted or

unsubstituted $C_4$-$C_{10}$ cyclic or $C_4$-$C_{12}$ polycyclic structure;

$R^7$, $R^8$, $R^9$, and $R^{10}$ are independently hydrogen atom or a $C_1$-$C_{25}$ alkyl group, $R^7$ and/or $R^8$ may be mutually connected with $R^9$ and/or $R^{10}$, to form a cycle;

n is 0 or 1.

In another preferable embodiment of the present invention, ruthenium alkilidene complex defined by the general Formula **2** is subjected to a reaction with carbene defined by the general Formula **3b**

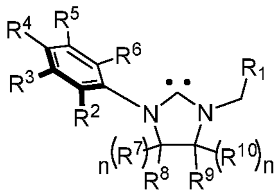

**3b**

in which:

$R^1$ is heteroaryl selected from a group encompassing furan, thiophene, benzothiophene, benzofuran;

$R^2$, $R^3$, $R^4$, $R^5$, and $R^6$ are independently hydrogen atom, methyl, isopropyl, halogen atom;

$R^7$, $R^8$, $R^9$, and $R^{10}$ are independently hydrogen atom, methyl;

n is 0 or 1.

In another preferable embodiment of the present invention, ruthenium alkylidene complex defined by general Formula 2

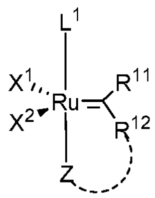

**2**

in which:

$R^{11}$, and $R^{12}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle, wherein $R^{11}$ and $R^{12}$ is preferably hydrogen atom or an aryl substituted by a nitro ($-NO_2$) group, cyanide (-CN) group, carboxyl (-COOH) group, an ester (-COOR') group, an amide ($-CONR'_2$) group, a sulphone ($-SO_2R'$) group, a formyl (-CHO) group, a sulphonamide ($-SO_2NR'_2$) group, a ketone (-COR') group, in which group R' is $C_1$-$C_5$ alkyl, $C_1$-$C_5$ perfluoroalkyl, $C_5$-$C_{24}$ aryl, $C_7$-$C_{24}$ aralkyl, $C_5$-$C_{24}$ perfluoroaryl;

$L^1$ is a neutral ligand selected from groups encompassing pyridine or substituted pyridine, $P(R')_3$, $P(OR')_3$, $O(R')_2$, $N(R')_3$, where each R' is independently a $C_1$-$C_{12}$ alkyl, $C_3$-$C_{12}$ cycloalkyl, $C_5$-$C_{20}$ aryl, $C_7$-$C_{24}$ aralkyl, $C_5$-$C_{24}$ perfluoroaryl, a 5-12 membered heteroaryl;

Z is selected from among such as, ligand $L^1$, and then substituents $R^{11}$ and $R^{12}$ are mutually connected to form cyclic structure selected from groups encompassing a $C_3$-$C_7$ cycloalkyl, $C_3$-$C_{25}$ cycloalkenyl, $C_3$-$C_{25}$ cycloalkynyl, $C_5$-$C_{24}$ aryl, $C_5$-$C_{20}$ heteroaryl, $C_5$-$C_{24}$ perfluoroaryl, 3-12 membered heterocycle, which may be independently substituted with at least one or more substituents selected from groups encompassing hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle, and then dashed line between Z and $R^{12}$ does not mean a chemical bond; heteroatom selected from group encompassing oxygen, nitrogen, sulphur, or phosphorus atom, optionally substituted by group selected from such as hydrogen atom, $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, cyanide (-CN) group, carboxyl (-COOH) group, an ester (-COOR') group, an amide ($-CONR'_2$), a sulphone ($-SO_2R'$) group, a formyl (-CHO) group, a sulphonamide ($-SO_2NR'_2$) group, a ketone (-COR') group, in which group R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, and then dashed line between heteroatom and $R^{12}$ group means a chemical bond, which is aryl optionally substituted by 1, 2, 3, or 4 substituents independently selected from group encompassing hydrogen atom, halogen atom, $C_1$-$C_{25}$ alkyl, $C_1$-$C_{25}$ perfluoroalkyl, a alkene $C_2$-$C_{25}$, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro ($-NO_2$) group, Cyanide (-CN) group, carboxyl (-COOH) group, an ester (-COOR') group, an amide ($-CONR'_2$) group, an imide (-CONR'COR') group, an amine ($-NR'_2$) group, an ammonium ($-N^+R'_3$) group, an amide (-NR'COR') group, a sulphonamide ($-NR'SO_2R'$) group, a sulphone ($-SO_2R'$) group, a formyl (-CHO) group, a sulphonamide ($-SO_2NR'_2$) group, a ketone (-COR') group, in which R' group has the following meaning: a $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

$X^1$, and $X^2$ are an anionic ligand independently selected from groups encompassing halide anions, a -CN, - SCN, -OR', -SR', -O(C=O)R', $-O(SO_2)R'$, $-OSi(R')_3$, where R' is $C_1$-$C_{12}$ alkyl, $C_3$-$C_{12}$ cycloalkyl, $C_2$-$C_{12}$ alkenyl, $C_5$-$C_{20}$ aryl, which may possibly be substituted with at least one a $C_1$-$C_{12}$ alkyl, $C_1$-$C_{12}$ perfluoroalkyl, $C_1$-$C_{12}$ alkoxyl, $C_5$-$C_{24}$ aryloxyl, $C_5$-$C_{20}$ heteroaryloxyl or halogen atom;

is subjected to a reaction with a carbene generated *in situ* as a result of the reaction of base selected from such as potassium *tert*-pentoxide, potassium *tert*-butoxide, potassium *N*,*N*-bis(trimethylsilyl)amide, or sodium hydride, with carbene precursor defined by general Formula **4a**

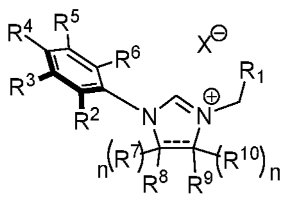

**4a**

in which:

$R^1$ is heteroaryl group;

$R^2$, $R^3$, $R^4$, $R^5$, and $R^6$ are independently hydrogen atom, $C_1$-$C_{25}$ alkyl, $C_1$-$C_{25}$ alkoxyl, or $C_2$-$C_{25}$ alkenyl, wherein substituents $R^2$, $R^3$, $R^4$, $R^5$, and $R^6$ may be mutually connected, to form substituted or unsubstituted $C_4$-$C_{10}$ cyclic or $C_4$-$C_{12}$ polycyclic structure;

$R^7$, $R^8$, $R^9$, and $R^{10}$ are independently hydrogen atom or a $C_1$-$C_{25}$ alkyl, $R^7$ and/or $R^8$ may be mutually connected with $R^9$ and/or $R^{10}$, to form cycle;

n is 0 or 1;

$X^-$ is a halide anion, or $BF_4^-$, $PF_6^-$, or $ClO_4^-$.

In another preferable embodiment of the present invention, ruthenium alkylidene complex defined by general Formula 2 is subjected to a reaction with a carbene generated *in situ* as a result of the reaction of base selected from such as potassium *tert*-pentoxide, potassium *tert*-butoxide, potassium *N,N'*-bis(trimethylsilyl)amide, or sodium hydride, with carbene precursor defined by general Formula **4b**

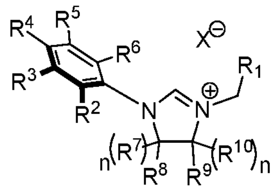

**4b**

in which:

$R^1$ is heteroaryl selected from a group encompassing furan, thiophene, benzothiophene, benzofuran;
$R^2$, $R^3$, $R^4$, $R^5$, and $R^6$ are independently hydrogen atom or methyl, isopropyl, halogen atom;
$R^7$, $R^8$, $R^9$, and $R^{10}$ are independently hydrogen atom or methyl;
n is 0 or 1;
$X^-$ is halogen anion, or $BF_4^-$, $PF_6^-$, $ClO_4^-$.

In another preferably embodiment of the present invention, as a ruthenium alkylidene complex is used compound defined by general Formula **2a**

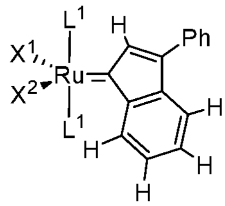

**2a**

in which:
$X^1$, $X^2$, and $L^1$ have the same meaning as defined by Formula **2.**

In another preferably embodiment of the present invention, as a ruthenium alkylidene complex is used compound defined by general Formula **2b**

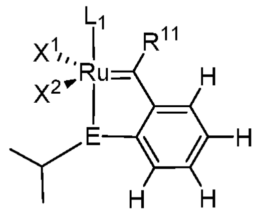

**2b**

in which:

$X^1$, $X^2$, and $L^1$, have the same meaning as defined above;

$R^{11}$ is hydrogen atom;

E is a heteroatom selected from such as oxygen, sulphur, nitrogen, phosphorus.

The subject of the present invention is also the use of compound defined by general Formula **1a** or **1b** as a precatalyst and/or catalyst in olefin metathesis reactions.

Preferably, compound defined by general Formula **1a or 1b** is used as a precatalyst and/or catalyst in ring closing metathesis (RCM), homometathesis, cross metathesis (CM), ethenolysis, isomerisation, in diastero ring-rearrangement metathesis (DRRM), in en-yne metathesis or ROMP type polymerization reaction.

Preferably, compound defined by general Formula **1a or 1b** is used as a precatalyst and/or catalyst in the ring opening metathesis polymerization (ROMP) of dicyclopentadiene or norbornene.

Preferably, compound defined by general Formula **1a or 1b** is used as a precatalyst and/or catalyst in reaction mixture in time from 1 minute to 24 hours.

Preferably, compound defined by general Formula **1a or 1b** is used as a precatalyst and/or catalyst in non-polar solvents or without solvent.

The subject of the present invention is also the use of compound defined by general Formula **1a or 1b** as a substrate for the synthesis of other ruthenium complex compound which are precatalyst and/or catalyst in the olefin metathesis reactions.

**Brief description of drawings** :

**Fig. 1** shows summary of olefin metathesis precatalysts and catalysts available on the market as well as novel precatalysts and catalysts according to the present invention.
**Fig. 2** shows structure of compound **1aa** obtained by x-ray structural analysis.
**Fig. 3** shows structure of compound **1ab** obtained by x-ray structural analysis.
**Fig. 4** shows structure of compound **1ac** obtained by x-ray structural analysis.
**Fig. 5** shows structure of compound **1ba** obtained by x-ray structural analysis.
**Fig. 6** shows structure of compound **1bb** obtained by x-ray structural analysis.
**Fig. 7** shows kinetic profile of RCM reaction of diethyl diallylmalonate (DEDAM) catalyzed by selected ruthenium complexes at 90°C in toluene, carried out under argon.
**Fig. 8** shows kinetic profile of RCM reaction of diethyl diallylmalonate (DEDAM) catalyzed by selected ruthenium complexes at 90 °C in toluene, carried out in the air.
**Fig. 9** shows kinetic profile of RCM reaction of diethyl diallylmalonate (DEDAM) catalyzed by selected ruthenium complexes at 70 °C in toluene, carried out under argon.
**Fig. 10** shows kinetic profile of RCM reaction of diethyl diallylmalonate (DEDAM) catalyzed by selected ruthenium complexes at 70 °C in toluene, carried out in the air.
**Fig. 11** shows kinetic profile of RCM reaction of diethyl diallylmalonate (DEDAM) catalyzed by selected ruthenium complexes at 50 °C in toluene, carried out under argon.
**Fig. 12** shows kinetic profile of RCM reaction of diethyl diallylmalonate (DEDAM) catalyzed by selected ruthenium complexes at 50 °C in 2-methyltetrahydrofuran, carried out under argon.

**Definitions of frequently used terms**

In the present invention the terms have the meanings as follow. Terms not specifically defined herein should be given the meanings that would be given to them by one of skill in the art in light of the disclosure and the context. As used in the specification, however, unless specified to the contrary, the following terms have the meaning indicated and the following conventions are adhered to.

The term *"halogen atom"* is an element selected from among F, Cl, Br, and I.

The term *"carbene"* is a molecule containing a neutral carbon atom with the valence number 2 and two unpaired (triplet carbene) or paired (singlet carbene) valence electrons. The term *"carbene"* also encompasses carbene analogues in which the carbon atom has been substituted by another chemical element such as boron, silicon, germanium, tin, lead, nitrogen, phosphorus, sulphur, selenium, and tellurium.

The term *"alkyl"* refers to a saturated, linear or branched hydrocarbon substituent with an indicated number of carbon atoms. Examples of alkyl substituents are -methyl, -ethyl, -*n*-propyl, -*n*-butyl, -*n*-pentyl, -*n*-hexyl, -*n*-heptyl, -*n*-octyl, -*n*-nonyl, and -*n*-decyl.

Representative branched -($C_1$-$C_{10}$) alkyls encompass -isopropyl, -*sec*-butyl, -isobutyl, -*tert*-butyl, -isopentyl, -neopentyl, -1-methylbutyl, -2-methylbutyl, -3-methylbutyl, -1,1-dimethylpropyl, -1,2-dimethylpropyl, -1-methylpentyl, -2-methylpentyl, -3-methylpentyl, -4-methylpentyl, -1-ethylbutyl, -2-ethylbutyl, -3-ethylbutyl, -1,1-dimethylbutyl, -1,2-dimethylbutyl, -1,3-dimethylbutyl, -2,2-dimethylbutyl, -2,3-dimethylbutyl, -3,3-dimethylbutyl, -1-methylhexyl, -2-methylhexyl, -3-methylhexyl, -4-methylhexyl, -5-methylhexyl, -1,2-dimethylpentyl, -1,3- dimethylpentyl, -1,2-dimethylhexyl, -1,3-dimethylhexyl, -3,3-dimethylhexyl, -1,2-dimethylheptyl, -1,3-dimethylheptyl, and -3,3-dimethylheptyl, and the like.

The term *"alkoxyl"* refers to alkyl or cycloalkyl substituents as defined above and attached via an oxygen atom.

The term *"perfluoroalkyf'* is an alkyl group as defined above, wherein all hydrogen atoms have been substituted by identical or different halide atoms.

The term *"cycloalkyl"* refers to a saturated mono- or polycyclic hydrocarbon substituent with the indicated number of carbon atoms. Examples of cycloalkyl substituents are -cyclopropyl, -cyclobutyl, -cyclopentyl, - cyclohexyl, -cycloheptyl, -cyclooctyl, -cyclononyl, cyclodecyl, and the like.

The term *"alkenyl"* refers to an unsaturated, linear, or branched acyclic hydrocarbon substituent with the indicated number of carbon atoms and containing at least one double carbon-carbon bond. Examples of an alkenyl substituent include -vinyl, -allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, -2-octenyl, -3-octenyl, -1-nonenyl, -2-nonenyl, -3-nonenyl, -1-decenyl, -2-decenyl, -3-decenyl, and the like.

The term *"cycloalkenyl"* refers to an unsaturated mono- or polycyclic hydrocarbon substituent with the indicated number of carbon atoms and containing at least one double carbon-carbon bond. Examples of a cycloalkenyl substituent include -cyclopentenyl, -cyclopentadienyl, -cyclohexenyl, -cyclohexadienyl, - cycloheptenyl, -cycloheptadienyl, -cycloheptatrienyl, -cyclooctenyl, -cyclooctadienyl, -cyclooctatrienyl, - cyclooctatetraenyl, -cyclononenyl, -cyclononadienyl, -cyclodecenyl, -cyclodecadienyl, and the like.

The term *"alkynyl"* refers to an unsaturated, linear, or branched acyclic hydrocarbon substituent with the indicated number of carbon atoms and containing at least one triple carbon-carbon bond. Examples of an alkynyl substituent include -acetylenyl, -propynyl, -1-butynyl, -2-butynyl, -1-pentynyl, -2-pentynyl, -3-methyl-1-butynyl, -4-pentynyl, -1-hexynyl, -2-hexynyl, -5-hexynyl, and the like.

The term *"cycloalkynyl"* refers to an unsaturated mono- or polycyclic hydrocarbon substituent with the indicated number of carbon atoms and containing at least one triple carbon-carbon bond. Examples of a cycloalkynyl substituent include -cyclohexynyl, -cycloheptynyl, -cyclooctynyl, and the like.

The term *"aryl"* refers to an aromatic mono- or polycyclic hydrocarbon substituent with the indicated number of carbon atoms, possibly substituted with at least one alkyl, alkoxyl, aryloxyl, halogen atom, hydroxyl group, nitro group, ester group or ketone group, cyanide group, amide group, carboxyl group, sulphonamide group, formyl group or ether group. Examples of an aryl substituent include -phenyl, -tolyl, -xylyl, -naphthyl, -2,4,6-trimethylphenyl, -2-fluorophenyl, -4-fluorophenyl, -2,4,6-trifluorophenyl, -2,6-difluorophenyl, -4-nitrophenyl, and the like.

The term *"aralkyl"* refers to alkyl substituents as defined above, substituted with at least one aryl as defined above. Examples of an aralkyl substituent include -benzyl, -diphenylmethyl, -triphenylmethyl, and the like.

The term *"heteroaryl"* refers to an aromatic mono- or polycyclic hydrocarbon substituent with the indicated number of carbon atoms, in which at least one carbon atom has been substituted by a heteroatom selected from among O, N, and S. Examples of heteroaryl substituents include -furyl, -thienyl, -imidazolyl, -oxazolyl, - thiazolyl, -isoxazolyl, -triazolyl, -oxadiazolyl, -tiadiazolyl, -tetrazolyl, -pyridyl, -pyrimidyl, -triazynyl, -indolyl, - benzo[b]furyl, -benzo[b]tienyl, -indazolyl, -benzoimidazolyl, -azaindolyl, -quinolyl, -isoquinolyl, -carbazolyl, and the like.

The term *"perfluoroaryl"* is an aryl group as defined above in which all hydrogen atoms have been substituted by identical or different halogen atoms.

The term *"heterocycle"* refers to a saturated or partially unsaturated, mono- or polycyclic hydrocarbon substituent, with the indicated number of carbon atoms, in which at least one carbon atom has been substituted by a heteroatom selected from among O, N and S. Examples of a heterocyclic substituent include -furyl, -thiophenyl, -pyrolyl, -oxazolyl, -imidazolyl, -thiazolyl, -isoxazolyl, -pirazolyl, -isothiazolyl, -triazynyl, - pyrolidynonyl, -pyrolidynyl, -hydantoinyl, -oxiranyl, -oxethanyl, -tetrahydrofuranyl, -tetrahydrotiophenyl, - quinolinyl, -isoquinolinyl, -chromonyl, -cumarynyl, -indolyl, -indolizynyl, -benzo[b]furanyl, -benzo[b]tiophenyl, - indazolyl, -purynyl, -4*H*-quinolizynyl, -isoquinolyl, -quinolyl, -phthalazynyl, -naphthyrydynyl, -carbazolyl, -β-carbolinyl, and the like.

The term *"neutral ligand"* refers to a substituent devoid of charge, capable of coordinating with a metallic centre (ruthenium atom). Examples of such ligands may include: amines, phosphines and their oxides, phosphorines, alkyl and aryl phosphates, arsines and their oxides, ethers, aryl and alkyl sulphides, coordinated hydrocarbons, alkyl and aryl halides.

The term *"indenylidene"* refers to unsaturated hydrocarbon substituent of indene backbone (benzocyclopentadiene) connected via double bound with metallic atom center.

The term *"heteroindenylidene"* refers to substituent indenylidene defined above, in which at least one carbon atom has been substituted by a heteroatom selected from among: nitrogen, oxygen, and sulphur.

The term *"anionic ligand"* refers to a substituent capable of coordinating with a metallic centre (ruthenium atom) possessing a charge, capable of partially or completely compensating the charge of the metallic centre. Examples of such ligands may be: such anions as fluoride, bromide, iodide, cyanide, cyanate and thiocyanate, carboxylic acid anions, alcohol anions, phenolic anions, thiol and thiophenol anions, hydrocarobon anions with a delocalised charge (i.e. cyclopentadiene), anions of (organo)sulphuric and (organo)phosphoric acids and esters thereof (such as i.e. anions of alkylsulphonic and arylsulphonic acids, anions of arylphsophoric and alkylphosphoric acids, anions of aryl and alkyl esters of sulphuric acids, anions of aryl and alkyl esters of phosphoric acids, anions of anions of aryl and alkyl esters of alkylphosphoric and arylophosphoric acids). Possibly, an anionic ligand may possess bound groups $L^1$, $L^2$, and $L^3$, connected such as a catechol anion, an acetylacetone anion or a salicyl aldehyde anion. The anionic ligands ($X^1$, $X^2$) and neutral ligands ($L^1$, $L^2$, $L^3$) may be together connected to one another forming multidentate ligands, such as a: bidentate ligand ($X^1$-$X^2$), tridentate ligand ($X^1$-$X^2$-$L^1$), tetradentate ligand ($X^1$-$X^2$-$L^1$-$L^2$), bidentate ligand ($X^1$-$L^1$ tridentate ligand ($X^1$-$L^1$-$L^2$), or bidentate ligand ($L^1$-$L^2$). Examples of such ligands include a catechol anion, an acetylacetone anion as well as a salicylic aldehyde anion.

The term *"heteroatom"* refers to element selected from among oxygen, sulphur, nitrogen, phosphorus, and the like.

The term *"chlorinated solven"'* refers to solvents, in which at least one hydrogen atom has been substituted by halogen atom from among F, Cl, Br and I; preferably more than one. Examples of a chlorinated solvents are: dichloromethane, chloroform, tetrachloromethane (carbon tetrachloride), 1,2-dichloroethane, chlorobenzene, 1,2-dichlorobenzene, hexafluorobenzene, octafluorotoluene, freons, and the like.

The term *"non-polar solvent"* refers to solvent with zero or a very small dipole moment. Examples of a chlorinated solvents are: pentane, hexane, octane, nonane, decane, benzene, toluene, tetrahydrofuran (THF) and derivatives thereof, diethyl ether, dichloromethane, ethyl acetate, chloroform, and the like.

The term *"DEDAM"* is diethyl diallylmalonate, used as a model diene in RCM reactions that compare the activity of olefin metathesis precatalysts and catalysts.

The term *"GC"* is gas chromatography.

The term *"HPLC"* is high pressure liquid chromatography, whereas the solvents indicated as "HPLC solventa" refers to solvents of sufficient purity for HPLC analysis.

The term *"NMR"* is nuclear magnetic resonance.

The term *"NHC"* is *N*-heterocyclic carbene.

The term *"TLC"* is thin layer chromatography.

The term *"en-yne"* is compound having in its structure a double and a triple bond.

The term *"precatalyst"* refers to ruthenium complexes 16-electron chemical compound, which after dissociation of one ligand or compound's structure reorganization turn into the corresponding 14-electron olefin metathesis catalyst, which is actively involved in the catalytic cycle.

Compounds of the invention have been prepared according to the sequence of the following reaction shown in schemes 1-3. In the first step, amine's synthesis of general formula (**III**) have been obtained, which is shown on the scheme 1 (examples I-IV).

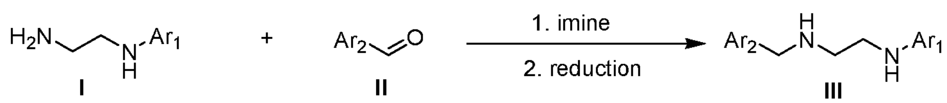


Scheme 1

The basic reaction for preparation of *N,N'*-disubstituted diamine (**III**) is conducted according to the one pot procedure, i.e. the procedure in one reaction vessel, involving formation of the imine (reaction between aldehyde (**I**) and diamine (**II**)) and then reduction of imine into diamine (**III**).

Then, corresponding NHC hydrochloride salt were obtained via condensation reaction of diamine (**III**) hydrochloride, by treating them with triethylorthoformate and anion exchange into tetrafluoroborate using $NH_4BF_4$. Synthesis of compounds (**IV**) were conducted according to the general reactions presented on scheme 2 (Examples V-VIII).

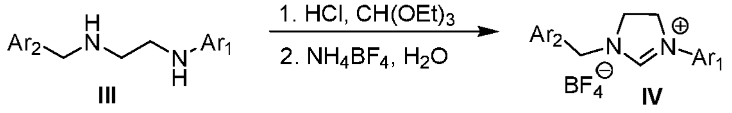

**III** **IV**

Scheme 2

Ruthenium complex, compound of the general formula **1** according to the present invention is obtained in the general method described on scheme 3 (experimental data, see Examples IX - XIII), consisting of reaction between the NHC carbene (**V**), generated *"in situ"* from the corresponding NHC salt (**IV**), and corresponding first generation ruthenium alkylidene complex (Umicore **M1™** bearing two phosphine ligands or **Hov-I** bearing one phosphine ligand). NHC carbene was obtained in the deprotonation of the corresponding NHC salt with a base, preferably potassium *tert*-pentoxide, sodium hydride, KHMDS; carried out in non-polar solvents, aromatic or aliphatic hydrocarbons, preferably toluene, hexane.

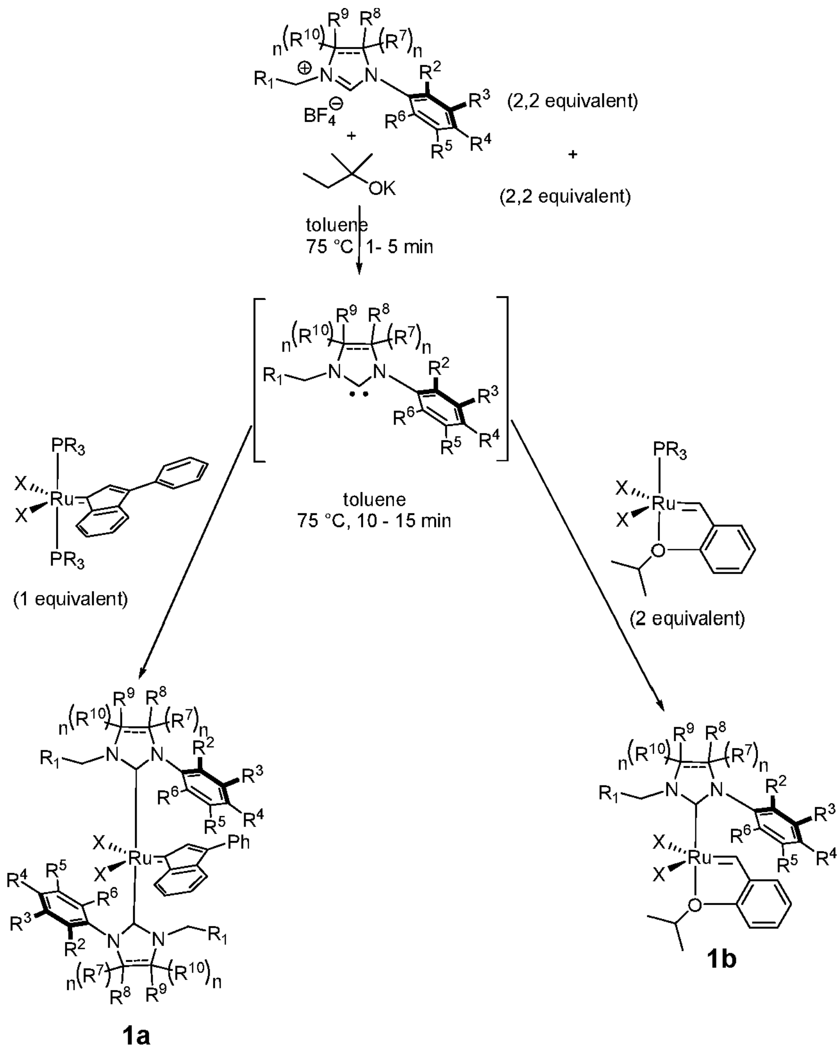

**1a** **1b**

Scheme 3

In the embodiments illustrated below, the preparation method and use of new ruthenium complexes of the invention are described. The examples below are designed for a better understanding of the present invention, but they are not intended to limit the scope thereof in any way. The examples of comparative reactions using known complexes confirmed that the novel complexes of the invention are more thermally stable than complexes already known in the art. Moreover, novel ruthenium complexes show different catalytic properties.

## Examples

### Example I

Synthesis of *N*-(2,6-diisopropylphenyl)-*N*'-(furan-2-ylmethyl)-1,2-diaminoethane (**diamine 1** according to Scheme 1)

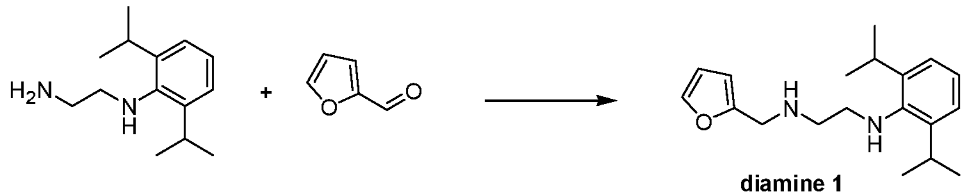

**diamine 1**

To the solution of diamine (*N*-(2,6-diisopropylphfenyl)-1,2-diaminoethane 11.0 g, 50 mmol) in methanol (150 ml), aldehyde (furfural 4.8 g, 50 mmol) was added in one portion, then subsequently 3 droplets of 85% formic acid and sodium sulphate (1 g) were added. Stirring for 24 hours at room temperature. Next, sodium borohydride (15.9 g, 400 mmol) was added portionwise and stirring was continued for additional 24 hours. Then, 5% solution of NaOH (200 ml) was added, and methanol was evaporated from the mixture. The mixture was washed with methylene chloride (4×100 ml). The combined fractions were dried over dry sodium sulphate, solvent was evaporated. Crude product was purified on column chromatography with silica gel, using as eluent 10%, and then 30% ethyl acetate/cyclohexane. After solvent evaporation, 12.1 g of yellow oil was obtained (which is 80% of theoretical yield).
$^{1}H$ NMR (400 MHz, $CDCl_3$): δ = 7.38 (dd, *J* = 1.9 Hz, *J* = 0.8 Hz, 1H), 7.12 - 7.04 (m, 3H), 6,34 (dd, *J* = 3.2 Hz, *J* = 1.9 Hz, 1H), 6.20 (dd, *J* = 3.2 Hz, *J* = 0.7 Hz, 1H), 3.86 (s, 2H), 3.31 (septet, *J* = 6.9 Hz, 2H), 3.00 - 2.97 (m, 2H), 2.89 - 2.87 (m, 2H), 2.53 (broad s, 2H), 1.24 (d, *J* = 6.9 Hz, 12H) ppm.
$^{13}C$ NMR (100 MHz, $CDCl_3$): δ = 154.2; 143.5; 142.6; 141.9; 123.7; 123.6; 110.3; 106.9; 51.2; 49.2; 46.1; 27.7; 24.4 ppm.
IR (film from $CH_2Cl_2$): *v* = 3357, 3114, 3060, 3029, 2961, 2929, 2868, 2835, 1678, 1590, 1505, 1459, 1445, 1383, 1362, 1334, 1254, 1199, 1147, 1111, 1073, 1055, 1011, 920, 884, 800, 755, 734, 599, 577, 529, 432 $cm^{-1}$.
Elemental Analysis for $C_{19}H_{28}N_2O$; calculated: %C, 75.96; %H, 9.39; %N, 9.32; found: %C, 75.86; %H, 9.35; %N, 9.31.
LRMS ESI (*m/z*) for $C_{19}H_{28}N_2NaO$ $[M+Na]^+$: calculated: 323.2; found: 323.1.

### Example II

Synthesis of *N*-(2,6-diizopropylphenyl)-*N*'-(tiofen-2-ylomethyl)-1,2-diaminoethan (**diamine 2** according to Scheme 1).

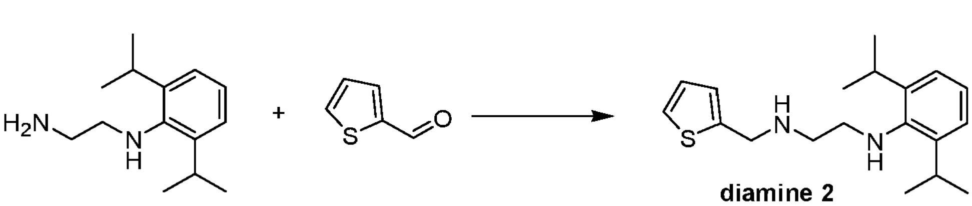

**diamine 2**

The same procedure as described for diamine 1 (in Example **I**) was employed [using *N*-(2,6-diisopropylphenyl)-1,2-diaminoethan - 11.0 g, 50 mmol and 2-thiophenecarboxaldehyde - 5.61 g, 50 mmol], white solid was obtained (13.0 g, 82% of yield). Melting point = 52-53 °C.
$^{1}H$ NMR (400 MHz, $CDCl_3$): δ = 7.25 (dd, *J* = 4.8 Hz, *J* = 1.4 Hz, 1H), 7.13 - 7.05 (m, 3H), 7,00 - 6.96 (m, 2H), 4.08 (s, 2H), 3.36 (septet, *J* = 6.8 Hz, 2H), 3.03 - 3.00 (m, 2H), 2.97 - 2.94 (m, 2H), 1.27 (d, *J* = 6.9 Hz, 12H) ppm. (NH hydrogen's were not observed).
$^{13}C$ NMR (100 MHz, $CDCl_3$): δ = 144.4; 143.6; 142.5; 126.7; 124.8; 124.5; 123.7; 123.6; 51.3; 49.3; 48.6; 27.7; 24.5 ppm.
IR (KBr): *v* = 3362, 3349, 3279, 3111, 3067, 3047, 3030, 2959, 2930, 2868, 2833, 2753, 1902, 1845, 1791, 1767, 1696,

1643, 1591, 1540, 1490, 1456, 1382, 1362, 1311, 1250, 1222, 1204, 1168, 1146, 1111, 1035, 1003, 974, 958, 941, 904, 852, 822, 804, 792, 749, 691, 619, 580, 534, 504, 478, 456, 426 $cm^{-1}$.

Elemental Analysis for $C_{19}H_{28}N_2S$; calculated: %C, 72.10; %H, 8.92; %N, 8.85; %S, 10.13; found: %C, 72.24; %H, 8.91; %N, 8.82; %S, 10.05.

LRMS ESI (*m/z*) for $C_{19}H_{28}N_2NaS$ $[M+Na]^+$: calculated: 339,2; found: 339,0.

**Example III**

Synthesis of *N*-benzo[b]thiophen-2-ylomethyl-*N'*-(2,6-diisopropylphenyl)-1,2-diaminoethane (**diamine 3** according to Scheme 1)

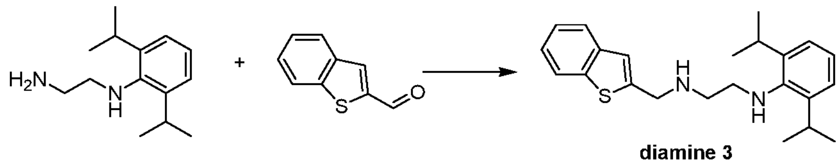

diamine 3

The same procedure as described for diamine 1 (in Example I) was employed (using *N*-(2,6-diisopropylphenyl)-1,2-diaminoethane - 7.71 g, 35 mmol and benzo[b]thiophen-2-carboxyaldehyde - 5.68 g, 35 mmol), white solid was obtained (11.2 g with 87% of yield). Melting point = 79 - 80°C.

$^1H$ NMR (400 MHz, $CDCl_3$): δ = 7.83 (dd, J = 7.7 Hz, J = 0.7 Hz, 1H), 7.73 - 7.71 (m, 1H), 7.37 - 7.29 (m, 2H), 7.19 (broad s, 1H), 7.14 - 7.05 (m, 3H), 4.17 (broad s, 2H), 3.62 (broad s, 1H), 3.37 (septet, *J* = 6.8 Hz, 2H), 3.02 - 2.99 (m, 4H), 1.68 (broad s, 1H), 1.27 (d, *J* = 6.9 Hz, 12H) ppm.

$^{13}C$ NMR (100 MHz, $CDCl_3$): δ = 145.6; 143.5; 142.5; 139.9; 139.8; 124.3; 124.0; 123.7; 123.6; 123.2; 122.5; 121.2; 51.3; 49.4; 49.3; 27.7; 24.5 ppm.

IR (KBr): *v* = 3345, 3281, 3080, 3070, 3057, 3041, 2965, 2927, 2865, 2826, 2701, 1925, 1897, 1864, 1820, 1808, 1790, 1690, 1661, 1588, 1458, 1442, 1417, 1381, 1367, 1361, 1340, 1317, 1302, 1279, 1254, 1242, 1230, 1206, 1185, 1143, 1129, 1116, 1090, 1067, 1051, 1040, 1014, 1002, 986, 959, 944, 933, 914, 896, 881, 856, 831, 791, 756, 742, 726, 708, 675, 626, 586, 566, 555, 534, 488, 475, 447, 428, 418 $cm^{-1}$.

Elemental Analysis for $C_{23}H_{30}N_2S$; calculated: %C, 75.36; %H, 8.25; %N, 7.64; %S, 8.75; found: %C, 75.52; %H, 8.16; %N, 7.68; %S, 8.50.

LRMS ESI *(m/z)* for $C_{23}H_{30}N_2NaS$ $[M+Na]^+$: calculated: 389.2; found: 389.1.

**Example IV**

Synthesis of *N*-(2,4,6-trimethylphenyl)-*N'*-(thiohen-2-ylmethyl)-1,2-diaminoethane (**diamine 4** according to Scheme 1

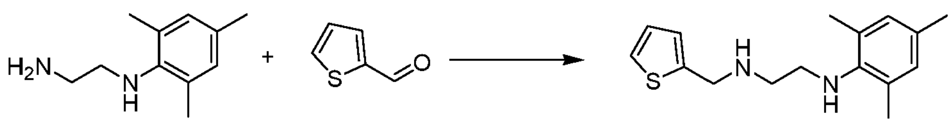

diamine 4

The same procedure as described for diamine 1 (in Example I) was employed (using *N*-(2,4,6-trimethylphenyl)-1,2-diaminoethane - 12.5 g, 70 mmol, 2-thiophenecarboxaldehyde - 8.01 g, 70 mmol and sodium borohydride - 223 g, 560 mmol), dense colourless oil was obtained (15.4 g which is 80% of theoretical yield).

$^1H$ NMR (500 MHz, $CDCl_3$): δ = 7.25 (dd, *J* = 5.0 Hz, *J* = 1.3 Hz, 1H), 7.00 - 6.97 (m, 2H), 6.86 (s, 2H), 4.07 (broad s, 2H), 3.10 - 3.07 (m, 2H), 2.93 - 2.91 (m, 2H), 2.67 (broad s, 2H), 2.33 (s, 6H), 2.27 (s, 3H) ppm.

$^{13}C$ NMR (125 MHz, $CDCl_3$): δ = 144.1; 143.7; 131.1; 129.7; 129.5; 126.7; 124.9; 124.5; 49.2; 48.3; 48.2; 20.6; 18.5 ppm.

IR (film from $CH_2Cl_2$): *v* = 3351, 3069, 2938, 2915, 2852, 2730, 1593, 1485, 1443, 1371, 1304, 1232, 1112, 1036, 852, 823, 697 $cm^{-1}$.

Elemental Analysis for $C_{16}H_{22}N_2S$; calculated: %C, 70.03; %H, 8.08; %N, 10.21; %S, 11.68; found: %C, 70.15; %H, 8.09; %N, 10.26; %S, 11.84.

LRMS ESI *(m/z)* for $C_{16}H_{22}N_2NaS$ $[M+Na]^+$: calculated: 297.1; found: 297.1.

## Example V

Synthesis of 1-(2,6-diizopropylofenylo)-3-(furano-2-ylometylo)-4,5-dihydro-3H-imidazoliowego tetrafluoroborate (**NHC 1** according to Scheme 2)

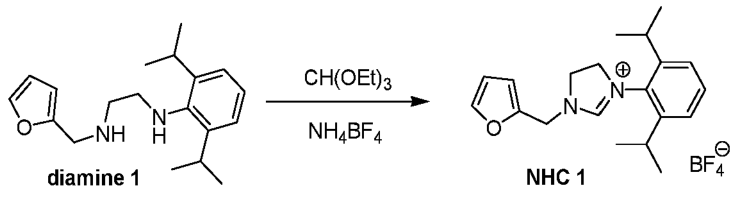


**diamine 1** **NHC 1**

**Diamine 1** (10.5 g, 35.0 mmol) and triethyl orthoformate (59.5 ml, 350 mmol) were placed in the flask and stirred at room temperature. After 10 minutes, HCl in 1,4-dioxane (17.5 ml, 4 mol/dm$^3$, 70.0 mmol) was added dropwise. Then, the reaction mixture was heated to 90°C and stirred for 12 hours. At the end, temperature was increased to 120°C and stirred for 2 hours. The reaction mixture was cooled down, white precipitate was filtered off, washed with n-hexane and dried. Crude product was dissolved in hot water, and stirring ammonium tetrafluoroborate (4,16 g, 38,5 mmola) was added portionwise. The mixture was cooled down, 100 ml of methylene chloride was added and the mixture was extracted (it was repeated $CH_2Cl_2$ 3x50 ml). The combined organic layers were dried over dry magnesium sulphate, filtered off and carefully concentrated. Then, toluene was added and resulting mixture was slowly concentrated under reduced pressure on the rotary evaporator without heating, to remove methylene chloride. The resulting suspension of NHC salt was filtered off on the fritted glass funnel under reduced pressure, washed with toluene, n-hexane and dried. White solid was obtained (11,2 g which is 80% of theoretical yield). Melting point: 158 - 160°C.
$^1$H NMR (400 MHz, $CD_2Cl_2$): δ = 8.10 (s, 1H), 7.50 (dd, *J* = 1.9 Hz, *J* = 0.8 Hz, 1H), 7.49 -7.45 (m, 1H), 7.28 - 7.26 (m, 2H), 6.61 (dd, *J* = 3.3 Hz, *J* = 0.7 Hz, 1H), 6.44 (dd, *J* = 3.3 Hz, *J* = 1.9 Hz, 1H), 4.87 (s, 2H), 4.23 - 4.12 (m, 4H), 2.86 (septet, *J* = 6.8 Hz, 2H), 1.26 (d, *J* = 6.8 Hz, 6H), 1.21 (d, *J* = 6.8 Hz, 6H) ppm.
$^{13}$C NMR (100 MHz. $CD_2Cl_2$): δ = 158.6; 146.9; 146.4; 144.5; 131.6; 130.0; 125.3; 112.0; 111.4; 54.0; 49.1; 45.0; 29.0; 24.8; 24.2 ppm.
IR (KBr): *v* = 3158, 3070, 2965, 2929, 2871, 1643, 1593, 1514, 1479, 1462, 1443, 1389, 1366, 1359, 1346, 1290, 1270, 1253, 1235, 1192, 1151, 1063, 1011, 980, 935, 913, 884, 834, 807, 764, 660, 602, 553, 540, 522, 481, 458, 413 cm$^{-1}$.
Elemental Analysis for $C_{20}H_{27}BF_4N_2O$; calculated: %C, 60.32; %H, 6.83; %N, 7.03; found: %C, 60.36; %H, 7.04; %N, 7.05.
LRMS ESI (*m/z*) for $C_{20}H_{27}N_2O$ $[M-BF_4]^+$: calculated: 311.2; found: 311.1.

## Example VI

Synthesis of 1-(2,6-diisopropylphenyl)-3-(thiophen-2-ylmethyl)-4,5-dihydro-3H-imidazolinium tetrafluoroborate **(NHC 2** according to Scheme 2)

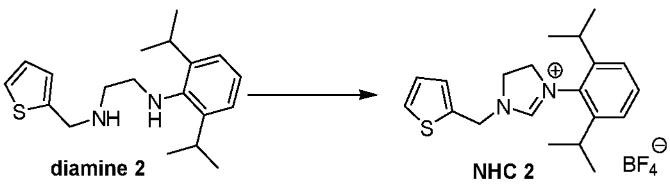

**diamine 2** **NHC 2**

The same procedure as described for NHC 1 (in Example V), using **diamine 2** (12.5 g, 39.4 mmol), triethyl orthoformate (66.9 ml, 394 mmol), HCl in 1,4-dioxane (19.7 ml, 4 mol/dm$^3$, 78.8 mmol) and ammonium tetrafluoroborate (4.68 g, 43.3 mmol), white solid was obtained (13.9 g with 85% of yield). Melting point: 184 - 185°C.
$^1$H NMR (400 MHz, $CD_2Cl_2$): δ = 8.12 (s, 1H), 7.48 - 7.43 (m, 2H), 7.28 - 7.25 (m, 3H), 7.07 (dd, *J* = 5.1 Hz, *J* = 3.5 Hz, 1H), 5.05 (s, 2H), 4.24 - 4.12 (m, 4H), 2.87 (septet, *J* = 6.8 Hz, 2H), 1.26 (d, *J* = 6.8 Hz, 6H), 1.21 (d, *J* = 6.8 Hz, 6H) ppm.
$^{13}$C NMR (100 MHz, $CD_2Cl_2$): δ = 158.1; 147.0; 134.5; 131.6; 130.3; 130.1; 128.3; 128.2; 125.3; 54.0; 48.8; 46.9; 29.1; 24.9; 24.1 ppm.
IR (KBr): *v* = 3124, 3072, 2963, 2928, 2870, 2587, 1972, 1898, 1828, 1646, 1590, 1532, 1512, 1477, 1461, 1449, 1387, 1364, 1336, 1343, 1292, 1270, 1259, 1233, 1214, 1194, 1171, 1148, 1067, 936, 912, 877, 852, 808, 764, 741, 679, 655, 612, 593, 553, 522, 482, 456, 410 cm$^{-1}$.
Elemental Analysis for $C_{20}H_{27}BF_4N_2S$; calculated: %C, 57.98; %H, 6.57; %N, 6.76; %S, 7.74; found: %C, 58.05; %H,

6.61; %N, 6.78; %S, 7.68.
LRMS ESI (*m/z*) for $C_{20}H_{27}N_2S$ [M-$BF_4$]$^+$: calculated: 327.2; found: 327.1.

**Example VII**

Synthesis of 1-(2,6-diisopropylphenyl)-3-(thiophen-2-ylmethyl)-4,5-dihydro-3*H*-imidazolinium tetrafluoroborate **(NHC 3** according to Scheme 2)

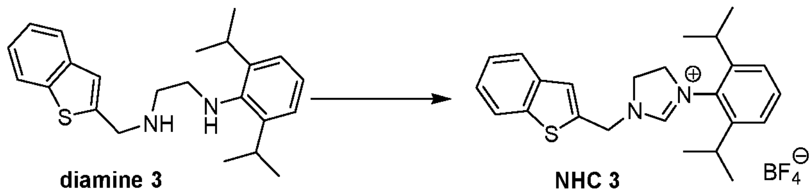

diamine 3 NHC 3

The same procedure as described for NHC 1 (in Example V), apart from that, after addition of solution of HCl in 1,4-dioxane, reaction mixture was heated to a 90°C and held at this temperature for 12 hours, then temperature was increased to 130°C and the whole mixture was mixing for two hours, using **diamine 3** (10.9 g, 29.7 mmol), triethyl orthoformate (50.5 ml, 297 mmol), HCl in 1,4-dioxane (14.8 ml, 4 mole/dm$^3$, 59.4 mmol) and ammonium tetrafluoroborate (3.53 g, 32.7 mmol)], white solid was obtained (10.4 g with 75% of yield). Melting point: 205 - 207°C.
$^1$H NMR (400 MHz, $CD_2Cl_2$): δ = 8.21 (s, 1H), 7.88 - 7.79 (m, 2H), 7.50 (broad s, 1H), 7.48 - 7.45 (m, 1H), 7.43 - 7.38 (m, 2H), 7.28 - 7.26 (m, 2H), 5.16 (s, 2H), 4.27 - 4.13 (m, 4H), 2.90 (septet, *J* = 6.8 Hz, 2H), 1.27 (d, *J* = 6.8 Hz, 6H), 1.24 (d, *J* = 6.8 Hz, 6H) ppm.
$^{13}$C NMR (100 MHz, $CD_2Cl_2$): δ = 158.3; 147.0; 140.8; 139.7; 135.3; 131.7; 130.0; 127.2; 125.8; 125.3; 125.3; 124.6; 122.9; 54.0; 48.8; 47.9; 29.1; 24.9; 24.2 ppm.
IR (KBr): *v* = 3072, 2964, 2929, 2871, 1641, 1592, 1570, 1537, 1508, 1476, 1458, 1452, 1387, 1368, 1357, 1334, 1292, 1274, 1255, 1237, 1184, 1164, 1149, 1060, 936, 869, 842, 812, 765, 742, 724, 708, 686, 674, 638, 611, 593, 566, 522, 494, 477, 456, 424 cm$^{-1}$.
Elemental Analysis for $C_{24}H_{29}BF_4N_2S$; calculated: %C, 62.07; %H, 6.29; %N, 6.03; %S, 6.91; found: %C, 62.11; %H, 6.21; %N, 5.96; %S, 6.82.
LRMS ESI *(m/z)* for $C_{24}H_{29}N_2S$ [M-$BF_4$]$^+$: calculated: 377.2; found: 377.1.

**Example VIII**

Synthesis of 1-(2,4,6-trimethylphfenyl)-3-(thiophen-2-ylmethyl)-4,5-dihydro-3H-imidazolinium tetrafluoroborate (**NHC 4** according to Scheme 2)

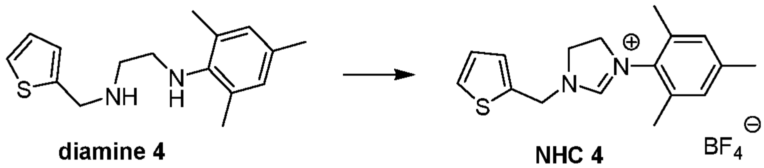

diamine 4 NHC 4

The same procedure as described for **NHC 1** (in Example V), apart from that, after addition of solution of HCl in 1,4-dioxane, reaction mixture was heated to a 80°C and held at this temperature for 12 hours, then temperature was increased to 120°C and the whole mixture was mixing for an hours, using **diamine 4** (15.4 g, 56.1 mmol), triethylu orthoformate (96 ml, 561 mmol), HCl in 1,4-dioxane (37.4 ml, 3 mole/dm$^3$, 112 mmol) and ammonium tetrafluoroborte (6.67 g, 61.7 mmol). At that time, formation of a yellow oil was observed. After cooling down of the mixture, solvent was evaporated, and resulting yellow oil was dissolved in the mixture of solvents water/methanol (2/1, volume) and ammonium tetrafluoroborate was added. Then, proceeded as in Example V. White solid was obtained (15.9 g with 76% of yield). Melting point: 142.1 - 143.2°C.
$^1$H NMR (500 MHz, $CDCl_3$): δ = 8.12 (s, 1H), 7.33 (dd, *J* = 5.1 Hz, *J* = 1.2 Hz, 1H), 7.21 (dd, *J* = 3.5 Hz, *J* = 1.2 Hz, 1H), 6.99 (dd, *J* = 5.1 Hz, *J* = 3.5 Hz, 1H), 6.88 (broad s, 2H), 5.01 (s, 2H), 4.17 - 4.08 (m, 4H), 2.25 (s, 3H), 2.21 (s, 6H) ppm.
$^{13}$C NMR (125 MHz, $CDCl_3$): δ = 158.0; 140.4; 135.5; 134.5; 130.6; 130.0; 129.9; 127.9; 127.6; 51.2; 48.3; 46.5; 21.1; 17.5 ppm.
IR (KBr): *v* = 3089, 2921, 2743, 1643, 1450, 1488, 1376, 1250, 1217, 1185, 1134, 1053, 860, 712, 470 cm$^{-1}$.

Elemental Analysis for $C_{17}H_{21}BF_4N_2S$; calculated: %C, 54.85; %H, 5.69; %N, 7.53; %S, 8.61; found: %C, 54.78; %H, 5.79; %N, 7.37; %S, 8.72.
LRMS ESI (*m/z*) for $C_{17}H_{21}N_2S$ $[M-BF_4^-]^+$: calculated: 285.1; found: 285.1.

## Example IX

Synthesis of indenylidene type of precatalyst bearing two NHC with furan $[(Fur\text{-}NHC\text{-}DiPP)_2RuCl_2(Ind)]$ (**1aa** according to Scheme 3)

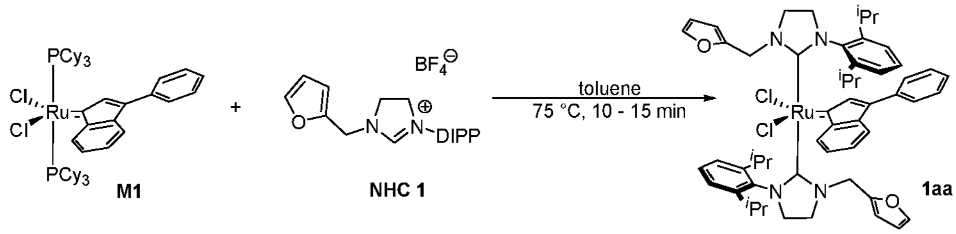

**M1** **NHC 1** **1aa**

Salt **NHC 1** (213 mg 0.535 mmol) and toluen (10 ml) was placed under argon in Schlenk vessel. Potassium *tert*-pentoxide (0.3 ml of 25% solution in toluene) was added to the reaction mixture at 75°C and stirred for 5 minutes. Then, complex Umicore **M1™** (224 mg, 0.243 mmol) was added to the reaction mixture and stirred for additional 10 minutes. After this time, TLC analysis indicated indicate the presence only a traces of Umicore **M1™.** The reaction mixture was cooled down, and concentrated on rotary evaporator under reduced pressure. The product was isolated on column chromatography with silica gel, using as eluent 5% ethyl acetate/cyclohexane, and then 20% ethyl acetate/cyclohexane. Collected fractions were concentrated on the rotary evaporator under reduced pressure, and the residues that containing significant amount of tricyclohexylphosphine, were purified for the second time on the column chromatography with silica gel, using as eluent 5% ethyl acetate/cyclohexane, until appeared the first drops containing the expected product, which was eluted with 10% solution of ethyl acetate/cyclohexane. Collected and combined fractions were concentrated on the rotary evaporator. The crude product was crystallised from a solvent mixture $CH_2Cl_2$/*n*-hexane. Precatalyst **1aa** was obtained as a brick red solid (165 mg which is 69% of theoretical field).
$^1H$ NMR (400 MHz, $CD_2Cl_2$): δ = 8.56 - 8.54 (m, 1H), 7.54 (dd, *J* = 1.8 Hz, *J* = 0.7 Hz, 1H), 7.52 - 7.48 (m, 3H), 7.41 - 7.29 (m, 4H), 7.22 (dd, *J* = 7.7 Hz, *J* = 1.4 Hz, 1H), 7.15 - 7.07 (m, 3H), 6.91 (dd, *J* = 7.7 Hz, *J* = 1.4 Hz, 1H), 6.67 (dd, *J* = 7.7 Hz, *J* = 1.3 Hz, 1H), 6.63 (dd, *J* = 7.8 Hz, *J* = 1.3 Hz, 1H), 6.44 (dd, *J* = 3.2, *J* = 1.8 Hz, 1H), 6.34 - 6.30 (m, 2H), 6.13 (dd, *J* = 3.2 Hz, *J* = 1.8 Hz, 1H), 5.91 (s, 1H), 5.89 (d, *J* = 3.2 Hz, 1H), 5.29 (d, *J* = 14.2 Hz, 1H), 4.29 (d, *J* = 14.2 Hz, 1H), 4.25 - 4.20 (m, 1H), 4.04 (d, *J* = 15.0 Hz, 1H), 3.97 (septet, *J* = 6.8 Hz, 1H), 3.76 - 3.68 (m, 1H), 3.59 - 3.37 (m, 4H), 3.21 - 3.06 (m, 4H), 2.90 (septet, *J* = 6.6 Hz, 1H), 2.37 (septet, *J* = 6.8 Hz, 1H), 1.73 (d, *J* = 6.6 Hz, 3H), 1.61 (d, *J* = 6.6 Hz, 3H), 1.27 (d, *J* = 6.6 Hz, 6H), 1.18 (d, *J* = 6.9 Hz, 3H), 0.81 (d, *J* = 6.7 Hz, 3H), 0.66 (d, *J* = 6.9 Hz, 3H), 0.31 (d, *J* = 6.6 Hz, 3H) ppm.
$^{13}C$ NMR (100 MHz, $CD_2Cl_2$): δ = 300.4; 215.6; 212.8; 151.5; 150.2; 149.9; 148.1; 147.5; 147.4; 142.9; 142.6; 141.9; 141.5; 140.9; 138.9; 137.3; 136.6; 135.9; 129.7; 129.3; 129.1; 128.8; 128.7; 128.0; 128.0; 127.0; 124.2; 124.1; 123.8; 123.8; 117.8; 111.4; 110.8; 110.7; 108.8; 55.0; 54.6; 49.0; 47.9; 47.0; 45.0; 30.3; 28.7; 28.3; 27.5; 27.3; 26.8; 26.7; 26.6; 24.2; 23.1; 22.6; 22.0 ppm.
IR (KBr): *ν* = 3113, 3059, 3027, 2959, 2926, 2867, 1587, 1530, 1477, 1457, 1442, 1419, 1386, 1346, 1300, 1249, 1174, 1146, 1112, 1073, 1050, 1027, 1009, 983, 928, 883, 849, 800, 773, 756, 734, 695, 664, 641, 615, 598, 580, 564, 532, 472, 429 $cm^{-1}$.
Elemental Analysis for $C_{55}H_{62}Cl_2N_4O_2Ru$; calculated: %C, 67.20; %H, 6.36; %Cl, 7.21; %N, 5.70; found: %C, 67.28; %H, 6.31; %N, 5.56; %Cl, 7.16.

Structure of compound **1aa** was confirmed using X-ray structural analysis and it is presented on Figure 2.

## Example X

Synthesis of indenylidene precatalysts bearing two NHC ligands with thiophene $[(Tio\text{-}NHC\text{-}DiPP)_2RuCl_2(Ind)]$ (**1ab** according to Scheme 3)

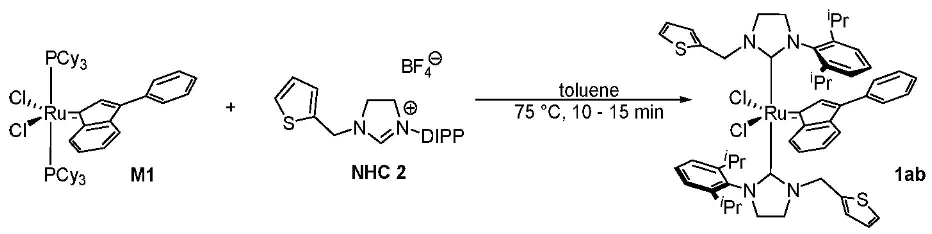

M1 NHC 2 1ab

The same procedure as described for **1aa** (in Example IX) using NHC 2 (221 mg 0.535 mmol), dry toluene (10 ml), potassium *tert*-pentoxide (0.3 ml 25% solution in toluene), Umicore **M1™** (224 mg, 0.243 mmol)], compound **1ab** was obtained as brick red solid (190 mg with 62% of yield).

$^1$H NMR (400 MHz, $CD_{29}Cl_2$): δ = 8.58 - 8.56 (m, 1H), 7.53 - 7.48 (m, 3H), 7.43 - 7.29 (m, 5H), 7.18 -7.15 (m, 2H), 7.11 - 7.08 (m, 2H), 7.07 - 7.03 (m, 2H), 6.97 (dd, *J* = 7.7 Hz, *J* = 1.5 Hz, 1H), 6.74 (dd, *J* = 5.1 Hz, *J* = 3.5 Hz, 1H), 6.69 - 6.66 (m, 2H), 6.60 (d, *J* = 3.4 Hz, 1H), 6.32 (t, *J* = 7.7 Hz, 1H), 5.86 (s, 1H), 5.44 (d, *J* = 14.2 Hz, 1H), 4.64 (d, *J* = 14.3 Hz, 1H), 4.26 (d, *J* = 14.3 Hz, 1H), 4.25 - 4.17 (m, 1H), 4.00 (septet, *J* = 6.7 Hz, 1H), 3.71 - 3.39 (m, 5H), 3.28 - 3.10 (m, 3H), 3.01 - 2.95 (m, 1H), 2.90 (septet, *J* = 6.6 Hz, 1H), 2.40 (septet, *J* = 6.7 Hz, 1H), 1.75 (d, *J* = 6.6 Hz, 3H), 1.61 (d, *J* = 6.6 Hz, 3H), 1.28 (d, *J* = 6.9 Hz, 3H), 1.26 (d, *J* = 6.6 Hz, 3H), 1.19 (d, *J*= 6.9 Hz, 3H), 0.81 (d, *J* = 6.7 Hz, 3H), 0.68 (d, *J* = 6.9 Hz, 3H), 0.34 (d, *J* = 6.6 Hz, 3H) ppm.

$^{13}$C NMR (100 MHz, $CD_2Cl_2$): δ = 300.5; 214.9; 212.5; 150.1; 148.2; 147.6; 147.5; 142.7; 141.6; 140.9; 140.7; 139.5; 139.0; 137.3; 136.8; 136.0; 129.6; 129.4; 129.0; 128.8; 128.8; 128.7; 128.0; 127.9; 127.5; 127.0; 126.7; 126.5; 126.4; 126.0; 124.3; 124.2; 123.9; 123.9; 117.9; 55.2; 54.4; 49.4; 48.5; 47.4; 46.8; 30.4; 28.8; 28.4; 27.8; 27.6; 26.9; 26.6; 26.6; 24.3; 23.2; 22.8; 22.1 ppm.

IR (KBr): *v* = 3059, 2962, 2925, 2898, 2866, 1702, 1586, 1533, 1475, 1441, 1389, 1361, 1340, 1300, 1248, 1212, 1177, 1138, 1072, 1050, 1025, 982, 926, 903, 850, 802, 774, 755, 708, 639, 617, 580, 560, 531, 486, 467, 426 $cm^{-1}$.

Elemental Analysis for $C_{55}H_{62}Cl_2N_4RuS_2$; calculated: %C, 65.07; %H, 6.16; %N, 5.52; %S, 6.32; %Cl, 6.98; found: %C, 65.33; %H, 5.95; %N, 5.42; %S, 6.28; %Cl, 6.77.

Structure of compound **1ab** was confirmed using X-ray structural analysis and it is presented on Figure 3.

## Example XI

Synthesis of indenylidene type of precatalyst bearing two NHC lignads with benzothiophene [(BTio-NHC-DiPP)$_2$RuCl$_2$(Ind)] (**1ac** according to Scheme 3).

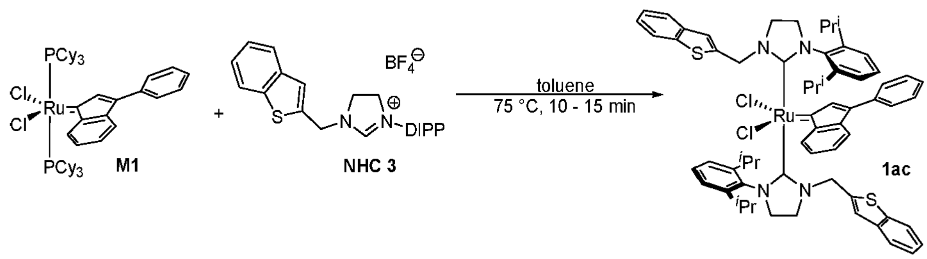

M1 NHC 3 1ac

The same procedure as described for **1aa** (in Example IX) using **NHC 3** (497 mg 1.07 mmol), dry toluene (20 ml), potassium *tert*-pentoxide (0.6 ml 25% solution in toluene), Umicore **M1™** (449 mg, 0.486 mmol), and compound **1ac** was obtained as red brick solid (358 mg with 66% of yield).

$^1$H NMR (400 MHz, $CD_2$-$Cl_2$): δ = 8.62 - 8.59 (m, 1H), 7.92 - 7.85 (m, 2H), 7.68 - 7.65 (m, 1H), 7.53 - 7.48 (m, 4H), 7.44 - 7.36 (m, 4H), 7.33 - 7.28 (m, 3H), 7.22 - 7.19 (m, 2H), 7.17 - 7.15 (m, 2H), 7.03 - 7.00 (m, 1H), 6.97 - 6.93 (m, 1H), 6.79 (s, 1H), 6.70 - 6.68 (m, 2H), 6.33 (t, *J* = 7.7 Hz, 1H), 5.90 (s, 1H), 5.53 (d, *J* = 14.3 Hz, 1H), 4.76 (d, *J* = 14.4 Hz, 1H), 4.34 (d, *J* = 14.5 Hz, 1H), 4.26 - 4.18 (m, 1H), 4.02 (septet, *J* = 6.7 Hz, 1H), 3.74 - 3.41 (m, 5H), 3.37 - 3.34 (m, 1H), 3.31 (d, *J* = 14.5 Hz, 1H), 3.26 - 3.18 (m, 1H), 3.09 - 3.04 (m, 1H), 2.99 (septet, *J* = 6.7 Hz, 1H), 2.43 (septet, *J* = 6.6 Hz, 1H), 1.79 (d, *J* = 6.6 Hz, 3H), 1.64 (d, *J* = 6.6 Hz, 3H), 1.35 (d, *J* = 6.5 Hz, 3H), 1.28 (d, *J* = 6.8 Hz, 3H), 1.20 (d, *J* = 6.9 Hz, 3H), 0.84 (d, *J* = 6.7 Hz, 3H), 0.69 (d, *J* = 6.9 Hz, 3H), 0.36 (d, *J* = 6.6 Hz, 3H) ppm.

$^{13}$C NMR (100 MHz, $CD_2Cl_2$): δ = 300.7; 215.3; 212.9; 150.2; 148.2; 147.6; 147.5; 142.7; 142.1; 141.5; 141.2; 141.0; 141.0; 140.8; 139.8; 139.7; 138.9; 137.2; 136.7; 136.0; 129.7; 129.5; 129.1; 128.8; 128.8; 128.1; 127.9; 127.0; 125.5; 124.6; 124.5; 124.3; 124.3; 124.2; 123.9; 123.9; 123.9; 123.4; 122.8; 122.5; 117.9; 55.3; 54.5; 50.3; 48.8; 47.8; 47.7; 30.4; 28.8; 28.4; 28.0; 27.6; 26.9; 26.6; 26.6; 24.3; 23.1; 23.0; 22.0 ppm.

IR (KBr): $v$ = 3057, 2960, 2926, 2899, 2866, 1586, 1533, 1475, 1458, 1439, 1384, 1350, 1294, 1253, 1175, 1154, 1118, 1054, 1027, 929, 901, 852, 802, 774, 748, 727, 696, 644, 615, 574, 530, 484, 463, 428 $cm^{-1}$.
Elemental Analysis for $C_{63}H_{66}Cl_2N_4RuS_2$; calculated: %C, 67.84; %H, 5.96; %N, 5.02; %S, 5.75; %Cl, 6.36; found: %C, 67.87; %H, 6.09; %N, 4.97; %S, 5.66; %Cl, 6.16.

Structure of compound **1ac** was confirmed using X-ray structural analysis and it is presented on Figure 4.

**Example XII**

Synthesis of Hoveyda type of precatalyst bearing one NHC ligand with thiophene [(Tio-NHC-Mes)$RuCl_2$(Hov)] (**1ba** according to Scheme 3).

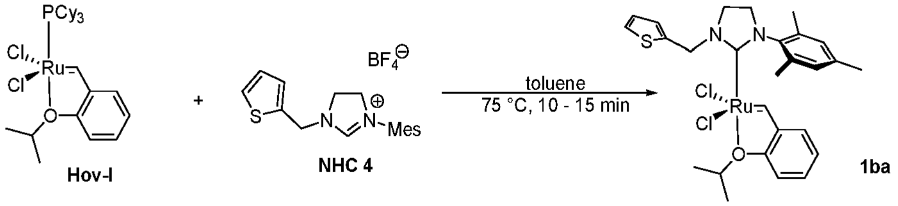


Hov-I + NHC 4 → 1ba

Salt **NHC 4** (265 mg, 0.713 mmol) and dry toluene (20 ml) were placed in the Schlenk vessel under argon. Potassium *tert*-pentoxide (0.4 ml 25% solution in toluene) was added at 75°C and stirred for 1 minute. Then, complex **Hov-I** (389 mg, 0.648 mmol) was added to the reaction mixture and stirred for 5 minutes. The reaction was monitored using TLC chromatography. After 5 minutes, CuCl (64.8 mg, 0.648 mmol) was added to the reaction mixture, and stirred for 5 minutes. The reaction mixture was filtered off through small amount of silica gel on the G4 fritted glass funnel under reduced pressure, additionally using for elution 75% solution of cyclohexane/ethyl acetate at temperature of 50°C. The filtrate was concentrated and crystallized from a mixture of $CH_2Cl_2/CH_3OH$. Methanol was decanted and crystallization was repeated, methanol from above product was discarded. The residue was dried on the rotary evaporator under reduced pressure, and then etyl acetal was added at 50°C until the entire quantity of green product was dissolved. The solution was quickly filtered through cotton, and then solvent was evaporated on rotary evaporator. The residue was crystallized three times from the mixture of $CH_2Cl_2/CH_3OH$, on rotary evaporator, to separate unreacted substrate - **Hov-I.** Methanol from the above the crystals was decanted, the crystals were washed with additional portion of methanol and decanted. Product was pre dried on the rotary evaporator under reduced pressure, and then under reduced pressure, to afford 252 mg compound **1ba** as a green solid powder, that is 64% theoretical yield.
$^1H$ NMR (400 MHz, $CD_2Cl_2$): $\delta$ = 16.13 (s, 1H), 7.57 (ddd, $J$ = 8.8 Hz, $J$ = 6.7 Hz, $J$ = 2.4 Hz, 1H), 7.40 (dd, $J$ = 5.1 Hz, $J$ = 1.2 Hz, 1H), 7.35 (d, $J$ = 3.4 Hz, 1H), 7.11 (s, 2H), 7.08 (dd, $J$ = 5.1 Hz, $J$ = 3.5 Hz, 1H), 7.02 - 6.95 (m, 3H), 5.78 (s, 2H), 5.19 (septet, $J$ = 6.1 Hz, 1H), 3.96 - 3.91 (m, 2H), 3.78 - 3.73 (m, 2H), 2.47 (s, 3H), 2.22 (s, 6H), 1.72 (d, $J$ = 6.1 Hz, 6H) ppm.
$^{13}C$ NMR (100 MHz, $CD_2Cl_2$): $\delta$ = 290.5; 209.8; 152.7; 144.0; 139.4; 139.2; 138.3; 138.0; 129.9; 128.5; 127.1; 126.9; 122.9; 122.5; 113.3; 75.6; 52.3; 51.0; 48.1; 22.2; 21.3; 18.1 ppm.
IR (KBr): $v$ = 3077, 3061, 3026, 2979, 2917, 2882, 1953, 1913, 1834, 1731, 1606, 1589, 1574, 1483, 1451, 1415, 1382, 1335, 1303, 1293, 1271, 1246, 1219, 1192, 1162, 1154, 1144, 1109, 1094, 1036, 964, 934, 903, 878, 853, 840, 796, 787, 772, 752, 732, 704, 665, 644, 616, 582, 570, 480, 438 $cm^{-1}$.
Elemental Analysis for $C_{27}H_{32}Cl_2N_2ORuS$; calculated: %C, 53.64; %H, 5.33; %N, 4.63; %S, 5.30; %Cl, 11.73; found: %C, 53.39; %H, 5.44; %N, 4.38; %S, 5.39; %Cl, 11.87.

Structure of compound **1ba** was confirmed using X-ray structural analysis and it is presented on Figure 5.

**Example XIII**

Synthesis of Hoveyda type precatalyst bearing one NHC ligand with thiophene [(Tio-NHC-DiPP)$RuCl_2$(Hov)] (**1bb** according to Scheme 3).

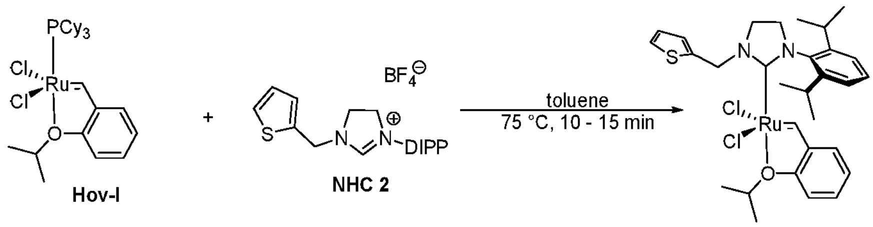


Hov-I NHC 2 1bb

The same procedure as described for **1ba** (in Example XI) using salt **NHC 2,** apart from the product's isolation method. According to which, the filtrate was concentrated to a small volume and the product was isolated by using silica gel column chromatography, with 5% ethyl acetate/cyclohexane as eluent, and then 20% ethyl acetate/cyclohexane. After that, the collected fractions were concentrated on the rotary evaporator under reduced pressure, and the resulting residues were dissolved in ethyl acetate, filtered off through cotton, and solvent was evaporated on the rotary evaporator, the resulting residue was crystallized from the mixture of solvents $CH_2Cl_2/CH_3OH$ and then proceeded as in Example XI, using salt **NHC 2** (295 mg 0.713 mmol), toluene (20 ml), potassium *tert*-pentoxide (0.4 ml 25% solution in toluene), **Hov-I** (389 mg, 0.648 mmol) and CuCl (64.8 mg, 0.648 mmol). Compound **1bb** was obtained as green solid (320 mg with 76% of yield).

$^{1}$H NMR (400 MHz, $CD_2Cl_2$): $\delta$ = 16.11 (s, 1H), 7.63 (t, *J* = 7.8 Hz, 1H), 7.54 (ddd, *J* = 8.4 Hz, *J* = 7.1 Hz, *J* = 1.9 Hz, 1H), 7.42 - 7.40 (m, 3H), 7.38 - 7.36 (m, 1H), 7.09 (dd, *J* = 5.1 Hz, *J* = 3.5 Hz, 1H), 7.00 (d, *J* = 8.4 Hz, 1H), 6.95 - 6.88 (m, 2H), 5.82 (s, 2H), 5.18 (septet, *J* = 6.1 Hz, 1H), 3.95 - 3.90 (m, 2H), 3.79 - 3.74 (m, 2H), 3.13 (septet, *J* = 6.8 Hz, 2H), 1.75 (d, *J = 6.1* Hz, 6H), 1.22 (d, *J = 6.9* Hz, 6H), 0.86 (d, *J = 6.7* Hz, 6H) ppm.

$^{13}$C NMR (100 MHz, $CD_2Cl_2$): $\delta$ = 288.1; 210.3; 153.0; 148.8; 143.5; 139.2; 137.7; 130.0; 129.7; 128.6; 127.1; 126.9; 125.2; 122.8; 122.2; 113.3; 75.7; 55.6; 51.3; 48.0; 28.2; 25.7; 23.9; 22.4 ppm.

IR (KBr): *v* = 3084, 3062, 3024, 2987, 2965, 2923, 2886, 2865, 1955, 1916, 1872, 1585, 1573, 1479, 1454, 1444, 1425, 1383, 1372, 1344, 1306, 1292, 1271, 1249, 1234, 1217, 1210, 1183, 1155, 1145, 1110, 1093, 1053, 1034, 962, 932, 879, 853, 840, 806, 795, 764, 753, 737, 665, 641, 622, 607, 575, 477, 442, 431 $cm^{-1}$.

Elemental Analysis for $C_{30}H_{38}Cl_2N_2ORuS$; calculated: %C, 55.72; %H, 5.92; %N, 4.33; %S, 4.96; %Cl, 10.96; found: %C, 55.68; %H, 5.71; %N, 4.34; %S, 4.99; %Cl, 10.78.

Structure of compound **1bb** was confirmed using X-ray structural analysis and it is presented on Figure 6.

**Example XIV**

The study of catalytic activity in RCM of diethyl diallvlmalonate (S1). $^{1}$H NMR method.

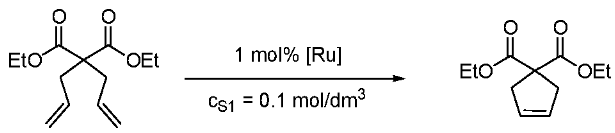


S1 P1

Procedure A. Samples preparation in dry solvents, methylene chloride and toluene under argon atmosphere. Solution of substrate **S1** (140 mg, 583,4 μmol) in dry methylene chloride or toluene under argon atmosphere was prepared in a measuring flask of 5 ml volume fitted with septa. In another measuring flask of 1 ml volume, solution of precatalyst (7.0 μmol) was prepared. Next, solution of substrate (0.6 ml, 70 μmol), was placed in a NMR tube fitted with septa under argon and then precatalyst (100 μl, 0.7 μmol) was added.

Procedure B. Samples preparation in toluene HPLC (Sigma-Aldrich) without using protective gas. Solution of substrate **S1** (140 mg, 583.4 μmol) in toluene HPLC was prepared in a measuring flask of 5 mL volume. In another measuring flask of 1 ml volume, solution of precatalyst (7.0 μmol) was prepared. Next, solution of substrate (0.6 ml, 70 μmol), was placed in a NMR tube, then precatalyst (100 μl, 0.7 μmol) was added and tube was closed with cap.

Procedure C. Samples preparation in dry 2-methyltetrahydrofuran under argon atmosphere. Solution of precatalyst (5.834 μmol) under argon atmosphere in dry 2-methyltetrahydrofuran was prepared in a measuring flask of 5 ml volume fitted with septa. In another measuring flask of 1 ml volume, solution of substrate **S1** (168 mg, 700 μmol) was prepared. Next, solution of substrate **S1** (100 μl, 70 μmol) was placed in NMR tube fitted with septa, and then solution of precatalyst (0.6 ml, 0.7 μmol) was added. In this procedure, a new solvent was used (2-methyltetrahydrofuran), which

is obtained from agricultural waste, such as oat hulls, corn cobs, or bagasse remaining after removal of juice, oil or plant material. Use of this solvent allows carrying out the reaction of olefin metathesis in the environment friendly reaction media instead of toxic chlorinated and/or aromatic solvents.

Measurement of the RCM reaction profile for S1: The time measurement was started just after precatalyst addition, NMR tube was placed in NMR apparatus and $^1$H NMR spectrum was recorded using "array" function. Recorded FID file was interpreted in the MesteNova programme, integrating methylene signals for **S1** $\delta_{H[ppm]}$ = 2,73 (dublet) and **P1** $\delta_{H[ppm]}$ = 3,04 (singlet). Yield was calculated based on the following formula :

$$\text{Yield (\%)} = \frac{[\text{product}]}{[\text{product}] + [\text{substrate}]} \times 100\%$$

Attention: Shifts of methylene signal for **S1** and **P1** are given for indication purposes, due to lack of the characteristic reference signal on the NMR spectrum, which normally is the residual signal of the deuterated solvent.

The course of kinetic profiles in the RCM reaction of diethyl diallylmalonate (DEDAM) catalyzed of selected ruthenium complexes has been illustrated on Figures from Fig. 7 to Fig. 12:

Figure 7 presents course of kinetic profiles in RCM reaction of DEDAM catalyzed by selected ruthenium complexes at 90°C in dry toluene carried out under argon (procedure A). Eight ruthenium complexes were studied. Ruthenium complexes commercially available (**M2™**, **Hov-II,** and **HovSIPr**) in the similar conditions catalysed reaction very fast in the uncontrolled manner, similarly new Hoveyda type complex (**1ba**) is very active, just about 2 minutes is enough to finish the reaction quantitatively. Using new ruthenium complexes (**1bb, 1ab, 1aa,** and **1ac**) reaction is carried out in the controlled manner, because end of the reaction took place after significantly longer period of time (about 10, 20, 40 and 50 minutes respectively) in the quantitatively manner.

Figure 8 presents course of kinetic profiles in RCM reaction of DEDAM catalyzed by selected ruthenium complexes at 90°C in commercially available non-dried toluene performer on the air (procedure B). On the Figure course of reaction catalysed by three selected bisNHC indenylidene ruthenium complexes (**1aa, 1ab,** and **1ac**) are presented. On the figure, it is shown controlled activity at high temperature. Among them, within the first 30 minutes the most active precatalyst was **1aa** (with furan group), less active was precatalyst **1ac** (with benzothiophene group), and the least active was precatalyst **1ab** (with thiophene group). Novel ruthenium complexes were studied, and they maintain their good catalytic properties despite the lack of inert gas atmosphere, as well as using non-dried solvent (containing water) toluene.

Figure 9 presents course of kinetic profiles in RCM reaction of DEDAM catalyzed by selected ruthenium complexes at 70°C in dry toluene carried out under argon (procedure A). Eight ruthenium complexes were studied. Ruthenium complexes commercially available (**M2, Hov-II,** and **HovSIPr**) in the similar conditions catalysed reaction very fast in the uncontrolled manner. New Hoveyda type complex (**1ba**) is very active, but better controlled than complexes available on the market. Using new ruthenium complexes (**1aa, 1ab, 1ac,** and **1bb**) reaction is carried out in the controlled manner, because the end of the reaction is after an extended time period (even over 200 minutes), that confirmed high stability of newly obtained complexes in the reaction medium.

Figure 10 presents course of kinetic profiles in RCM reaction of DEDAM catalyzed by selected ruthenium complexes at 70°C in commercially available non-dried toluene performer on the air (procedure B). The figure shows course of four reaction catalyzed by selected ruthenium Hoveyda type complexes (**1ba, 1bb, Hov-II,** and **HovSIPr**), the figure presents their controlled activity at a elevated temperature. Among them, commercially available ruthenium complexes (**Hov-II** and **HovSIPr**) finish reaction in less than 5 minutes, whereas novel Hoveyda type complexes (**1ba** and **1bb**) reach full conversion of the reaction much later (after about 10 and 40 minutes respectively). Novel ruthenium complexes were studied, and they maintain their good catalytic properties despite the lack of inert gas atmosphere, as well as using non-dried solvent (containing water) toluene.

Figure 11 presents course of kinetic profiles in RCM reaction of DEDAM catalyzed by selected ruthenium complexes at 50°C in toluene carried out under argon (procedure A). Four selected ruthenium complexes were studied. Commercially available ruthenium complexes (**Hov-II** and **HovSIPr**) in the said reaction conditions, catalyzed reactions fast and in an uncontrolled manner, novel Hoveyda type complex **1ba** is active, and better controlled than ruthenium complexes on the market (completion of the process after about 30 minutes). On the other hand, the best-controlled ruthenium complex is complex **1bb,** for which a completion of studied reaction was observed after about 150 minutes.

Figure 12 presents course of kinetic profiles in RCM reaction of DEDAM catalyzed by selected ruthenium complexes at 50°C in 2-methyltetrahydrofuran carried out under argon (procedure C). Four selected ruthenium complexes (**1ba, 1bb, Hov-II** and **HovSIPr**) were studied. Commercially available ruthenium complexes in the said conditions, catalyzed reactions fast and in an uncontrolled manner, novel Hoveyda type complex **1ba** is active, and better controlled than ruthenium complexes on the market (completion of the process after about 30 minutes). On the other hand, the best-controlled ruthenium complex is complex **1ba**, for which a completion of studied reaction was observed after about 150 minutes.

**Example XV**

The study of catalytic activity in dRRM of diene (**S2**) on the air. GC method

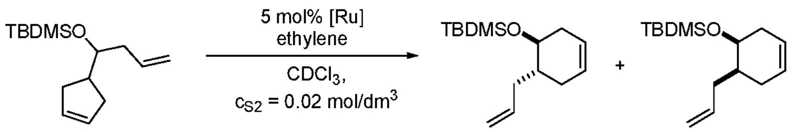


*trans* *cis*

**S2** **P2-trans** **P2-cis**

Substrate **S2** (5.89 mg, 23 μmol), durene (1,2,4,5-tetramethylbenzene; 1.57 mg, 11.6 μmol) were placed in the measuring flask of 1 ml volume and added $CDCl_3$ to the mark. In another measuring flask of 1 ml volume, solution of a precatalyst (7.0 μmol) was prepared. Then, solution of substrate **S2** (0.6 ml, 14 μmol) was placed in NMR tube fitted with septum, and cooled it down in the cooling bath and bubbling through ethylene. Then, solution of a precatalyst (100 μl, 0.7 μmol) was added at room temperature (Hoveyda complexes: **1ba** and **1bb**) or 50°C (bisNHC indenylidene complexes: **1aa, 1ab** and **1ac**). Then, conversion and proportion of *trans*/*cis* isomers were measured with GC method, using the following programme of heating the oven: 120°C for 1 minute, then increase of temperature 10°C/min to 200°C and next increase 20°C/min to 270°C. Under these conditions, the retention times are as follows: $t_{duren}$ = 4,90 [minutes], $t_{S2}$ = 8,43 [minutes], $t_{P3}$ = 8,51 [minutes], $t_{P2}$ = 8,72 [minutes].

In the reaction study using ruthenium complexes **1aa, 1ab** and **1ac,** after addition of substrate at 50°C there was no progress of the reaction for about 1.5 hours, that indicates dormant character of these precatalysts at the initial time of the reaction.

Table 1.

| Pre catalyst | Temperature [°C] | Time [hours] | Conversion [%] | *trans*/*cis* |
|---|---|---|---|---|
| **1ba** | RT | 17 | 95 | 3.5 |
| **1bb** | RT | 17 | 73 | 2.1 |
| **Hov-II** | RT | 17 | >99 | 2.7 |
| **HovSIPr** | RT | 17 | >99 | 1.6 |
| **M2** | RT | 17 | >99 | 2.0 |
| **M2** | 50 | 1 | >99 | 3.3 |
| **1ab** | 50 | 17 | 95 | 2.4 |
| **1aa** | 50 | 17 | 91 | 2.1 |
| **1ac** | 50 | 17 | 78 | 2.5 |
| **1ab** | 50 | 24 | >99 | 2.9 |
| **1aa** | 50 | 24 | 95 | 2.1 |
| **1ac** | 50 | 24 | 86 | 2.4 |

## Example XVI

The study of catalytic activity in RCM reaction (using **S3** and **S4**) as well as in RCEYM reaction(**S5**), GC methods.

An appropriate substrate **S3-S5** (0.1 mmol), 1,2,4,5-tetramethylbenzene (13.4 mg, 0.1 mmol) and toluene HPLC (**S3**, **S4**: 0.9 ml, **S5**: 0.8 ml) were placed in round bottom flak of 5 mL volume. Then, the mixtures were heated to 50°C (for complexes **1ba, 1bb**) or 90°C (for complexes **1aa, 1ab**, **1ac**) and solution of precatalyst (for **S3**, **S4**: 100 μl, 0.001 mmol; **S5**: 200 μl, 0.002 mmol) was added. In the appropriate time intervals, a sample (50 μl) of the reaction mixture was collected in a vial, ethyl vinyl ether (100 μl) and DCM (500 μl) were added. Conversion was measured with GC methods, using the following programme of heating the oven: 100°C for 1 minute, then increase of temperature 10°C/min to 300°C and after reaching it, the temperature was hold for 9 minutes. Under these conditions, the retention times are as follows: $t_{duren}$ = 6,33 [minutes], $t_{S3}$ = 15,51 [minutes], $t_{P4}$ = 16,16 [minutes], $t_{S4}$ = 17,24 [minutes], $t_{P5}$ = 18,16 [minutes], $t_{S5}$ = 14,58 [minutes], $t_{P6}$ = 16,24 [minutes].

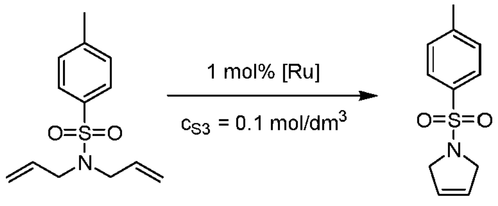


**S3** **P3**

Table 2.

| Substrate | Product | Precatalyst | Temperature [°C] | Time [min.] | Coversion [%] |
|---|---|---|---|---|---|
| | | **1ab** | 90 | 5 | 30 |
| | | | 90 | 10 | 60 |
| | | | 90 | 30 | 99 |
| | | | 90 | 60 | >99 |
| | | **1aa** | 90 | 5 | 55 |
| | | | 90 | 10 | 87 |
| | | | 90 | **30** | **>99** |
| | | **1ac** | 90 | 5 | 27 |
| | | | 90 | 10 | 56 |
| | | | 90 | 30 | 98 |
| | | | 90 | **60** | **>99** |

(continued)

| Substrate | Product | Precatalyst | Temperature [°C] | Time [min.] | Coversion [%] |
|---|---|---|---|---|---|
| 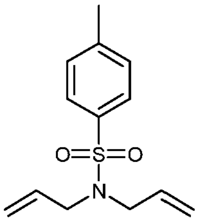 S3 | 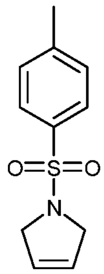 P3 | **Ind-II** | 50 | 5 | 17 |
| | | | 50 | 10 | 21 |
| | | | 50 | 30 | 53 |
| | | | 50 | 60 | 81 |
| | | | 50 | 120 | 93 |
| | | | 50 | **180** | **96** |
| | | **1ba** | 50 | 5 | 46 |
| | | | 50 | 10 | 65 |
| | | | 50 | 30 | 87 |
| | | | 50 | 60 | 93 |
| | | | 50 | 120 | 97 |
| | | | 50 | **180** | **98** |
| | | **1bb** | 50 | 5 | 3 |
| | | | 50 | 10 | 9 |
| | | | 50 | 30 | 45 |
| | | | 50 | 60 | 72 |
| | | | 50 | 120 | 91 |
| | | | 50 | 180 | 96 |
| | | | 50 | 240 | 98 |
| | | | 50 | **300** | **99** |
| | | **Hov-II** | 50 | 5 | 86 |
| | | | 50 | 10 | 90 |
| | | | 50 | 30 | 93 |
| | | | 50 | **60** | **94** |
| | | **HovSiPr** | 50 | 5 | 77 |
| | | | 50 | 10 | 93 |
| | | | 50 | **30** | **>99** |

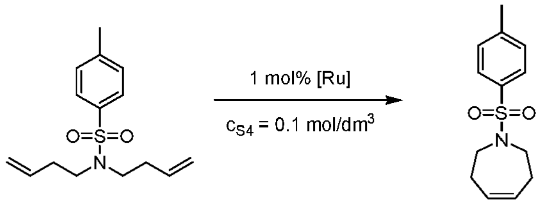


S4 P4

Table 3.

| Substrate | Product | Pre catalyst | Temperature [°C] | Time [min.] | Coversion [%] |
|---|---|---|---|---|---|
| 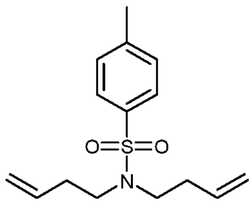 **S4** | 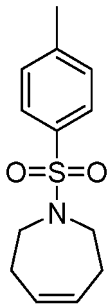 **P4** | **1ab** | 90 | 5 | 66 |
| | | | 90 | 10 | 95 |
| | | | 90 | **30** | **>99** |
| | | **1aa** | 90 | 5 | 60 |
| | | | 90 | 10 | 90 |
| | | | 90 | **30** | **>99** |
| | | **1ac** | 90 | 5 | 50 |
| | | | 90 | 10 | 85 |
| | | | 90 | **30** | **>99** |
| | | **Ind-II** | 50 | 5 | 45 |
| | | | 50 | 10 | 54 |
| | | | 50 | 30 | 95 |
| | | | 50 | **60** | **>99** |
| | | **1ba** | 50 | 5 | 77 |
| | | | 50 | 10 | 89 |
| | | | 50 | 30 | 98 |
| | | | 50 | 60 | >99 |
| | | **1bb** | 50 | 5 | 13 |
| | | | 50 | 10 | 43 |
| | | | 50 | 30 | 84 |
| | | | 50 | 60 | 95 |
| | | | 50 | 120 | 98 |
| | | | 50 | 180 | >99 |
| | | **Hov-II** | 50 | 5 | 99 |
| | | | 50 | 10 | >99 |
| | | **HovSIPr** | 50 | 5 | >99 |

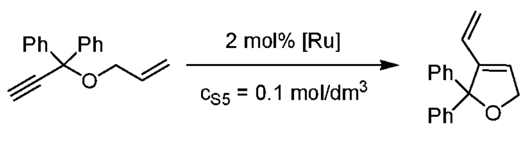


**S5** **P5**

Table 4.

| Substrate | Product | Pre catalyst | Temperature [°C] | Time [min.] | Conversion (%) |
|---|---|---|---|---|---|
| 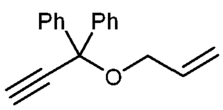 **S5** | 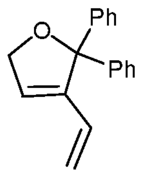 **P5** | **1ba** | 50 | 5 | 3 |
| | | | 50 | 10 | 7 |
| | | | 50 | 30 | 18 |
| | | | 50 | 60 | 30 |
| | | | 50 | 120 | 36 |
| | | | 50 | 180 | 41 |
| | | | **50** | **240** | **42** |
| | | **1bb** | 50 | 5 | 0 |
| | | | 50 | 10 | 0 |
| | | | 50 | 30 | 4 |
| | | | 50 | 60 | 9 |
| | | | 50 | 120 | 20 |
| | | | 50 | 180 | 28 |
| | | | **50** | **240** | **36** |
| | | | 50 | 300 | 42 |
| | | | 50 | 1200 | 86 |
| | | | 50 | 1440 | 89 |
| | | **Hov-II** | 50 | 5 | >99 |
| | | **HovSIPr** | 50 | 5 | 93 |
| | | | 50 | 10 | 96 |
| | | | 50 | 30 | 99 |
| | | **Ind-II** | 50 | 5 | 34 |
| | | | 50 | 10 | 86 |
| | | | 50 | **30** | **>99** |
| | | **1ab** | 90 | 5 | 21 |
| | | | 90 | 10 | 40 |
| | | | 90 | 30 | 90 |
| | | | 90 | **60** | **>99** |
| | | **1aa** | 90 | 5 | 24 |
| | | | 90 | 10 | 45 |
| | | | 90 | 30 | 85 |
| | | | 90 | 60 | 95 |
| | | | 90 | 120 | 98 |
| | | | 90 | **180** | **>99** |
| | | **1ac** | 90 | 5 | 17 |
| | | | 90 | 10 | 37 |
| | | | 90 | 30 | 82 |
| | | | 90 | 60 | 96 |

(continued)

| Substrate | Product | Pre catalyst | Temperature [°C] | Time [min.] | Conversion (%) |
|---|---|---|---|---|---|
| | | | 90 | 120 | **>99** |

Example XVII

The study of the catalytic activity of new precatalysts in CM reaction. GC methods

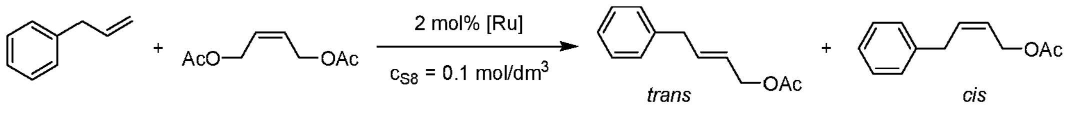


**S6** **S7** **P6** **P7**

Compound **S6** (100 mg, 0.83 mmol), compound **S7** (286 mg, 1.66 mmol) and toluene HPLC (7 ml) were placed in 25 mL round bottom flask. The solution was heated to 50°C or 90°C, and then solution of precatalyst in toluene HPLC (1 ml, 0.0166 mmol) was added. After 4 hours the reaction mixture was immediately cooled down to room temperature, solvent was evaporated and products **P6** and **P7** were isolated on column chromatography with silica gel, using as eluent 10% ethyl acetate/cyclohexane. Products were identified on TLC plate using water solution of $KMnO_4$. Solvents were evaporated, and the residue was dried under reduced pressure. The product mass was weighted, yield was calculated and proportion of *trans*/*cis* isomers was analysed using GC, using the following programme of heating the oven: 120°C during 1 minute, then increase of temperature 5°C/min to 170°C, then increase 10°C/min to 250°C and finally increase 15°C/min to 270°C. Under these conditions the retention times are as follows : $t_{P10}$ = 12,41 [min], $t_{P9}$ = 12,51 [min].

Table 5.

| Substrate | Product | Precatalyst | Temperature [°C] | Time [hours] | Yield [%] |
|---|---|---|---|---|---|
| 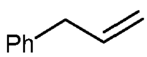 S6 + 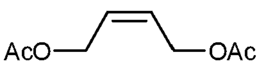 S7 | 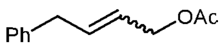 P6/P7 | **1ba** | 50 | 5 | 68%, *E/Z* = 3,7 |
| | | **1bb** | 50 | 5 | 46%, *E/Z* = 2,4 |
| | | **1bb** | 50 | 24 | 48%, *E/Z* = 2,4 |
| | | **Hov-II** | 50 | 5 | 76%, *E/Z* = 9,3 |
| | | **HovSiPr** | 50 | 5 | 83%, *E/Z* = 8,3 |
| | | **Ind-II** | 50 | 3 | 75%, *E/Z* = 9,4 |
| | | **1ab** | 90 | 4 | 36%, *E/Z* = 2,1 |
| | | **1aa** | 90 | 4 | 36%, *E/Z* = 1,9 |
| | | **1ac** | 90 | 4 | 32%, *E/Z* = 2,0 |

**Example XVIII**

The study of catalytic activity of new precatalysts in the ethenolysis reaction. GC methods.

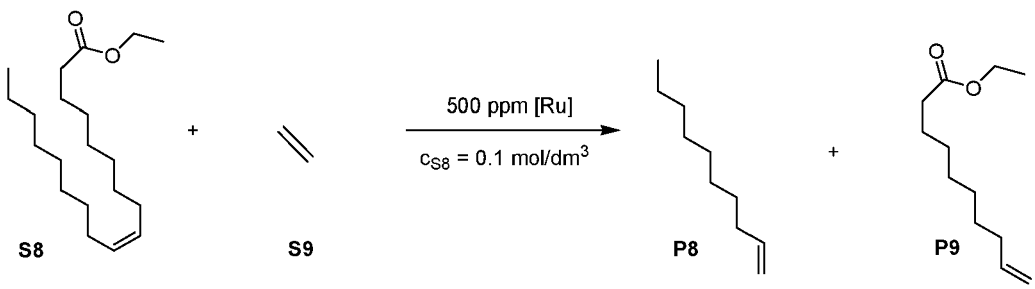

S8 + S9 → P8 + P9

Ethyl oleate 4.66 g (15 mmol) 0.8 ml tetradecane as a internal standard were placed in the pad of the autoclave. Before reaction, ethyl oleate was filtered off through thin pad of aluminum oxide (neutral, activity I) of about 2 cm height, diameter of column/glass tube of about 1 cm (without need of additional solvents). Then, two drops of the reaction mixture was taken, placed in GC vial and refilled with methylene chloride (this sample was used to record chromatogram before the ethenolysis reaction - ie. "blank sample"). Solution of the precatalyst (0.1 ml, 0.0075 mmol, 500 ppm) in dry methylene chloride was added using Hamilton syringe to ethyl oleate in the pad of the autoclave. After placing the pad in the autoclave the system was flushed three times with ethylene of about 10 bar pressure (by cyclic filling the autoclave with

ethylene to 10 bar and releasing ethylene from the autoclave). The ethenolysis reaction was performed from 3 to 72 hours (see Table 6) at temperature from 40 to 100°C (see Table 6) under constant pressure 10 bar (with a constant flow of ethylene, ie. line autoclave-gas cylinder was open - equipped with a reducer). After that time, ethylene was released from the autoclave. 1 mL of the reaction mixture was taken, and 4 ml (2M) of ethyl-vinyl ether solution in methylene chloride was added. From this solution, sample of 0.2 ml were collected in GC vial and methylene chloride was added to an overall volume of 1 ml. Then, each chromatogram was recorded twice, determining the composition of the sample (using an internal standard calibration) and the reaction parameters such as conversion and selectivity.

Table 6. Ethyl oleate ethenolysis

| Precatalyst [500 ppm] | Time [hours] | Temperature [°C] | Conversion S8 [%] | Yield P8. [%] | Yield P9. [%] | Unreacted S8 [%] | The molar fraction of ethanolysis products in the reaction mixture - GC | The molar fraction of homometatezy products in the reaction mixtures - GC | The mole fraction of substrate S8 in the reaction mixtures - GC |
|---|---|---|---|---|---|---|---|---|---|
| 1ab | 12 | 100 | 77 | 41 | 48 | 23 | 0,66 | 0,17 | 0,17 |
| Ind-III | 3 | 50 | 82 | 45 | 55 | 17 | 0,74 | 0,13 | 0,13 |
| Ind-III | 3 | 50 | 49 | 6 | 8 | 51 | 0,12 | 0,45 | 0,43 |
| Ind-III | 3 | 50 | 41 | 6 | 7 | 58 | 0,12 | 0,37 | 0,51 |
| 1ba | 3 | 50 | 61 | 37 | 43 | 39 | 0,59 | 0,12 | 0,29 |
| 1ab | 3 | 50 | 56 | 34 | 42 | 43 | 0,55 | 0,13 | 0,31 |
| 1ab | 3 | 90 | 65 | 39 | 47 | 35 | 0,63 | 0,11 | 0,26 |
| 1aa | 12 | 100 | 61 | 34 | 42 | 38 | 0,64 | 0,04 | 0,33 |
| 1ac | 12 | 100 | 53 | 7 | 8 | 47 | 0,14 | 0,44 | 0,42 |
| Ind-I | 3 | 50 | 68 | 34 | 41 | 32 | 0,54 | 0,23 | 0,23 |
| Hov-II | 3 | 50 | 37 | 22 | 28 | 62 | 0,41 | 0,07 | 0,51 |
| 1ab | 48 | 50 | 19 | 18 | 22 | 81 | 0,33 | 0,01 | 0,66 |
| 1ab | 72 | 40 | 67 | 50 | 65 | 32 | 0,75 | 0,04 | 0,21 |
| Gru-I | 3 | 50 | 65 | 39 | 47 | 35 | 0,63 | 0,11 | 0,26 |
| HovSIPr | 3 | 50 | 50 | 28 | 37 | 50 | 0,56 | 0,02 | 0,42 |
| HovSIPr | 3 | 50 | 82 | 45 | 55 | 17 | 0,74 | 0,13 | 0,13 |

Ethenolysis reaction is planned reaction of cross metathesis between substartes **S8** and S9.

Homometathesis reaction is undesired side reaction, in which substrate **S8** reacts with another molecule of substrate **S8** to form octadec-9-ene and diethyl ester of 1,18-dicarboxyloctadec-9-ene.

**Example XIX**

The study of catalytic activity of new precatalysts in ROMP polymerization.

Procedure A : synthesis of polydicyclopentadiene (pDCPD)

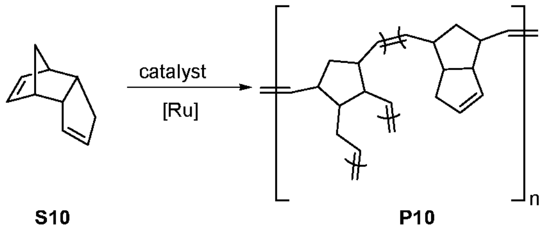


S10 P10

Dicyclopentadiene (1 g, 7.56 mmol) was placed in the vial on the air, which was melted before in the temperature of 35°C. Then, at temperature of 60°C precatalyst **1bb** (7.43 mg, 0,00756 mmol, 0.1 mol%) was added in 0.2 ml methylene chloride or **1bb** (4.89 mg, 0.00756 mmol, 0.1 mol%) in 0.2 ml methylene chloride, mixing whole the time. The hard solid was obtained, which was ROMP polymerization product. Additionally, it was observed during experiments, that after addition of precatalyst **1bb** polymerization underwent immediately to form a hard solid product, whereas in the case of precatalyst **1aa** only after 3 minutes reaction mixture started to thicken. After next 3 minutes, gelatinous mass was created, which after 40 minutes of heating has transformed into hard solid polymer.

Procedure B : synthesis of polynorbornene (pNB)

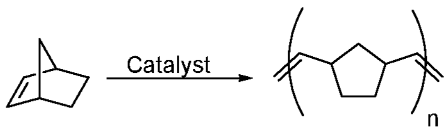


S11 P11

2-Norbornene was placed in round bottom flask of 10 mL volume on the air (100 mg, 1.06 mmol) and 5 ml methylene chloride. The solution was heated to 30°C and 100 μl (0.00106 mmol, 0.1 mol%) of catalyst was added, resulted from dissolving :

a) **1aa** (5,20 mg in 500 μl methylene chloride)
b) **1bb** (3,43 mg in 500 μl methylene chloride)

The reaction mixture was stirred for 20 hours. After that, methylene chloride was evaporated on rotary evaporator, 15 ml of methanol was added, a white solid was precipitated. Methanol was decanted, the solid was washed with additional portion of fresh methanol, was decanted and pre dried on a rotary evaporator, and then dried under reduced pressure on the vacuum pump. The following quantities of the product were obtained. Using **1aa** 21 mg of pNB was obtained, whereas using **1bb** 93 mg of pNB was obtained.

## Claims

**1.** A compound 1 defined by Formula **1a**

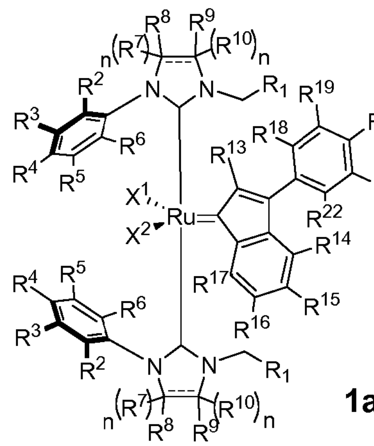

**1a**

in which:

$X^1$ and $X^2$ are an anionic ligand independently selected from group encompassing halogen anion, the groups - CN, -SCN, -OR', -SR', -O(C=O)R', -O($SO_2$)R', and -OSi(R')$_3$, where R' is $C_1$-$C_{12}$ alkyl, $C_3$-$C_{12}$ cycloalkyl, $C_2$-$C_{12}$ alkenyl, or $C_5$-$C_{20}$ aryl, which optionally may be substituted with at least one $C_1$-$C_{12}$ alkyl, $C_1$-$C_{12}$ perfluoroalkyl, $C_1$-$C_{12}$ alkoxyl, $C_5$-$C_{24}$ aryloxyl, $C_5$-$C_{20}$ heteroaryloxyl, or halogen atom;
$R^1$ is heteroaryl group;
$R^2$, $R^3$, $R^4$, $R^5$, and $R^6$ independently of one another are hydrogen atom, $C_1$-$C_{25}$ alkyl group, $C_1$-$C_{25}$ alkoxyl group, or $C_2$-$C_{25}$ alkenyl group, wherein the groups $R^2$, $R^3$, $R^4$, $R^5$ and $R^6$ may be mutually connected into ring that form substituted or unsubstituted $C_4$-$C_{10}$ cyclic or $C_4$-$C_{12}$ polycyclic structure;
$R^7$, $R^8$, $R^9$, and $R^{10}$ independently of one another are hydrogen atom, or $C_1$-$C_{25}$ alkyl group, $R^7$ and/or $R^8$ may be connected with $R^9$ and/or $R^{10}$, to form a cycle;
n is 0 or 1;
$R^{13}$, $R^{14}$, $R^{15}$, $R^{16}$, $R^{17}$, $R^{18}$, $R^{19}$, $R^{20}$, $R^{21}$, and $R^{22}$ independently of one another are hydrogen atom, halogen atom, $C_1$-$C_{25}$ alkyl, $C_1$-$C_{25}$ alkylamino, $C_1$-$C_{25}$ alkylammonium, $C_1$-$C_{25}$ perfluoroalkyl, $C_2$-$C_{25}$ alkenyl, $C_3$-$C_7$ cycloalkyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryl, $C_5$-$C_{20}$ heteroaryl, $C_3$-$C_{12}$ heterocycle, 3-12 membered heterocycle, a thioether (-SR'), an ester (-COOR'), an amide (-CONR'$_2$), a sulphone (-$SO_2$R'), a sulphonamide (-$SO_2$NR'$_2$), or a ketone (-COR'), in which group R' is $C_1$-$C_5$ alkyl, $C_1$-$C_5$ perfluoroalkyl, $C_5$-$C_{25}$ aryl, or $C_5$-$C_{25}$ perfluoroaryl.

2. A compound according to Claim 1 defined by Formula **1a,** in which:

$R^1$ is heteroaryl selected from a group encompassing furan, thiophene, benzothiophene, benzofuran;
$R^2$, $R^3$, $R^4$, $R^5$, and $R^6$ are independently hydrogen atom, methyl, isopropyl, halogen atom;
$R^7$, $R^8$, $R^9$, and $R^{10}$ are independently hydrogen atom or methyl group;
n is 0 or 1;
$X^1$, and $X^2$ are halogen atom;
$R^{13}$, $R^{14}$, $R^{15}$, $R^{16}$, $R^{17}$, $R^{18}$, $R^{19}$, $R^{20}$, $R^{21}$, and $R^{22}$ are independently hydrogen atom, halogen atom, $C_1$-$C_{25}$ alkyl, $C_1$-$C_{25}$ perfluoroalkyl, $C_1$-$C_{25}$ alkoxyl.

3. A compound defined by Formula **1b**

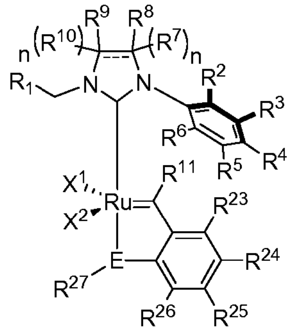

**1b**

in which:

$X^1$ and $X^2$ are an anionic ligand independently selected from group encompassing halogen anion, the groups - CN, -SCN, -OR', -SR', -O(C=O)R', -O($SO_2$)R', and -OSi(R')$_3$, where R' is $C_1$-$C_{12}$ alkyl, $C_3$-$C_{12}$ cycloalkyl, $C_2$-$C_{12}$ alkenyl, or $C_5$-$C_{20}$ aryl, which optionally may be substituted with at least one $C_1$-$C_{12}$ alkyl, $C_1$-$C_{12}$ perfluoroalkyl, $C_1$-$C_{12}$ alkoxyl, $C_5$-$C_{24}$ aryloxyl, $C_5$-$C_{20}$ heteroaryloxyl, or halogen atom;
$R^1$ is heteroaryl group;
$R^2$, $R^3$, $R^4$, $R^5$, and $R^6$ independently of one another are hydrogen atom, $C_1$-$C_{25}$ alkyl group, $C_1$-$C_{25}$ alkoxyl group, or $C_2$-$C_{25}$ alkenyl group, wherein the groups $R^2$, $R^3$, $R^4$, $R^5$ and $R^6$ may be mutually connected into ring that form substituted or unsubstituted $C_4$-$C_{10}$ cyclic or $C_4$-$C_{12}$ polycyclic structure;
$R^7$, $R^8$, $R^9$, and $R^{10}$ independently of one another are hydrogen atom, or $C_1$-$C_{25}$ alkyl group, $R^7$ and/or $R^8$ may be connected with $R^9$ and/or $R^{10}$, to form a cycle;
n is 0 or 1;
$R^{11}$ is hydrogen atom;
$R^{23}$, $R^{24}$, $R^{25}$, and $R^{26}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro (-$NO_2$) group, cyanide (-CN) group, carboxyl (-COOH) group, an ester (-COOR') group, an amide (-CONR'$_2$) group, an imide (-CONR'COR') group, an amine (-NR'$_2$) group, an ammonium (-$N^+R'_3$) group, an amide (-NR'COR') group, a sulphonamide (-NR'$SO_2$R') group, a sulphone (-$SO_2$R') group, a formyl (-CHO) group, a sulphonamide (-$SO_2$NR'$_2$) group, a ketone (-COR') group, in which substituent R' denotes: a $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, whereas $R^{23}$, $R^{24}$, $R^{25}$, and $R^{26}$ are preferentially hydrogen atom;
$R^{27}$ is hydrogen atom, $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, cyanide (-CN) group, carboxyl (-COOH) group, an ester (-COOR') group, an amide (-CONR'$_2$) group, a sulphone (-$SO_2$R') group, a formyl (-CHO) group, a sulphonamide (-$SO_2$NR'$_2$) group, a ketone (-COR') group, in which substituent R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;
E is heteroatom selected from atoms encompassing oxygen, sulphur, nitrogen, phosphorus.

**4.** A compound according to Claim 3 defined by Formula **1b,** in which:

$R^1$ is heteroaryl selected from a group encompassing furan, thiophene, benzothiophene, benzofuran;
$R^2$, $R^3$, $R^4$, $R^5$, and $R^6$ are independently hydrogen atom, methyl, isopropyl, halogen atom;
$R^7$, $R^8$, $R^9$, and $R^{10}$ are independently hydrogen atom or methyl;
n is 0 or 1;
$X^1$, and $X^2$ are halogen atom;
$R^{11}$ is a hydrogen atom;
$R^{23}$, $R^{24}$, $R^{25}$, and $R^{26}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro (-$NO_2$) group, cyanide (-CN) group, carboxyl (-COOH) group, an ester (-COOR') group, an amide (-CONR'$_2$) group, an imide (-CONR'COR') group, an amine (-NR'$_2$) group, an

ammonium ($-N^+R'_3$) group, an amide (-NR'COR') group, a sulphonamide ($-NR'SO_2R'$) group, a sulphone ($-SO_2R'$) group, a formyl (-CHO) group, a sulphonamide ($-SO_2NR'_2$) group, a ketone (-COR') group, in which substituents R' are $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, 3-12 membered heterocycle, wherein $R^{23}$, $R^{24}$, $R^{25}$, and $R^{26}$ are preferably hydrogen atom;

$R^{27}$ is $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, cyanide (-CN) group, carboxyl (-COOH) group, an ester (-COOR') group, an amide ($-CONR'_2$) group, a sulphone ($-SO_2R'$) group, a formyl (-CHO) group, a sulphonamide ($-SO_2NR'_2$) group, a ketone (-COR') group, in which substituent R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

E is heteroatom selected from atoms encompassing oxygen or sulphur atom.

**5.** A compound according to claim **1** or claim **2,** of the structural formula selected from the following formulae **1aa, 1ab, 1ac, 1ba,** and **1bb:**

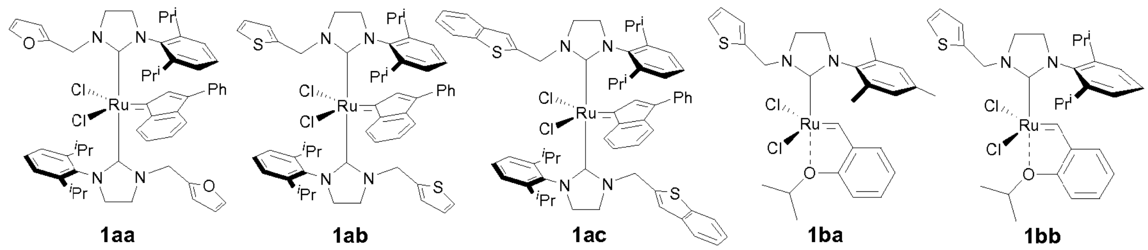

**1aa** **1ab** **1ac** **1ba** **1bb**

**6.** A method of producing a compound defined by Formula **1a** or **1b** according to Claims from 1 to 5, **characterized in that** the alkylidene ruthenium complex defined by Formula **2**

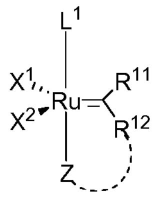

**2**

in which:

$R^{11}$, and $R^{12}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle,

wherein groups $R^{11}$ and $R^{12}$ may be mutually connected into ring selected from among $C_3$-$C_7$ cycloalkyl, $C_3$-$C_{25}$ cycloalkenyl, $C_3$-$C_{25}$ cycloalkynyl, $C_5$-$C_{24}$ aryl, $C_5$-$C_{20}$ heteroaryl, $C_5$-$C_{24}$ perfluoroaryl, which may be independently substituted by at least one and/or more substituents selected from group encompassing hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocyle;

wherein groups $R^{11}$, and $R^{12}$ are preferentially hydrogen atom or aryl independently substituted by an ether group (-OR'), a thioether group (-SR'), a sulphoxide group (-S(O)R'), a sulphonium group ($-S^+R'_2$), a sulphone group ($-SO_2R'$), a sulphonamide group ($-SO_2NR'_2$), an amino group ($-NR'_2$), an ammonium group ($-N^+R'_3$), a nitro group ($-NO_2$), cyanide group (-CN), a phosphonate group ($-P(O)(OR)_2$), a phosphinate group (-P(O)R'(OR')), a phosphonite group ($-P(OR')_2$), a phosphine group ($-PR'_2$), a phosphine oxide group ($-P(O)R'_2$), a phosphonium group ($-P^+R'_3$), carboxyl group (-COOH), an ester group (-COOR'), an amide group ($-CONR'_2$), a formyl group (-CHO), a ketone group (-COR'), where each R' independently is $C_1$-$C_5$ alkyl, $C_1$-$C_5$ perfluoroalkyl, $C_5$-$C_{24}$ aryl, $C_7$-$C_{24}$ aralkyl, $C_5$-$C_{24}$ perfluoroaryl;

$L^1$ is neutral ligand selected from group encompassing pyridine or substituted pyridine, $P(R')_3$, $P(OR')_3$, $O(R')_2$, $N(R')_3$, in which each R' is independently a $C_1$-$C_{12}$ alkyl, $C_3$-$C_{12}$ cycloalkyl, $C_5$-$C_{20}$ aryl, $C_7$-$C_{24}$ aralkyl, $C_5$-$C_{24}$ perfluoroaryl, a 5-12 membered heteroaryl;

Z is selected from such as ligand $L^1$, and then substituents $R^{11}$, and $R^{12}$ are mutually connected to form ring selected from group encompassing a $C_3$-$C_7$ cycloalkyl, $C_3$-$C_{25}$ cycloalkenyl, $C_3$-$C_{25}$ cycloalkynyl, $C_5$-$C_{24}$ aryl, $C_5$-$C_{20}$ heteroaryl, $C_5$-$C_{24}$ perfluoroaryl, 3-12 membered heterocycle, which may be independently substituted by at least one substituent selected from group encompassing hydrogen atom, a halogen, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle, and then dashed line between Z and $R^{12}$ does not mean a chemical bond; heteroatom selected from group encompassing oxygen, nitrogen, sulphur, or phosphorus atom, optionally substituted by group selected from such as hydrogen atom, $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, cyanide (-CN) group, carboxyl (-COOH) group, an ester (-COOR') group, an amide (-CONR'$_2$) group, a sulphone (-$SO_2$R') group, a formyl (-CHO) group, a sulphonamide (-$SO_2$NR'$_2$) group, a ketone (-COR') group, in which group R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, and then dashed line between heteroatom and $R^{12}$ means a chemical bond between a heteroatom and $R^{12}$ substituent, which is aryl optionally substituted by 1, 2, 3, or 4 substituents independently selected from group encompassing hydrogen atom, halogen atom, $C_1$-$C_{25}$ alkyl, $C_1$-$C_{25}$ perfluoroalkyl, $C_2$-$C_{25}$ alken, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$ aralkyl, $C_5$-$C_{24}$ perfluoroaryl, $C_5$-$C_{20}$ heteroaryl, 3-12 membered heterocycle, an ether (-OR') group, a thioether (-SR') group, a nitro (-$NO_2$) group, cyanide (-CN) group, carboxyl (-COOH) group, an ester (-COOR') group, an amide (-CONR'$_2$) group, an imide (-CONR'COR') group, an amine (-NR'$_2$) group, an ammonium (-$N^+$R'$_3$) group, an amide (-NR'COR') group, a sulphonamide (-NR'$SO_2$R') group, a sulphone (-$SO_2$R') group, a formyl (-CHO) group, a sulphonamide (-$SO_2$NR'$_2$) group, a ketone (-COR') group, in which R' has the following meaning: a $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

$X^1$, and $X^2$ are an anionic ligand selected independently from group encompassing halide anions, a -CN, - SCN, -OR', -SR', -O(C=O)R', -O($SO_2$)R', -OSi(R')$_3$ group, where R' is $C_1$-$C_{12}$ alkyl, $C_3$-$C_{12}$ cycloalkyl, $C_2$-$C_{12}$ alkenyl, or $C_5$-$C_{20}$ aryl, which is optionally substituted at least one $C_1$-$C_{12}$ alkyl, $C_1$-$C_{12}$ perfluoroalkyl, $C_1$-$C_{12}$ alkoxyl, $C_5$-$C_{24}$ aryloxyl, $C_5$-$C_{20}$ heteroaryloxyl or halogen atom;

is subjected to a reaction with a carbene defined by Formula **3a**

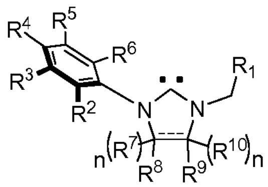

**3a**

in which:

$R^1$ is heteroaryl group;

$R^2$, $R^3$, $R^4$, $R^5$, and $R^6$ are independently hydrogen atom, $C_1$-$C_{25}$ alkyl, $C_1$-$C_{25}$ alkoxyl, or alkenyl $C_2$-$C_{25}$, wherein substituents $R^2$, $R^3$, $R^4$, $R^5$, and $R^6$ may be mutually connected into ring that form substituted or unsubstituted $C_4$-$C_{10}$ cyclic or $C_4$-$C_{12}$ polycyclic structure;

$R^7$, $R^8$, $R^9$, and $R^{10}$ are independently hydrogen atom or a $C_1$-$C_{25}$ alkyl group, $R^7$ and/or $R^8$ may be mutually connected with $R^9$ and/or $R^{10}$, to form a cycle;

n is 0 or 1.

7. A method of producing a compound defined by Formula **1a or 1b** according to Claim 6, **characterized in that** the alkylidene ruthenium complex defined by Formula **2** is subjected to a reaction with a carbene compound defined by Formula **3b**

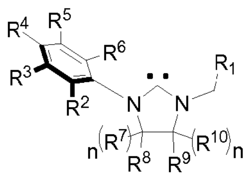

**3b**

in which:

$R^1$ is heteroaryl selected from a group encompassing furan, thiophene, benzothiophene, benzofuran;
$R^2$, $R^3$, $R^4$, $R^5$, and $R^6$ are independently hydrogen atom, methyl, isopropyl, halogen atom;
$R^7$, $R^8$, $R^9$, and $R^{10}$ are independently hydrogen atom, methyl;
n is 0 or 1.

**8.** A method of producing a compound defined by Formula **1a or 1b** according to any of Claims from 1 to 5 **characterized in that** the alkylidene ruthenium complex defined by Formula **2**

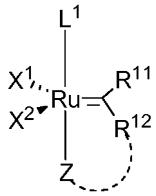

**2**

in which:

$R^{11}$, and $R^{12}$ are independently hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle, wherein $R^{11}$ and $R^{12}$ is preferably hydrogen atom or an aryl substituted by a nitro ($-NO_2$) group, cyanide (-CN) group, carboxyl (-COOH) group, an ester (-COOR') group, an amide ($-CONR'_2$) group, a sulphone ($-SO_2R'$) group, a formyl (-CHO) group, a sulphonamide ($-SO_2NR'_2$) group, a ketone (-COR') group, in which group R' is $C_1$-$C_5$ alkyl, $C_1$-$C_5$ perfluoroalkyl, $C_5$-$C_{24}$ aryl, $C_7$-$C_{24}$ aralkyl, $C_5$-$C_{24}$ perfluoroaryl;
$L^1$ is a neutral ligand selected from groups encompassing pyridine or substituted pyridine, $P(R')_3$, $P(OR')_3$, $O(R')_2$, $N(R')_3$, where each R' is independently a $C_1$-$C_{12}$ alkyl, $C_3$-$C_{12}$ cycloalkyl, $C_5$-$C_{20}$ aryl, $C_7$-$C_{24}$ aralkyl, $C_5$-$C_{24}$ perfluoroaryl, a 5-12 membered heteroaryl;
Z is selected from among such as, ligand $L^1$, and then substituents $R^{11}$ and $R^{12}$ are mutually connected to form cyclic structure selected from groups encompassing a $C_3$-$C_7$ cycloalkyl, $C_3$-$C_{25}$ cycloalkenyl, $C_3$-$C_{25}$ cycloalkynyl, $C_5$-$C_{24}$ aryl, $C_5$-$C_{20}$ heteroaryl, $C_5$-$C_{24}$ perfluoroaryl, 3-12 membered heterocycle, which may be independently substituted with at least one or more substituents selected from groups encompassing hydrogen atom, halogen atom, $C_1$-$C_{25}$ alkyl, $C_1$-$C_{25}$ perfluoroalkyl, $C_2$-$C_{25}$ alkene, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_1$-$C_{25}$ alkoxyl, $C_5$-$C_{24}$ aryloxyl, $C_5$-$C_{20}$ heteroaryloxyl, $C_5$-$C_{24}$ aryl, $C_5$-$C_{20}$ heteroaryl, $C_7$-$C_{24}$ aralkyl, $C_5$-$C_{24}$ perfluoroaryl, 3-12 membered heterocycle, and then dashed line between Z and $R^{12}$ does not mean a chemical bond; heteroatom selected from group encompassing oxygen, nitrogen, sulphur, or phosphorus atom, optionally substituted by group selected from such as hydrogen atom, $C_1$-$C_{25}$ alkyl, $C_1$-$C_{25}$ perfluoroalkyl, $C_3$-$C_7$ cycloalkyl, $C_5$-$C_{24}$ aryl, $C_5$-$C_{24}$ perfluoroaryl, $C_5$-$C_{20}$ heteroaryl, $C_7$-$C_{24}$ aralkyl, 3-12 membered heterocycle, an acyl (-COR') group, cyanide (-CN) group, carboxyl (-COOH) group, an ester (-COOR') group, an amide ($-CONR'_2$), a sulphone ($-SO_2R'$) group, a formyl (-CHO) group, a sulphonamide ($-SO_2NR'_2$) group, a ketone (-COR') group, in which group R' is $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl, and then dashed line between heteroatom and $R^{12}$ group means a chemical bond, which is aryl optionally substituted by 1, 2, 3, or 4 substituents independently selected from group encompassing hydrogen atom, halogen atom, $C_1$-$C_{25}$ alkyl, $C_1$-$C_{25}$ perfluoroalkyl, a alkene $C_2$-$C_{25}$, $C_3$-$C_7$ cycloalkyl, $C_2$-$C_{25}$ alkenyl, $C_3$-$C_{25}$ cycloalkenyl, $C_2$-$C_{25}$ alkynyl, $C_3$-$C_{25}$ cycloalkynyl, $C_5$-$C_{24}$ aryl, $C_7$-$C_{24}$

aralkyl, $C_5$-$C_{24}$ perfluoroaryl, $C_5$-$C_{20}$ heteroaryl, 3-12 membered heterocycle, an ether (-OR') group, a thioether (-SR') group, a nitro (-$NO_2$) group, cyanide (-CN) group, carboxyl (-COOH) group, an ester (-COOR') group, an amide (-CONR'$_2$) group, an imide (-CONR'COR') group, an amine (-NR'$_2$) group, an ammonium (-$N^+R'_3$) group, an amide (-NR'COR') group, a sulphonamide (-NR'$SO_2$R') group, a sulphone (-$SO_2$R') group, a formyl (-CHO) group, a sulphonamide (-$SO_2$NR'$_2$) group, a ketone (-COR') group, in which group R' has the following meaning: a $C_1$-$C_5$ alkyl, $C_1$-$C_5$ perfluoroalkyl, $C_5$-$C_{24}$ aryl, $C_5$-$C_{24}$ perfluoroaryl, $C_7$-$C_{24}$ aralkyl;

$X^1$, and $X^2$ are an anionic ligand independently selected from groups encompassing halide anions, a -CN, -SCN, -OR', -SR', -O(C=O)R', -O($SO_2$)R', -OSi(R')$_3$, where R' is $C_1$-$C_{12}$ alkyl, $C_3$-$C_{12}$ cycloalkyl, $C_2$-$C_{12}$ alkenyl, $C_5$-$C_{20}$ aryl, which may possibly be substituted with at least one a $C_1$-$C_{12}$ alkyl, $C_1$-$C_{12}$ perfluoroalkyl, $C_1$-$C_{12}$ alkoxyl, $C_5$-$C_{24}$ aryloxyl, $C_5$-$C_{20}$ heteroaryloxyl or halogen atom;

is subjected to a reaction with a carbene generated *in situ* as a result of the reaction of base selected from such as potassium *tert*-pentoxide, potassium *tert*-butoxide, potassium *N,N'*-bis(trimethylsilyl)amide, or sodium hydride, with carbene precursor defined by Formula **4a**

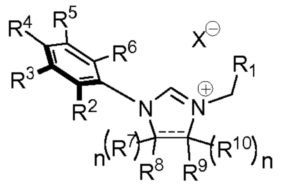

**4a**

in which:

$R^1$ is heteroaryl group;

$R^2$, $R^3$, $R^4$, $R^5$, and $R^6$ are independently hydrogen atom, $C_1$-$C_{25}$ alkyl, $C_1$-$C_{25}$ alkoxyl, or $C_2$-$C_{25}$ alkenyl, wherein substituents $R^2$, $R^3$, $R^4$, $R^5$, and $R^6$ may be mutually connected, to form substituted or unsubstituted $C_4$-$C_{10}$ cyclic or $C_4$-$C_{12}$ polycyclic structure;

$R^7$, $R^8$, $R^9$, and $R^{10}$ are independently hydrogen atom or a $C_1$-$C_{25}$ alkyl, $R^7$ and/or $R^8$ may be mutually connected with $R^9$ and/or $R^{10}$, to form cycle;

n is 0 or 1;

$X^-$ is a halide anion, or $BF_4^-$, $PF_6^-$, or $ClO_4^-$.

**9.** A method of producing a compound defined by Formula **1a or 1b** according to Claim 8, **characterised in that** the alkylidene ruthenium complex defined by Formula **2** is subjected to a reaction with a carbene generated *in situ* as a result of the reaction of base selected from such as potassium *tert*-pentoxide, potassium *tert*-butoxide, potassium *N,N'*-bis(trimethylsilyl)amide, or sodium hydride, with carbene precursor defined by Formula **4b**

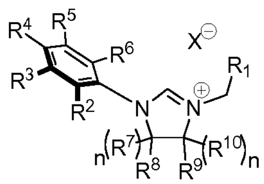

**4b**

in which:

$R^1$ is heteroaryl selected from a group encompassing furan, thiophene, benzothiophene, benzofuran;

$R^2$, $R^3$, $R^4$, $R^5$, and $R^6$ are independently hydrogen atom or methyl, isopropyl, halogen atom;

$R^7$, $R^8$, $R^9$, and $R^{10}$ are independently hydrogen atom or methyl;

n is 0 or 1;

$X^-$ is halogen anion, or $BF_4^-$, $PF_6^-$, $ClO_4^-$.

**10.** A method of producing a compound defined by Formula **1a or 1b** according to any of Claims from 6 to 9, **characterised**

**in that** the alkylidene ruthenium complex defined by Formula **2a**

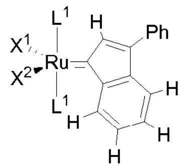

**2a**

in which:
$X^1$, $X^2$, and $L^1$ have the same meaning as defined by Formula **2.**

**11.** A method of producing a compound according to any of Claims from 6 to 9, **characterised in that** as the alkylidene ruthenium complex is used compound defined by Formula **2b**

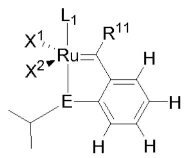

**2b**

in which:

$X^1$, $X^2$, and $L^1$, have the same meaning as defined above;
$R^{11}$ is hydrogen atom;
E is a heteroatom selected from such as oxygen, sulphur, nitrogen, phosphorus.

**12.** A use of a compound defined by Formula **1a or 1b** as defined in Claims from 1 to 5, as a precatalyst and/or catalyst in the olefin metathesis reactions, such as ring closing metathesis (RCM), homometathesis, cross metathesis (CM), ethenolysis, isomerisation, in diastero ring-rearrangement metathesis (DRRM), in enyne metathesis or ROMP type polymerization reaction, particularly in ring opening metathesis polymerization (ROMP) of dicyclopentadiene or norbornene.

**13.** The use according to Claim 12, in which compound defined by Formula **1a or 1b** is used as a precatalyst and/or catalyst in the reaction mixture from 1 minute to 24 hours, in non-polar solvents or without solvents (neat mixtures).

**14.** A use of compound defined by Formula **1a or 1b** according to any of Claims from 1 to 5, as a substrate for the synthesis of other ruthenium complex compound which are precatalyst and/or catalyst in the olefin metathesis reactions.

## Patentansprüche

**1.** Verbindung nach Anspruch 1 mit der Formel **1a**

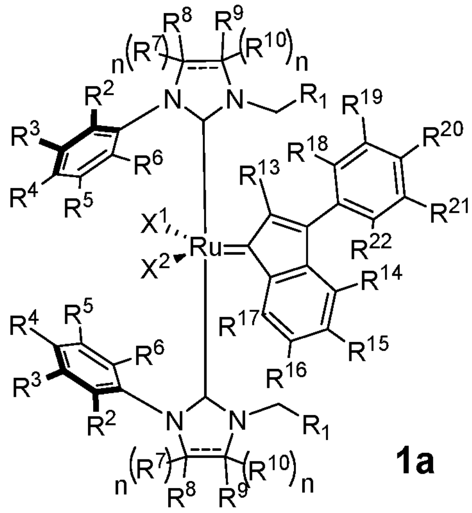

**1a**

wobei:

wobei:

$X^1$ und $X^2$ unabhängig voneinander den anionischen Liganden bedeuten, ausgewählt aus solchen Halogenatomen, -CN, -SCN, -OR', -SR', -O(C=O)R', -O($SO_2$)R', -OSi(R')$_3$-Gruppe, wobei R' $C_1$-$C_{12}$-Alkyl, $C_3$-$C_{12}$-Cycloalkyl, $C_2$-$C_{12}$-Alkenyl, $C_5$-$C_{20}$-Aryl, das mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryoxyl, $C_5$-$C_{20}$-Heteroaryl oder einem Halogenatom substituiert sein kann;

$R^1$ bedeutet eine Heteroarylgruppe;

$R^2$, $R^3$, $R^4$, $R^5$, $R^6$ unabhängig voneinander ein Wasserstoffatom, eine $C_1$-$C_{25}$-Alkylgruppe, eine $C_1$-$C_{25}$-Alkoxygruppe oder eine $C_2$-$C_{25}$-Alkylgruppe bedeuten, wobei die Substituenten $R^2$, $R^3$, $R^4$, $R^5$ und $R^6$ kombiniert sein können, um ein substituiertes oder unsubstituiertes $C_4$-$C_{10}$- oder polyzyklisches $C_4$-$C_{12}$-System zu bilden;

$R^7$, $R^8$, $R^9$ und $R^{10}$ unabhängig voneinander ein Wasserstoffatom oder eine $C_1$-$C_{25}$-Alkylgruppe bedeuten, wobei $R^7$ und/oder $R^8$ mit $R^9$ und/oder $R^{10}$ kombiniert sein können, um ein zyklisches System zu bilden;

n beträgt 0 oder 1;

$R^{13}$, $R^{14}$, $R^{15}$, $R^{16}$, $R^{17}$, $R^{18}$, $R^{19}$, $R^{20}$, $R^{21}$ und $R^{22}$ unabhängig voneinander das Wasserstoffatom, das Halogenatom, eine $C_1$-$C_{25}$-Alkylgruppe, eine $C_1$-$C_{25}$-Alkylamingruppe, eine $C_1$-$C_{25}$-Alkylammoniumgruppe, eine $C_1$-$C_{25}$-Perfluoralkylgruppe, eine $C_2$-$C_{25}$-Alkenylgruppe, eine $C_3$-$C_7$-Cycloalkylgruppe, eine $C_3$-$C_{25}$-Cycloalkenylgruppe, eine $C_2$-$C_{25}$-Alkylgruppe, eine $C_3$-$C_{25}$-Cycloalkinylgruppe, eine $C_1$-$C_{25}$-Alkoxygruppe, eine $C_5$-$C_{24}$-Arylgruppe, eine $C_5$-$C_{20}$-Heteroarylgruppe, eine $C_3$-$C_{12}$-Heterocyclusgruppe, 5-12-gliedriger Heterocyclus, eine Sulfid- (-SR'), eine Ester- (-COOR'), eine Amid- (-CONR'$_2$), eine Sulfon- (-$SO_2$R'), eine Sulfonamid- (-$SO_2$NR'$_2$), oder eine Ketogruppe (-COR'), wobei R' eine $C_1$-$C_5$-Alkylgruppe, eine $C_1$-$C_5$-Perfluoralkylgruppe, eine $C_5$-$C_{25}$-Arylgruppe oder eine $C_5$-$C_{25}$-Perfluorarylgruppe ist.

**2.** Verbindung nach Anspruch 2 mit der Formel **1a**
wobei:

$R^1$ Heteroaryl bedeutet, ausgewählt aus der Gruppe, die Furan, Thiophen, Benzothiophen, Benzofuran umfasst;

$R^2$, $R^3$, $R^4$, $R^5$, $R^6$ unabhängig das Wasserstoffatom, Methyl, Isopropyl, Halogenatom bedeuten;

$R^7$, $R^8$, $R^9$, $R^{10}$ unabhängig voneinander ein Wasserstoffatom oder Methyl bedeuten;

n beträgt 0 oder 1;

$X^1$, $X^2$ ein Halogenatom sind;

$R^{13}$, $R^{14}$, $R^{15}$, $R^{16}$, $R^{17}$, $R^{18}$, $R^{19}$, $R^{20}$, $R^{21}$ und $R^{22}$ unabhängig voneinander Wasserstoffatom, Halogenatom, $C_1$-$C_{25}$-Alkylgruppe, $C_1$-$C_{25}$-Perfluoralkylgruppe, $C_1$-$C_{25}$-Alkoxygruppe sind.

**3.** Verbindung nach Anspruch 1 mit der Formel **1b**

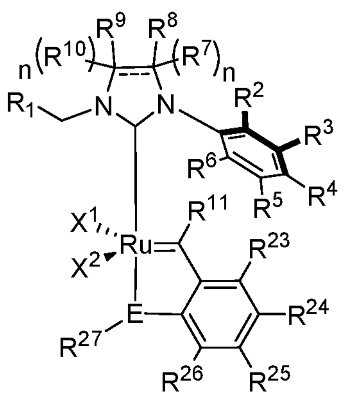

**1b**

wobei:

$X^1$ und $X^2$ unabhängig voneinander den anionischen Liganden bedeuten, ausgewählt aus solchen Halogenatomen, -CN, -SCN, -OR', -SR', -O(C=O)R', -O($SO_2$)R', -OSi(R')$_3$-Gruppe, wobei R' $C_1$-$C_{12}$-Alkyl, $C_3$-$C_{12}$-Cycloalkyl, $C_2$-$C_{12}$-Alkenyl, $C_5$-$C_{20}$-Aryl, das mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryoxyl, $C_5$-$C_{20}$-Heteroaryl oder einem Halogenatom substituiert sein kann;
$R^1$ bedeutet eine Heteroarylgruppe;
$R^2$, $R^3$, $R^4$, $R^5$, $R^6$ unabhängig voneinander ein Wasserstoffatom, eine $C_1$-$C_{25}$-Alkylgruppe, eine $C_1$-$C_{25}$-Alkoxygruppe oder eine $C_2$-$C_{25}$-Alkylgruppe bedeuten, wobei die Substituenten $R^2$, $R^3$, $R^4$, $R^5$ und $R^6$ kombiniert sein können, um ein substituiertes oder unsubstituiertes $C_4$-$C_{10}$- oder polyzyklisches $C_4$-$C_{12}$-System zu bilden;
$R^7$, $R^8$, $R^9$ und $R^{10}$ unabhängig voneinander ein Wasserstoffatom oder eine $C_1$-$C_{25}$-Alkylgruppe bedeuten, wobei $R^7$ und/oder $R^8$ mit $R^9$ und/oder $R^{10}$ kombiniert sein können, um ein zyklisches System zu bilden;
n beträgt 0 oder 1;
$R^{11}$ ein Wasserstoffatom bedeutet;
$R^{23}$, $R^{24}$, $R^{25}$, $R^{26}$ unabhängig ein Wasserstoffatom, Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl bedeutet, $C_2$-$C_{25}$-Alkinyl, $C_3$-$C_{25}$-Cycloalkinyl, $C_5$-$C_{24}$-Aryl, $C_7$-$C_{24}$-Aralkyl, $C_5$-$C_{24}$-Perfluoraryl, $C_5$-$C_{20}$-Heteroaryl, 3-12-gliedriger Heterocyclus, eine Alkoxyl- (-OR'), eine Sulfid- (-SR'), eine Nitro- (-$NO_2$), eine Cyan- (-CN), eine Carboxyl- (-COOH), eine Ester- (-COOR'), eine Amid- (-CONR'$_2$), eine Amin- (-NR'$_2$), eine Ammonium- (-N+R'$_3$), eine Amid- (-NR'COR'), eine Sulfon- (-$SO_2$R'), eine Formyl- (-CHO), eine Sulfonamid- (-$SO_2$NR'$_2$), eine Ketogruppe (-COR') bedeuten, wobei R' die folgende Bedeutung hat: $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl, wobei $R^{23}$, $R^{24}$, $R^{25}$, $R^{26}$ vorzugsweise ein Wasserstoffatom bedeuten;
$R^{27}$ ein Wasserstoffatom, $C_1$-$C_{25}$-Alkyl, $C_1$-$C_{25}$-Perfluoralkyl, $C_3$-$C_7$-Cycloalkyl, $C_5$-$C_{24}$-Aryl, $C_5$-$C_{24}$-Perfluoraryl, $C_5$-$C_{20}$-Heteroaryl, $C_7$-$C_{24}$-Aralkyl, 3-12-gliedriger Heterocyclus, eine Acyl- - COR', Cyan- (-CN), Carboxy- (-COOH), eine Ester- (-COOR'), eine Amid- (-CONR'$_2$), Sulfongruppe (-$SO_2$R'), eine Formyl- (-CHO), eine Sulfonamid- (-$SO_2$NR'$_2$), eine Ketogruppe (-COR') bedeutet, wobei die Gruppe R' $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl bedeutet;
E bedeutet ein Heteroatom, ausgewählt aus den Atomen wie Sauerstoff, Schwefel, Stickstoff, Phosphor.

**4.** Verbindung nach Anspruch 3 mit der Formel **1b**
wobei:

$R^1$ Heteroaryl bedeutet, ausgewählt aus der Gruppe, die Furan, Thiophen, Benzothiophen, Benzofuran umfasst;
$R^2$, $R^3$, $R^4$, $R^5$, $R^6$ unabhängig das Wasserstoffatom, Methyl, Isopropyl, Halogenatom bedeuten;
$R^7$, $R^8$, $R^9$, $R^{10}$ unabhängig voneinander ein Wasserstoffatom oder Methyl bedeuten;
n beträgt 0 oder 1;
$X^1$, $X^2$ ein Halogenatom sind;
$R^{11}$ ein Wasserstoffatom bedeutet;
$R^{23}$, $R^{24}$, $R^{25}$, $R^{26}$ unabhängig ein Wasserstoffatom, Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl bedeutet, $C_2$-$C_{25}$-Alkinyl, $C_3$-$C_{25}$-Cycloalkinyl, $C_5$-$C_{24}$-Aryl, $C_7$-$C_{24}$-Aralkyl, $C_5$-$C_{24}$-Perfluoraryl, $C_5$-$C_{20}$-Heteroaryl, 3-12-gliedriger Heterocyclus, eine

Alkoxyl- (-OR'), eine Sulfid- (-SR'), eine Nitro- (-$NO_2$), eine Cyan- (-CN), eine Carboxyl- (-COOH), eine Ester- (-COOR'), eine Amid- (-$CONR'_2$), eine Amin- (-$NR'_2$), eine Ammonium- (N+$R'_3$), eine Amid- (-NR'COR'), eine Sulfon- (-$SO_2R'$), eine Formyl- (CHO), eine Sulfonamid- (-$SO_2NR'_2$), eine Ketogruppe (-COR'), 3-12-gliedriger Heterocyclus, bedeuten, wobei R' die folgende Bedeutung hat: $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl, wobei $R^{23}$, $R^{24}$, $R^{25}$, $R^{26}$ vorzugsweise ein Wasserstoffatom bedeuten;
$R^{27}$ $C_1$-$C_{25}$-Alkyl, $C_1$-$C_{25}$-Perfluoralkyl, $C_3$-$C_7$-Cycloalkyl, $C_5$-$C_{24}$-Aryl, $C_5$-$C_{24}$-Perfluoraryl, $C_5$-$C_{20}$-Heteroaryl, $C_7$-$C_{24}$-Aralkyl, 3-12-gliedriger Heterocyclus, eine Acyl- -COR', Cyan- (-CN), Carboxy- (-COOH), eine Ester- (-COOR'), eine Amid- (-$CONR'_2$), Sulfongruppe (-$SO_2R'$), eine Formyl- (-CHO), eine Sulfonamid- (-$SO_2NR'_2$), eine Ketogruppe (-COR') bedeutet, wobei die Gruppe R' $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl bedeutet;
E bedeutet ein Heteroatom, das aus den Atomen wie dem Sauerstoff- oder Schwefelatom ausgewählt wird.

5. Verbindung nach Anspruch 1 oder nach Anspruch 2 wobei die Struktur durch das aus **1aa, 1ab, 1ac, 1ba und 1bb** ausgewählte Modell dargestellt wird:

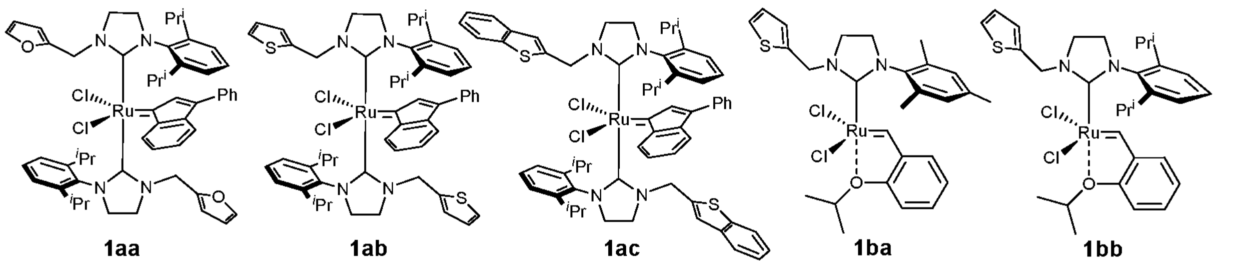

**1aa** **1ab** **1ac** **1ba** **1bb**

6. Herstellungsverfahren für eine Verbindung mit der Formel **1a** oder **1b** nach einem der Ansprüche 1-5, **dadurch gekennzeichnet, dass** der Alkyliden-Rutheniumkomplex mit der Formel 2

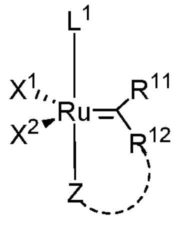

**2**

wobei:

$R^{11}$, $R^{12}$ unabhängig voneinander Wasserstoffatom, Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl, $C_2$-$C_{25}$-Alkinyl, $C_3$-$C_{25}$-Cycloalkinyl, $C_1$-$C_{25}$-Alkoxyl, $C_5$-$C_{24}$-Aryloxyl, $C_5$-$C_{20}$-Heteroaryloxyl, $C_5$-$C_{24}$-Aryl, $C_5$-$C_{20}$-Heteroaryl, $C_7$-$C_{24}$-Aralkyl, $C_5$-$C_{24}$-Perfluoraryl, 3-12-gliedriger Heterocyclus bedeuten,
wobei $R^{11}$- und $R^{12}$-Substituenten kombiniert sein können, um einen Ring zu bilden, der aus der Gruppe ausgewählt ist, die $C_3$-$C_7$-Cycloalkyl, $C_3$-$C_{25}$-Cycloalkenyl, $C_3$-$C_{25}$-Cycloalkinyl, $C_5$-$C_{24}$-Aryl, $C_5$-$C_{20}$-Heteroaryl, $C_5$-$C_{24}$-Perfluoraryl umfasst, die unabhängig voneinander mit einem und/oder mehreren Substituenten substituiert sein können, die aus der Gruppe ausgewählt sind, die das Wasserstoffatom, das Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkyl, $C_3$-$C_{25}$-Cycloalkenyl, $C_2$-$C_{25}$-Alkinyl, $C_3$-$C_{25}$-Cycloalkinyl, $C_1$-$C_{25}$-Alkoxyl, $C_5$-$C_{24}$-Aryloxyl, $C_5$-$C_{20}$-Heteroaryl, $C_5$-$C_{24}$-Aryl, $C_5$-$C_{20}$-Heteroaryl, $C_7$-$C_{24}$-Aralkyl, $C_5$-$C_{24}$-Perfluoraryl, 3-12-gliedriger Heterocyclus umfasst;
wobei $R^{11}$, $R^{12}$ vorzugsweise entweder ein Wasserstoffatom oder ein durch eine Alkoxyl- (-OR'), eine Sulfid- (-SR'), eine Sulfoxid- (-S(O)R'), eine Sulfonium- (-S+$R'_2$), eine Sulfonium- (-$SO_2R'$), eine Sulfonamid-(-$SO_2NR'_2$), eine Amin- (-$NR'_2$), eine Ammonium- (-N+$R'_3$), eine Nitro- (-$NO_2$), eine Cyanid- (-CN), eine Phosphonyl- (-P(O)$(OR)_2$), eine Phosphonyl- (-P(O)R'(OR')), eine Phosphonin- (-P$(OR')_2$), eine Phosphin- (-$PR'_2$), eine Phosphinoxid- (-P(O)$R'_2$), eine Phosphit-(-P+$R'_3$), eine Carboxyl- (-COOH), eine Ester- (-COOR'), eine Amid- (-$CONR'_2$), eine Formyl- (-CHO), eine Ketogruppe (-COR') unabhängig substituiertes Aryl, wobei R' $C_1$-$C_5$-Alkyl, $C_1$-$C_5$-Perfluoralkyl, $C_5$-$C_{24}$-Aryl, $C_7$-$C_{24}$-Aralkyl, $C_5$-$C_{24}$-Perfluoraryl bedeutet;

$L^1$ einen inerten Liganden bedeutet, der aus einer Gruppe ausgewählt ist, die Pyridin oder substituiertes Pyridin, $P(R')_3$, $P(OR')_3$, $O(R')_2$, $N(R')_3$ einschließt, wobei jedes R' unabhängig voneinander $C_1$-$C_{12}$-Alkyl, $C_3$-$C_{12}$-Zykloalkyl, $C_5$-$C_{20}$-Aryl, $C_7$-$C_{24}$-Aralkyl, $C_5$-$C_{24}$-Perfluoraryl, 5-12-gliedriges Heteroaryl bedeutet;

Z aus einem solchen Liganden wie $L^1$ ausgewählt ist, und dann die Substituenten $R^{11}$ und $R^{12}$ kombiniert werden, um einen Ring zu bilden, der aus der Gruppe ausgewählt ist, die $C_3$-$C_7$-Cycloalkyl, $C_3$-$C_{25}$-Cycloalkenyl, $C_3$-$C_{25}$-Cycloalkinyl, $C_5$-$C_{24}$-Aryl, $C_5$-$C_{20}$-Heteroaryl, $C_5$-$C_{24}$-Perfluoraryl, 3-12-gliedriger Heterocyclus, umfasst, der unabhängig voneinander durch einen und/oder mehrere Substituenten substituiert sein kann, die aus der Gruppe ausgewählt sind, die ein Wasserstoffatom, ein Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl, $C_2$-$C_{25}$-Alkinyl, $C_3$-$C_{25}$-Cycloalkinyl, $C_1$-$C_{25}$-Alkoxyl, $C_5$-$C_{24}$-Aryloxyl, $C_5$-$C_{20}$-Heteroaryloxyl, $C_5$-$C_{24}$-Aryl, $C_5$-$C_{20}$-Heteroaryl, $C_7$-$C_{24}$-Aralkyl, $C_5$-$C_{24}$-Perfluoraryl, 3-12-gliedriger Heterocyclus umfasst und die gestrichelte Linie zwischen Z und $R^{12}$ keine chemische Bindung zeigt; Heteroatom, ausgewählt aus einer Gruppe, die Sauerstoff-, Stickstoff-, Schwefel- und Phosphoratome umfasst, möglicherweise substituiert durch eine Gruppe, ausgewählt aus einem Wasserstoffatom, $C_1$-$C_{25}$-Alkyl, $C_1$-$C_{25}$-Perfluoralkyl, $C_3$-$C_7$-Cycloalkyl, $C_5$-$C_{24}$-Aryl, $C_5$-$C_{24}$-Perfluoraryl, $C_5$-$C_{20}$-Heteroaryl, $C_7$-$C_{24}$-Aralkyl, 3-12-gliedriger Heterocyclus, eine Acyl- -COR', eine Cyan- (-CN), eine Carboxyl- (-COOH), eine Ester- (-COOR'), eine Amid- (-CONR'$_2$), eine Sulfon- (-SO$_2$R'), eine Formyl- (-CHO), eine Sulfonamid- (-SO$_2$NR'$_2$), eine Ketongruppe (-COR'), wobei die Gruppe R' $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl bedeutet, und dann bedeutet die gestrichelte Linie eine direkte Bindung zwischen dem Heteroatom und dem Substituenten $R^{12}$, der Aryl ist, das möglicherweise durch 1-4 Substituenten substituiert ist, die unabhängig voneinander aus einer Gruppe ausgewählt sind, die ein Wasserstoffatom, ein Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl, $C_2$-$C_{25}$-Alkinyl, $C_3$-$C_{25}$-Cycloalkinyl, $C_5$-$C_{24}$-Aryl, $C_7$-$C_{24}$-Aralkyl, $C_5$-$C_{24}$-Perfluoraryl, $C_5$-$C_{20}$-Heteroaryl, 3-12-gliedriger Heterocyclus, eine Alkoxy- (-OR'), eine Sulfid- (-SR'), eine Nitro- (-NO$_2$), eine Cyanid- (-CN), eine Carboxyl- (-COOH), eine Ester- (-COOR'), eine Amid- (-CONR'$_2$), eine Imid- (-CONR'COR'), eine Amin- (-NR'$_2$), eine Ammonium- (-N+R'$_3$), eine Amid- (-NR'COR'), eine Sulfonamid- (-NR'SO$_2$R'), eine Sulfon- (-SO$_2$R'), eine Formyl-(-CHO), eine Sulfonamid- (-SO$_2$NR'$_2$), eine Ketogruppe (-COR') umfasst, wobei die Gruppen R' die folgende Bedeutung haben: $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl;

$X^1$, $X^2$ bedeutet einen anionischen Liganden, der unabhängig aus einer Gruppe ausgewählt ist, die Halogenidanionen, -CN, -SCN, -OR', -SR', -O(C=O)R', -O(SO$_2$)R', -OSi(R')$_3$-Gruppe umfasst, wobei R' $C_1$-$C_{12}$-Alkyl, $C_3$-$C_{12}$-Cycloalkyl, $C_2$-$C_{12}$-Alkenyl, $C_5$-$C_{20}$-Aryl, das mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryoxyl, $C_5$-$C_{20}$-Heteroaryl oder einem Halogenatom substituiert sein kann;

einer Reaktion mit dem Carben mit der Formel **3a** unterzogen wird

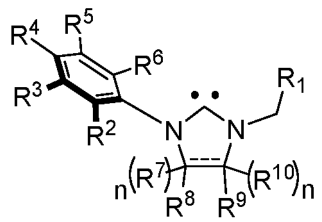

**3a**

wobei:

$R^1$ bedeutet eine Heteroarylgruppe;

$R^2$, $R^3$, $R^4$, $R^5$, $R^6$ unabhängig voneinander ein Wasserstoffatom, eine $C_1$-$C_{25}$-Alkylgruppe, eine $C_1$-$C_{25}$-Alkoxygruppe oder eine $C_2$-$C_{25}$-Alkylgruppe bedeuten, wobei die Substituenten $R^2$, $R^3$, $R^4$, $R^5$, $R^6$ zur Bildung eines substituierten oder unsubstituierten zyklischen Systems $C_4$-$C_{10}$ oder eines polyzyklischen Systems $C_4$-$C_{12}$ kombiniert werden können;

$R^7$, $R^8$, $R^9$ und $R^{10}$ unabhängig voneinander ein Wasserstoffatom oder eine $C_1$-$C_{25}$-Alkylgruppe bedeuten, wobei $R^7$ und/oder $R^8$ mit $R^9$ und/oder $R^{10}$ kombiniert sein können, um ein zyklisches System zu bilden;

n beträgt 0 oder 1;

7. Herstellungsverfahren für eine Verbindung mit der Formel **1a** oder **1b** nach dem Anspruch **6, dadurch gekennzeichnet, dass** der Alkyliden-Rutheniumkomplex mit der Formel **2** einer Reaktion mit dem Carben mit der Formel

**3b** unterzogen wird,

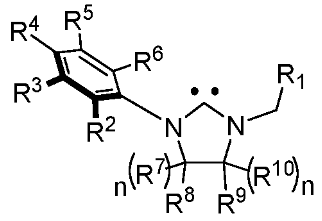

**3b**

wobei:

$R^1$ Heteroaryl bedeutet, ausgewählt aus der Gruppe, die Furan, Thiophen, Benzothiophen, Benzofuran umfasst;
$R^2$, $R^3$, $R^4$, $R^5$, $R^6$ unabhängig das Wasserstoffatom, Methyl, Isopropyl, Halogenatom bedeuten;
$R^7$, $R^8$, $R^9$, $R^{10}$ unabhängig voneinander ein Wasserstoffatom oder Methyl bedeuten;
n beträgt 0 oder 1;

**8.** Herstellungsverfahren für eine Verbindung mit der Formel **1a** oder **1b** nach einem der Ansprüche 1-5, **dadurch gekennzeichnet, dass** der Alkyliden-Rutheniumkomplex mit der Formel 2

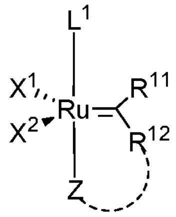

**2**

wobei:

$R^{11}$, $R^{12}$ unabhängig voneinander Wasserstoffatom, Halogenatom, $C_1$-$C_{25}$-Alkyl, $C_1$-$C_{25}$-Perfluoralkyl, $C_2$-$C_{25}$-Alken, Cycloalkyl $C_3$-$C_7$, Alkenyl $C_2$-$C_{25}$, Cycloalkenyl $C_3$-$C_{25}$, Alkinyl $C_2$-$C_{25}$, $C_3$-$C_{25}$-Cycloalkinyl, $C_1$-$C_{25}$-Alkoxyl, $C_5$-$C_{24}$-Aryloxyl, $C_5$-$C_{20}$-Heteroaryloxyl, $C_5$-$C_{24}$-Aryl, $C_5$-$C_{20}$-Heteroaryl, $C_7$-$C_{24}$-Aralkyl, $C_5$-$C_{24}$-Perfluoraryl, 3-12-gliedriger Heterocyclus bedeuten, wobei $R^{11}$ und $R^{12}$ vorzugsweise ein Wasserstoffatom oder ein durch eine Aryl- (-$NO_2$), Cyanid-(-CN), Carboxyl- (-COOH), Ester- (-COOR'), Amid- (-CONR'$_2$), Sulfon- (-$SO_2$R'), eine Formyl- (-CHO), eine Sulfonamid- (-$SO_2$NR'$_2$), eine Ketogruppe (-COR') substituiertes Aryl bedeuten, wobei R' $C_1$-$C_5$-Alkyl, $C_1$-$C_5$-Perfluoralkyl, $C_5$-$C_{24}$-Aryl, $C_7$-$C_{24}$-Aralkyl, $C_5$-$C_{24}$-Perfluoraryl bedeutet;
$L^1$ einen inerten Liganden bedeutet, der aus einer Gruppe ausgewählt ist, die Pyridin oder substituiertes Pyridin, P(R')$_3$, P(OR')$_3$, O(R')$_2$, N(R')$_3$ einschließt, wobei jedes R' unabhängig voneinander $C_1$-$C_{12}$-Alkyl, $C_3$-$C_{12}$-Zykloalkyl, $C_5$-$C_{20}$-Aryl, $C_7$-$C_{24}$-Aralkyl, $C_5$-$C_{24}$-Perfluoraryl, 5-12-gliedriges Heteroaryl bedeutet;
Z aus einem solchen Liganden wie $L^1$ ausgewählt ist, und dann die Substituenten $R^{11}$ und $R^{12}$ kombiniert werden, um einen Ring zu bilden, der aus der Gruppe ausgewählt ist, die $C_3$-$C_7$-Cycloalkyl, $C_3$-$C_{25}$-Cycloalkenyl, $C_3$-$C_{25}$-Cycloalkinyl, $C_5$-$C_{24}$-Aryl, $C_5$-$C_{20}$-Heteroaryl, $C_5$-$C_{24}$-Perfluoraryl, 3-12-gliedriger Heterocyclus, umfasst, der unabhängig voneinander durch einen und/oder mehrere Substituenten substituiert sein kann, die aus der Gruppe ausgewählt sind, die ein Wasserstoffatom, ein Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl, $C_2$-$C_{25}$-Alkinyl, $C_3$-$C_{25}$-Cycloalkinyl, $C_1$-$C_{25}$-Alkoxyl, $C_5$-$C_{24}$-Aryloxyl, $C_5$-$C_{20}$-Heteroaryloxyl, $C_5$-$C_{24}$-Aryl, $C_5$-$C_{20}$-Heteroaryl, $C_7$-$C_{24}$-Aralkyl, $C_5$-$C_{24}$-Perfluoraryl, 3-12-gliedriger Heterocyclus umfasst und die gestrichelte Linie zwischen Z und $R^{12}$ keine chemische Bindung zeigt; Heteroatom, ausgewählt aus einer Gruppe, die Sauerstoff-, Stickstoff-, Schwefel- und Phosphoratome umfasst, möglicherweise substituiert durch eine Gruppe, ausgewählt aus einem Wasserstoffatom, $C_1$-$C_{25}$-Alkyl, $C_1$-$C_{25}$-Perfluoralkyl, $C_3$-$C_7$-Cycloalkyl, $C_5$-$C_{24}$-Aryl, $C_5$-$C_{24}$-Perfluoraryl, $C_5$-$C_{20}$-Heteroaryl, $C_7$-$C_{24}$-Aralkyl, 3-12-gliedriger Heterocyclus, eine Acyl- -COR', eine Cyan- (-CN), eine Carboxyl- (-COOH), eine Ester- (-COOR'), eine Amid- (-CONR'$_2$), eine Sulfon- (-$SO_2$R'), eine Formyl- (-CHO), eine Sulfonamid- (-$SO_2$NR'$_2$), eine Ketongruppe (-COR'), wobei die Gruppe R' $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl bedeutet, und dann bedeutet die gestrichelte Linie eine direkte Bindung zwischen dem Heteroatom und dem Substituenten $R^{12}$, der Aryl ist, das möglicherweise durch 1-4 Substituenten substituiert ist, die unabhängig voneinander aus einer Gruppe ausgewählt sind, die ein Wasserstoffatom, ein Halogenatom, $C_1$-$C_{25}$-Alkyl, $C_1$-$C_{25}$-Perfluoralkyl, $C_2$-$C_{25}$-Alken, $C_3$-$C_7$-Cycloalkyl, $C_2$-$C_{25}$-Alkenyl, $C_3$-$C_{25}$-Cycloalkenyl, $C_2$-$C_{25}$-Alkinyl, $C_3$-$C_{25}$-Cycloalkinyl, $C_5$-$C_{24}$-Aryl, $C_7$-$C_{24}$-Aralkyl, $C_5$-$C_{24}$-Perfluoraryl, $C_5$-$C_{20}$-Heteroaryl, 3-12-gliedriger Heterocyclus, eine Alkoxy- (-OR'), eine Sulfid- (-SR'), eine Nitro- (-$NO_2$), eine Cyanid- (-CN), eine Carboxyl- (-COOH), eine Ester- (-COOR'), eine Amid- (-CONR'$_2$), eine Imid- (-CONR'COR'), eine Amin- (-NR'$_2$), eine Ammonium- (-N+R'$_3$), eine Amid- (-NR'COR'), eine Sulfonamid- (-NR'$SO_2$R'), eine Sulfon- (-$SO_2$R'), eine Formyl-(-CHO), eine Sulfonamid- (-$SO_2$NR'$_2$), eine Ketogruppe (-COR') umfasst, wobei die Gruppen R' die folgende Bedeutung haben: $C_1$-$C_5$-Alkyl, $C_1$-$C_5$-Perfluoralkyl, $C_5$-$C_{24}$-Aryl, $C_5$-$C_{24}$-Perfluoraryl, $C_7$-$C_{24}$-Aralkyl;

$X^1$, $X^2$ bedeutet einen anionischen Liganden, der unabhängig aus einer Gruppe ausgewählt ist, die Halogenidanionen, -CN, -SCN, -OR', -SR', -O(C=O)R', -O($SO_2$)R', -OSi(R')$_3$-Gruppe einschließt, wobei R' $C_1$-$C_{12}$-Alkyl, $C_3$-$C_{12}$-Cycloalkyl, $C_2$-$C_{12}$-Alkenyl, $C_5$-$C_{20}$-Aryl, das mit mindestens einem $C_1$-$C_{12}$-Alkyl, $C_1$-$C_{12}$-Perfluoralkyl, $C_1$-$C_{12}$-Alkoxyl, $C_5$-$C_{24}$-Aryoxyl, $C_5$-$C_{20}$-Heteroaryl oder einem Halogenatom substituiert sein kann;

mit dem In-situ entstandenen Carben, das sich aus der Reaktion der Base ergibt, die aus Kalium-tert-amylat, Kalium-tert-butanolat, Kalium-N,N'-bis(trimethylsilyl)amid und Natriumhydrid ausgewählt ist, mit einem Carben-Ausgangsstoff der Formel 4a

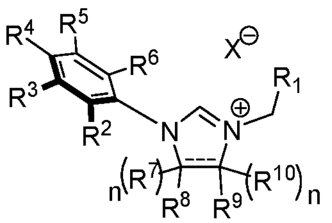

**4a**

wobei:

$R^1$ bedeutet eine Heteroarylgruppe;

$R^2$, $R^3$, $R^4$, $R^5$, $R^6$ unabhängig voneinander ein Wasserstoffatom, eine $C_1$-$C_{25}$-Alkylgruppe, eine $C_1$-$C_{25}$-Alkoxygruppe oder eine $C_2$-$C_{25}$-Alkylgruppe bedeuten, wobei die Substituenten $R^2$, $R^3$, $R^4$, $R^5$, $R^6$ zur Bildung eines substituierten oder unsubstituierten zyklischen Systems $C_4$-$C_{10}$ oder eines polyzyklischen Systems $C_4$-$C_{12}$ kombiniert werden können;

$R^7$, $R^8$, $R^9$ und $R^{10}$ unabhängig voneinander ein Wasserstoffatom oder eine $C_1$-$C_{25}$-Alkylgruppe bedeuten, wobei $R^7$ und/oder $R^8$ mit $R^9$ und/oder $R^{10}$ kombiniert sein können, um ein zyklisches System zu bilden;

n beträgt 0 oder 1;

X- steht für Halogenidanion, oder BF4-, PF6-, ClO4-.

**9.** Herstellungsverfahren für eine Verbindung mit der Formel **1a** oder **1b** nach dem Anspruch **8, dadurch gekennzeichnet, dass** der Alkyliden-Rutheniumkomplex mit der Formel 2 mit dem In-situ generierten Carben, das sich aus der Reaktion der Base ergibt, die aus Kalium-tert-amylat, Kalium-tert-butanolat, Kalium-N,N'-bis(trimethylsilyl)amid und Natriumhydrid ausgewählt ist, mit einem Carben-Ausgangsstoff der Formel **4b**

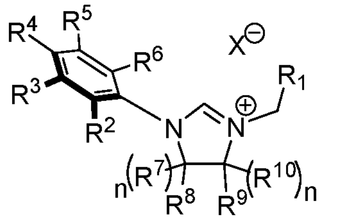

**4b**

wobei:

$R^1$ Heteroaryl bedeutet, ausgewählt aus der Gruppe, die Furan, Thiophen, Benzothiophen, Benzofuran umfasst;
$R^2$, $R^3$, $R^4$, $R^5$, $R^6$ unabhängig das Wasserstoffatom, Methyl, Isopropyl, Halogenatom bedeuten;
$R^7$, $R^8$, $R^9$, $R^{10}$ unabhängig voneinander ein Wasserstoffatom oder Methyl bedeuten;
n beträgt 0 oder 1;
X- steht für Halogenidanion, oder BF4-, PF6-, ClO4-.

10. Herstellungsverfahren für eine Verbindung mit der Formel **1a** oder **1b** nach einem der Ansprüche 6-9, **dadurch gekennzeichnet, dass** als Alkyliden-Rutheniumkomplex die Verbindung mit der Formel **2a** verwendet wird,

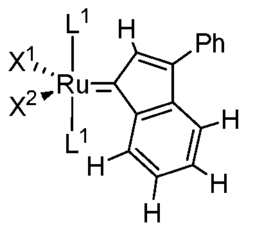

**2a**

wobei:
$X^1$, $X^2$, $L^1$ die gleiche Bezeichnung wie in Formel 2 haben.

11. Verfahren zur Herstellung des Rutheniumkomplexes nach einem der Ansprüche 6-9, **dadurch gekennzeichnet, dass** als Alkyliden-Rutheniumkomplex die Verbindung mit der Formel **2b** verwendet wird,

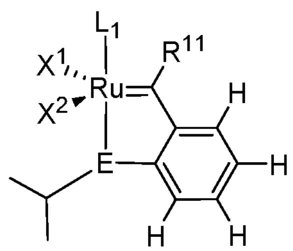

**2b**

wobei:

$X^1$, $X^2$, $L^1$, die gleiche Bedeutung wie oben haben;
$R^{11}$ ein Wasserstoffatom bedeutet;
E bedeutet ein Heteroatom, ausgewählt aus den Atomen wie Sauerstoff, Schwefel, Stickstoff, Phosphor.

12. Verwendung der Verbindung mit der Formel **1a** oder **1b** nach dem Anspruch 1-5, wobei die Verbindung als Vorkatalysator und/oder Katalysator in der Ringschlussmetathese (RCM), Homometathese, Kreuzmetathese (CM), Ethenolyse, Isomerisierung, in der Metathese vom diastereoselektiven Umgruppierungstyp, der Alken-Alkin-Metathese (en□yn) oder in ROMP-Polymerisationsreaktionen, insbesondere in einer metathetischen ROMP-Polymerisationsreaktionen von Dicyclopentadien oder Norbornen, verwendet wird.

**13.** Verwendung gemäß dem Anspruch 12, wobei eine Verbindung der Formel 1a oder 1b als Vorkatalysator und/oder Katalysator in einem Reaktionsgemisch von 1 Minute bis 24 Stunden in unpolaren oder lösungsmittelfreien Lösungsmitteln verwendet wird.

**14.** Verwendung der Verbindung mit der Formel **1a** oder **1b** nach einem der Ansprüche 1-5 als Substrat für die Synthese von anderen Rutheniumkomplexverbindungen, die Vorkatalysatoren und/oder Katalysatoren für die Olefinmetathese sind.

## Revendications

**1.** Le composé selon la revendication 1 de formule **1a**

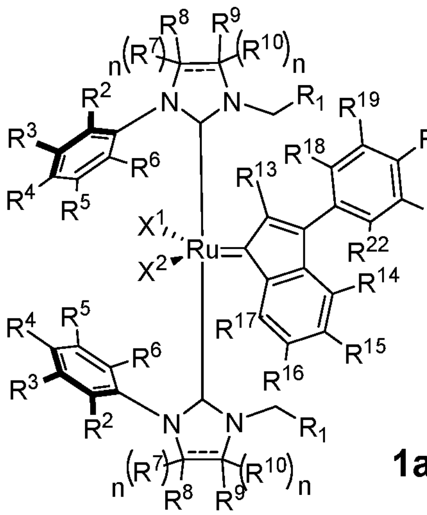

**1a**

où:

$X^1$ et $X^2$ signifient indépendamment un ligand anionique choisi parmi tels qu'atomes d'halogène, groupe -CN, -SCN, -OR', -SR', -O(C=O)R', $-O(SO_2)R'$, $-OSi(R')_3$, où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryloxy en $C_5$-$C_{20}$, ou atome d'halogène;
$R^1$ siginifie un groupe hétéroaryle;
$R^2$, $R^3$, $R^4$, $R^5$, $R^6$ signifient indépendamment un atome d'hydrogène, un groupe alkyle en $C_1$-$C_{25}$, un groupe alcoxy en $C_1$-$C_{25}$, ou un groupe alcényle en $C_2$-$C_{25}$, et les substituants $R^2$, $R^3$, $R^4$, $R^5$ et $R^6$ peuvent être liés entre eux en formant un système cyclique $C_4$-$C_{10}$ subsitué ou non substitué ou un système polycyclique $C_4$-$C_{12}$;
$R^7$, $R^8$, $R^9$, et $R^{10}$ signifient indépendamment un atome d'hydrogène, un groupe alkyle en $C_1$-$C_{25}$, $R^7$ et/ou $R^8$ peuvent être liés avec $R^9$ et/ou $R^{10}$, en formant un système cyclique;
n est égal à 0 ou 1;
$R^{13}$, $R^{14}$, $R^{15}$, $R^{16}$, $R^{17}$, $R^{18}$, $R^{19}$, $R^{20}$, $R^{21}$, et $R^{22}$ signifient indépendamment un atome d'hydrogène, un atome d'halogène, un groupe alkyle en $C_1$-$C_{25}$, alkylamine en $C_1$-$C_{25}$, alkylammonium en $C_1$-$C_{25}$, perfluoroalkyle en $C_1$-$C_{25}$, alcényle en $C_2$-$C_{25}$, cycloalkyle en $C_3$-$C_7$, cycloalcényle en $C_3$-$C_{25}$, alcynyle en $C_2$-$C_{25}$, cycloalcynyle en $C_3$-$C_{25}$, alcoxy en $C_1$-$C_{25}$, aryle en $C_5$-$C_{24}$, hétéroaryle en $C_5$-$C_{20}$, hétérocyclique en $C_3$-$C_{12}$, hétérocycle à 3-12 chaînons, sulfide (-SR'), ester (-COOR'), amide ($-CONR'_2$), sulfone ($-SO_2R'$), sulfonamide ($-SO_2NR'_2$), ou cétone (-COR'), où le groupe R' signifie un groupe alkyle en $C_1$-$C_5$, perfluoroalkyle en $C_1$-$C_5$, aryle en $C_5$-$C_{25}$, ou perfluoroaryle en $C_5$-$C_{25}$.

**2.** Le composé selon la revendication 2 de formule **1a,**
où:

$R^1$ signifie un hétéroaryle choisi dans le groupe consistant en furane, thiophène, benzothiophène, benzofurane;
$R^2$, $R^3$, $R^4$, $R^5$, $R^6$ signifient indépendamment un atome d'hydrogène, méthyle, isopropyle, un atome d'halogène;
$R^7$, $R^8$, $R^9$, $R^{10}$ signifient indépendamment un atome d'hydrogène ou méthyle;
n est égal à 0 ou 1;
$X^1$, $X^2$ siginifie un atome d'halogène;
$R^{13}$, $R^{14}$, $R^{15}$, $R^{16}$, $R^{17}$, $R^{18}$, $R^{19}$, $R^{20}$, $R^{21}$, et $R^{22}$ signifient indépendamment un atome d'hydrogène, un atome d'halogène, un groupe alkyle en $C_1$-$C_{25}$, perfluoroalkyle en $C_1$-$C_{25}$, alcoxy en $C_1$-$C_{25}$.

**3.** Le composé selon la revendication 1 de formule **1b,**

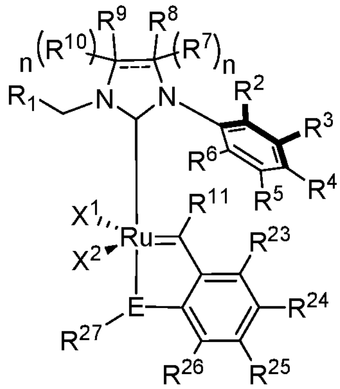

**1b**

où:

$X^1$ i $X^2$ signifient indépendamment un ligand anionique choisi parmi tels qu'atomes d'halogène, groupe -CN, -SCN, -OR', -SR', -O(C=O)R', -O($SO_2$)R', -OSi(R')$_3$, où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryloxy en $C_5$-$C_{20}$ ou atome d'halogène;
$R^1$ signifie un groupe hétéroaryle;
$R^2$, $R^3$, $R^4$, $R^5$, $R^6$ signifient indépendamment un atome d'hydrogène, un groupe alkyle en $C_1$-$C_{25}$, groupe alcoxy en $C_1$-$C_{25}$, ou groupe alcényle en $C_2$-$C_{25}$, et les substituants $R^2$, $R^3$, $R^4$, $R^5$ et $R^6$ peuvent être liés entre eux en formant un système cyclique $C_4$-$C_{10}$ subsitué ou non substitué ou un système polycyclique $C_4$-$C_{12}$;
$R^7$, $R^8$, $R^9$, et $R^{10}$ signifient indépendamment un atome d'hydrogène ou un groupe alkyle en $C_1$-$C_{25}$, $R^7$ et/ou $R^8$ peuvent être liés à
$R^9$ et/ou $R^{10}$, en formant un système cyclique;
n est égal à 0 ou 1;
$R^{11}$ signifie un atome d'hydrogène;
$R^{23}$, $R^{24}$, $R^{25}$, $R^{26}$ signifient indépendamment un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, aryle $C_5$-$C_{24}$, aralkyle en $C_7$-$C_{24}$, perfluoroaryle $C_5$-$C_{24}$, hétéroaryle $C_5$-$C_{20}$, hétérocycle à 3-12 chaînons, groupe alcoxy (-OR'), sulfide (-SR'), nitro (-$NO_2$), cyano (-CN), carboxyle (-COOH), ester (-COOR'), amide (-CONR'$_2$), imide (-CONR'COR'), amine (-NR'$_2$), ammonium (-N+R'$_3$), amide (-NR'COR'), sulfonamide (-NR'$SO_2$R'), sulfone (-$SO_2$R'), formyle (-CHO), sulfonamide (-$SO_2$NR'$_2$), cétone (-COR'), dans ces groupes R' a la signification suivante: alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$, où $R^{23}$, $R^{24}$, $R^{25}$, $R^{26}$ signifient de préférence un atome d'hydrogène;
$R^{27}$ signifie un atome d'hydrogène, alkyle en $C_1$-$C_{25}$, perfluoroalkyle en $C_1$-$C_{25}$, cycloalkyle en $C_3$-$C_7$, aryle en $C_5$-$C_{24}$, perfluoroaryle en $C_5$-$C_{24}$, hétéroaryle en $C_5$-$C_{20}$, aralkyle en $C_7$-$C_{24}$, hétérocycle à 3-12 chaînons, groupe acyle -COR', cyano (-CN), carboxyle (-COOH), ester (-COOR'), amide (-CONR'$_2$), sulfone (-$SO_2$R'), formyle (-CHO), sulfonamide (-$SO_2$NR'$_2$), cétone (-COR'), où groupe R' signifie alkyle en $C_1$-$C_5$, perfluoroalkyle en $C_1$-$C_5$, aryle en $C_5$-$C_{24}$,
perfluoroaryle en $C_5$-$C_{24}$, aralkyle en $C_7$-$C_{24}$;
E signifie en hétéroatome choisi parmi les atomes tels qu'atome d'oxygène, de soufre, d'azote, de phosphore.

4. Le composé selon la revendication 3 de formule **1b,**
où:

$R^1$ signifie un hétéroaryle choisi dans le groupe consistant en furane, thiophène, benzothiophène, benzofurane;
$R^2$, $R^3$, $R^4$, $R^5$, $R^6$ signifient indépendamment un atome d'hydrogène, méthyle, isopropyle, un atome d'halogène;
$R^7$, $R^8$, $R^9$, $R^{10}$ signifient indépendamment un atome d'hydrogène ou méthyle;
n est égal à 0 ou 1;
$X^1$, $X^2$ signifient un atome d'halogène;
$R^{11}$ signifie un atome d'hydrogène;
$R^{23}$, $R^{24}$, $R^{25}$, $R^{26}$ signifient indépendamment un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, aryle en $C_5$-$C_{24}$, aralkyle en $C_7$-$C_{24}$, perfluoroaryle en $C_5$-$C_{24}$, hétéroaryle en $C_5$-$C_{20}$, hétérocycle à 3-12 chaînons, groupe alcoxy (-OR'), sulfide (-SR'), nitro ($-NO_2$), cyano (-CN), carboxyle (-COOH), ester (-COOR'), amide ($-CONR'_2$), imide (-CONR'COR'), amine ($-NR'_2$), ammonium ($-N+R'_3$), amide (-NR'COR'), sulfonamide ($-NR'SO_2R'$), sulfone ($-SO_2R'$), formyle (-CHO), sulfonamide ($-SO_2NR'_2$), cétone (-COR'), où R' signifie alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$, hétérocycle à 3-12 chaînons, où $R^{23}$, $R^{24}$, $R^{25}$, $R^{26}$ signifie de préférence un atome d'hydrogène;
$R^{27}$ signifie alkyle en $C_1$-$C_{25}$, perfluoroalkyle en $C_1$-$C_{25}$, cycloalkyle en $C_3$-$C_7$, aryle en $C_5$-$C_{24}$, perfluoroaryle en $C_5$-$C_{24}$, hétéroaryle en $C_5$-$C_{20}$, aralkyle en $C_7$-$C_{24}$, hétérocycle à 3-12 chaînons, groupe acyle -COR', cyano (-CN), carboxyle (-COOH), ester (-COOR'), amide ($-CONR'_2$), sulfone ($-SO_2R'$), formyle (-CHO), sulfonamide ($-SO_2NR'_2$), cétone (-COR'), où R' signifie alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$;
E signifie en hétéroatome choisi parmi les atomes tels qu'atome d'oxygène ou un atome de soufre.

5. Le composé selon la revendication **1** ou la revendication **2,** avec une structure représentée par une formule choisie parmi telles que: **1aa, 1ab, 1ac, 1ba** et **1bb**:

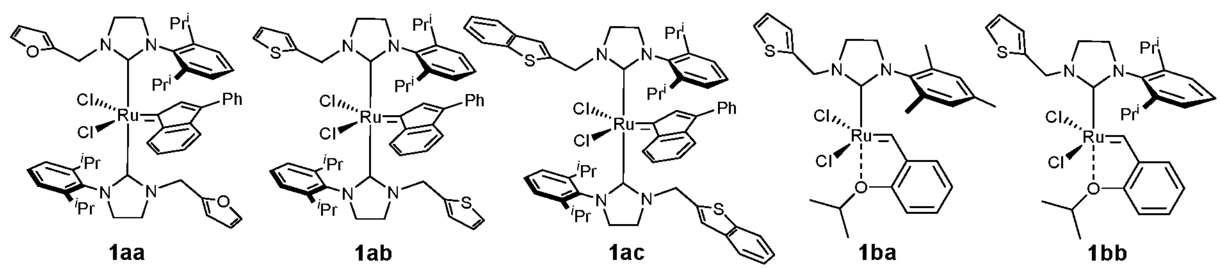

**1aa** **1ab** **1ac** **1ba** **1bb**

6. Procédé de préparation d'un composé de formule **1a** ou **1b** selon l'une quelconque des revendications 1 à 5 **caractérisé en ce que** le complexe alkylidène ruthénium de formule **2**

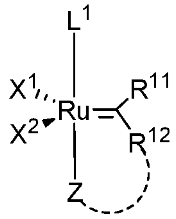

**2**

où:

$R^{11}$, $R^{12}$ signifient indépendamment un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, alcoxy en $C_1$-$C_{25}$, aryloxy en $C_5$-$C_{24}$, hétéroaryloxy en $C_5$-$C_{20}$, aryle en $C_5$-$C_{24}$, hétéroaryle en $C_5$-$C_{20}$, aralkyle en $C_7$-$C_{24}$, perfluoroaryle en $C_5$-$C_{24}$, hétérocycle à 3-12 chaînons,

où les substituants $R^{11}$ et $R^{12}$ peuvent être liés entre eux en formant un cycle choisi dans le groupe consistant en cycloalkyle en $C_3$-$C_7$, cycloalcényle en $C_3$-$C_{25}$, cycloalcynyle en $C_3$-$C_{25}$, aryle en $C_5$-$C_{24}$, hétéroaryle en $C_5$-$C_{20}$, perfluoroaryle en $C_5$-$C_{24}$, qui peut être indépendamment substitué par un et/ou plusieurs substituants choisis dans le groupe consistant en un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, alcoxy en $C_1$-$C_{25}$, aryloxy en $C_5$-$C_{24}$, hétéroaryloxy en $C_5$-$C_{20}$, aryle en $C_5$-$C_{24}$, hétéroaryle en $C_5$-$C_{20}$, aralkyle en $C_7$-$C_{24}$, perfluoroaryle en $C_5$-$C_{24}$, hétérocycle à 3-12 chaînons;

où $R^{11}$, $R^{12}$ signifient de préférence un atome d'hydrogène ou aryle indépendamment substitué par un groupe alcoxy (-OR'), sulfide (-SR'), sulfoxyde (-S(O)R'), sulfonium ($-S^+R'_2$), sulfone ($-SO_2R'$), sulfonamide ($-SO_2NR'_2$), amine ($-NR'_2$), ammonium ($-N+R'_3$), nitro ($-NO_2$), cyano (-CN), phosphonite ($-P(O)(OR)_2$), phosphinite (-P(O)R'(OR')), phosphinate ($-P(OR')_2$), phosphine ($-PR'_2$), oxydes de phospines ($-P(O)R'_2$), phosphonate ($-P^+R'_3$), carboxyle (-COOH), ester (-COOR'), amide ($-CONR'_2$), formyle (-CHO), cétone (-COR'), où R' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$;

$L^1$ signifie un ligand neutre choisi dans le groupe constitant en pyridine ou pyridine substituée, $P(R')_3$, $P(OR')_3$, $O(R')_2$, $N(R')_3$, où chaque R' signifie indépendamment alkyle en $C_1$-$C_{12}$, cycloalkyle en $C_3$-$C_{12}$, aryle en $C_5$-$C_{20}$, aralkyle en $C_7$-$C_{24}$, perfluoroaryle en $C_5$-$C_{24}$, hétéroaryle à 5-12 chaînons;

Z est choisi parmi tels que ligand $L^1$, et alors les substituants $R^{11}$ et $R^{12}$ sont liés entre eux en formant un cycle choisi dans le groupe consistant en cycloalkyle en $C_3$-$C_7$, cycloalcényle en $C_3$-$C_{25}$, cycloalcynyle en $C_3$-$C_{25}$, aryle en $C_5$-$C_{24}$, hétéroaryle en $C_5$-$C_{20}$, perfluoroaryle en $C_5$-$C_{24}$, hétérocycle à 3-12 chaînons, qui peut être indépendamment substitué par un et / ou plusieurs substituants choisis dans le groupe consistant en un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, alcoxy en $C_1$-$C_{25}$, aryloxy en $C_5$-$C_{24}$, hétéroaryloxy en $C_5$-$C_{20}$, aryle en $C_5$-$C_{24}$, hétéroaryle en $C_5$-$C_{20}$, aralkyle en $C_7$-$C_{24}$, perfluoroaryle en $C_5$-$C_{24}$, hétérocycle à 3-12 chaînons, et la ligne en pointillé entre Z et $R^{12}$ ne signifie pas de laison chimique; un hétéroatome choisi dans le groupe consistant en atome d'oxygène, azote, soufre et phosphore, éventuellement substitué par un groupe choisi parmi tel qu'atome d'hydrogène, alkyle en $C_1$-$C_{25}$, perfluoroalkyle en $C_1$-$C_{25}$, cycloalkyle en $C_3$-$C_7$, aryle en $C_5$-$C_{24}$, perfluoroaryle en $C_5$-$C_{24}$, hétéroaryle en $C_5$-$C_{20}$, aralkyle en $C_7$-$C_{24}$, hétérocycle à 3-12 chaînons, groupe acyle -COR', cyano (-CN), carboxyle (-COOH), ester (-COOR'), amide ($-CONR'_2$), sulfone ($-SO_2R'$), formyle (-CHO), sulfonamide ($-SO_2NR'_2$), cétone (-COR'), où groupe R' signifie alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$, et alors la ligne en pointillé signifie une laison directe de l'hétératome avec le substituant $R^{12}$, qui est aryle éventuellement substitué par 1-4 substituants indépendamment choisis dans le groupe consistant en un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, aryle en $C_5$-$C_{24}$, aralkyle en $C_7$-$C_{24}$, perfluoroaryle en $C_5$-$C_{24}$, hétéroaryle en $C_5$-$C_{20}$, hétérocycle à 3-12 chaînons, groupe alcoxy (-OR'), sulfide (-SR'), nitro ($-NO_2$), cyano (-CN), carboxyle (-COOH), ester (-COOR'), amide ($-CONR'_2$), imide (-CONR'COR'), amine ($-NR'_2$), ammonium ($-N^+R'_3$), amide (-NR'COR'), sulfonamide ($-NR'SO_2R'$), sulfone ($-SO_2R'$), formyle (-CHO), sulfonamide ($-SO_2NR'_2$), cétone (-COR'), où R' a la signification suivante: alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$;

$X^1$, $X^2$ signifient un ligand anionique indépendamment choisi dans le groupe consistant en anions halogénures, groupe -CN, -SCN, -OR', -SR', -O(C=O)R', $-O(SO_2)R'$, $-OSi(R')_3$, où R' signifie alkyle en $C_1$-$C_{12}$, cycloalkyle en $C_3$-$C_{12}$, alcényle en $C_2$-$C_{12}$, ou aryle en $C_5$-$C_{20}$, qui est éventuellement substitué par au moins un alkyle en $C_1$-$C_{12}$, perfluoroalkyle en $C_1$-$C_{12}$, alcoxy en $C_1$-$C_{12}$, aryloxy en $C_5$-$C_{24}$, hétéroaryloxy en $C_5$-$C_{20}$ ou un atome d'halogène;

réagit avec carbène de fomule **3a**

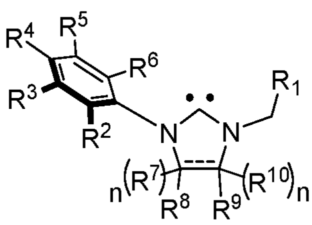

**3a**

où:

$R^1$ signifie un groupe hétéroaryle;
$R^2$, $R^3$, $R^4$, $R^5$, $R^6$ signifient indépendamment un atome d'hydrogène, un groupe alkyle en $C_1$-$C_{25}$, alcoxy en $C_1$-$C_{25}$, ou alcényle en $C_2$-$C_{25}$, et les substituants $R^2$, $R^3$, $R^4$, $R^5$, $R^6$ peuvent être liés entre eux en formant un système cyclique $C_4$-$C_{10}$ subsitué ou non substitué ou un système polycyclique $C_4$-$C_{12}$;
$R^7$, $R^8$, $R^9$, et $R^{10}$ signifient indépendamment un atome d'hydrogène ou un groupe alkyle en $C_1$-$C_{25}$, $R^7$ et/ou $R^8$ peuvent être liés à $R^9$ e/ou $R^{10}$, en formant un système cyclique;
n est égal à 0 ou 1.

**7.** Procédé de préparation d'un composé de formule **1a** ou **1b** selon la revendication 6 **caractérisé en ce que** le complexe alkylidène ruthénium de formule **2** réagit avec carbène de formule **3b**

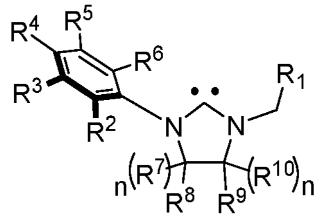

**3b**

où:

$R^1$ signifie hétéroaryle choisi dans le groupe consistant en furane, thiophène, benzothiophène, benzofurane;
$R^2$, $R^3$, $R^4$, $R^5$, $R^6$ signifient indépendamment un atome d'hydrogène, méthyle, isopropyle, un atome d'halogène;
$R^7$, $R^8$, $R^9$, $R^{10}$ signifient indépendamment un atome d'hydrogène ou méthyle;
n est égal à 0 ou 1.

**8.** Procédé de préparation d'un composé de formule **1a** ou **1b** selon l'une quelconque des revendications 1 à 5, **caractérisé en ce que** le complexe alkylidène ruthénium de formule **2**

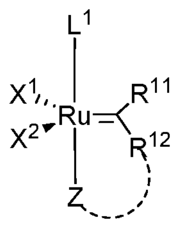

**2**

où:

$R^{11}$, $R^{12}$ signifient indépendamment un atome d'hydrogène, un atome d'halogène, alkyle en $C_1$-$C_{25}$, perfluoroalkyle en $C_1$-$C_{25}$, alcène $C_2$-$C_{25}$, cycloalkyle en $C_3$-$C_7$, acényle en $C_2$-$C_{25}$, cycloalcényle en $C_3$-$C_{25}$, alcynyle en $C_2$-$C_{25}$, cycloalcynyle en $C_3$-$C_{25}$, alcoxy en $C_1$-$C_{25}$, aryloxy en $C_5$-$C_{24}$, hétéroaryloxy en $C_5$-$C_{20}$, aryle en $C_5$-$C_{24}$, hétéroaryle en $C_5$-$C_{20}$, aralkyle en $C_7$-$C_{24}$, perfluoroaryle en $C_5$-$C_{24}$, hétérocycle à 3-12 chaînons, et $R^{11}$ et $R^{12}$ signifient de préférence un atome d'hydrogène ou aryle substitué par un groupe nitro ($-NO_2$), cyano (-CN), carboxyle (-COOH), ester (-COOR'), amide ($-CONR'_2$), sulfone ($-SO_2R'$), formyle (-CHO), sulfonamide ($-SO_2NR'_2$), cétone (-COR'), où R' signifie alkyle en $C_1$-$C_5$, perfluoroalkyle en $C_1$-$C_5$, aryle en $C_5$-$C_{24}$, aralkyle en $C_7$-$C_{24}$, perfluoroaryle en $C_5$-$C_{24}$;
$L^1$ signifie un ligand neutre choisi dans le groupe constitant en pyridine ou pyridine substituée, $P(R')_3$, $P(OR')_3$, $O(R')_2$, $N(R')_3$, où chaque R' signifie indépendamment alkyle en $C_1$-$C_{12}$, cycloalkyle en $C_3$-$C_{12}$, aryle en $C_5$-$C_{20}$, aralkyle en $C_7$-$C_{24}$, perfluoroaryle en $C_5$-$C_{24}$, hétéroaryle à 5-12 chaînons;
Z est choisi parmi tels que ligand $L^1$, et alors les substituants $R^{11}$ et $R^{12}$ sont liés entre eux en formant un cycle choisi dans le groupe consistant en cycloalkyle en $C_3$-$C_7$, cycloalcényle en $C_3$-$C_{25}$, cycloalcynyle en $C_3$-$C_{25}$,

aryle en $C_5$-$C_{24}$, hétéroaryle en $C_5$-$C_{20}$, perfluoroaryle en $C_5$-$C_{24}$, hétérocycle à 3-12 chaînons, qui peut être indépendamment substitué par un et / ou plusieurs substituants choisis dans le groupe consistant en un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, alcoxy en $C_1$-$C_{25}$, aryloxy en $C_5$-$C_{24}$, hétéroaryloxy en $C_5$-$C_{20}$, aryle en $C_5$-$C_{24}$, hétéroaryle en $C_5$-$C_{20}$, aralkyle en $C_7$-$C_{24}$, perfluoroaryle en $C_5$-$C_{24}$, hétérocycle à 3-12 chaînons, et la ligne en pointillé entre Z et $R^{12}$ ne signifie pas de laison chimique; un hétéroatome choisi dans le groupe consistant en un atome d'oxygène, azote, soufre et phosphore, éventuellement substitué par un groupe choisi parmi tel qu'atome d'hydrogène, alkyle en $C_1$-$C_{25}$, perfluoroalkyle en $C_1$-$C_{25}$, cycloalkyle en $C_3$-$C_7$, aryle en $C_5$-$C_{24}$, perfluoroaryle en $C_5$-$C_{24}$, hétéroaryle en $C_5$-$C_{20}$, aralkyle en $C_7$-$C_{24}$, hétérocycle à 3-12 chaînons, groupe acyle -COR', cyano (-CN), carboxyle (-COOH), ester (-COOR'), amide (-CONR'$_2$), sulfone (-$SO_2$R'), formyle (-CHO), sulfonamide (-$SO_2$NR'$_2$), cétone (-COR'), où le groupe R' signifie alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$, et alors la ligne en pointillé signifie une laison directe de l'hétératome avec le substituant $R^{12}$, qui est aryle éventuellement substitué par 1-4 substituants indépendamment choisis dans le groupe consistant en un atome d'hydrogène, un atome d'halogène, alkyle en $C_1$-$C_{25}$, perfluoroalkyle en $C_1$-$C_{25}$, alcène en $C_2$-$C_{25}$, cycloalkyle en $C_3$-$C_7$, alcényle en $C_2$-$C_{25}$, cycloalcényle en $C_3$-$C_{25}$, alcynyle en $C_2$-$C_{25}$, cycloalcynyle en $C_3$-$C_{25}$, aryle en $C_5$-$C_{24}$, aralkyle en $C_7$-$C_{24}$, perfluoroaryle en $C_5$-$C_{24}$, hétéroaryle en $C_5$-$C_{20}$, hétérocycle à 3-12 chaînons, groupe alcoxy (-OR'), sulfide (-SR'), nitro (-$NO_2$), cyano (-CN), carboxyle (-COOH), ester (-COOR'), amide (-CONR'$_2$), imide (-CONR'COR'), amine (-NR'$_2$), ammonium (-$N^+$R'$_3$), amide (-NR'COR'), sulfonamide (-NR'$SO_2$R'), sulfone (-$SO_2$R'), formyle (-CHO), sulfonamide (-$SO_2$NR'$_2$), cétone (-COR'), où R' a la signification suivante: alkyle en $C_1$-$C_5$, perfluoroalkyle en $C_1$-$C_5$, aryle en $C_5$-$C_{24}$, perfluoroaryle en $C_5$-$C_{24}$, aralkyle en $C_7$-$C_{24}$;

$X^1$, $X^2$ signifient un ligand anionique indépendamment choisi dans le groupe consistant en anions halogénures, groupe -CN, -SCN, -OR', -SR', -O(C=O)R', -O($SO_2$)R', -OSi(R')$_3$, où R' signifie alkyle en $C_1$-$C_{12}$, cycloalkyle en $C_3$-$C_{12}$, alcényle en $C_2$-$C_{12}$, aryle en $C_5$-$C_{20}$, qui sont éventuellement substitués par au moins un alkyle en $C_1$-$C_{12}$, perfluoroalkyle en $C_1$-$C_{12}$, alcoxy en $C_1$-$C_{12}$, aryloxy $C_5$-$C_{24}$, hétéroaryloxy en $C_5$-$C_{20}$ ou un atome d'halogène;

réagit avec carbène formé *in situ* par l'action d'une base choisie parmi telles que *tert*-amylate de potassium, *tert*-butylate de potassium, *N, N'*-bis (triméthylsilyl) amide de potassium, hydrure de sodium, sur le précurseur de carbène de formule **4a**

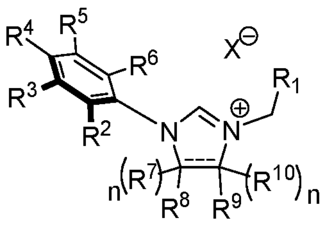

**4a**

où

$R^1$ signifie un groupe hétéroaryle;

$R^2$, $R^3$, $R^4$, $R^5$, $R^6$ signifient indépendamment un atome d'hydrogène, un groupe alkyle en $C_1$-$C_{25}$, alcoxy $C_1$-$C_{25}$, ou alcényle en $C_2$-$C_{25}$, et les substituants $R^2$, $R^3$, $R^4$, $R^5$, $R^6$ peuvent être liés entre eux en formant un système cyclique $C_4$-$C_{10}$ subsitué ou non substitué ou un système polycyclique $C_4$-$C_{12}$;

$R^7$, $R^8$, $R^9$, et $R^{10}$ signifient indépendamment un atome d'hydrogène ou un groupe alkyle en $C_1$-$C_{25}$, $R^7$ et/ou $R^8$ peuvent être liés à $R^9$ e/ou $R^{10}$, en formant un système cyclique;

n est égal à 0 ou 1;

$X^-$ signifie un anion halogénure, ou $BF_4^-$, $PF_6^-$, $ClO_4^-$.

9. Procédé de préparation d'un composé de formule **1a** ou **1b** selon la revendication 8 **caractérisé en ce que** le complexe alkylidène ruthénium de formule **2** réagit avec carbène généré *in situ* par l'action d'une base choisie parmi telles que *tert*-amylate de potassium, *tert*-butylate de potassium, *N*, *N*'-bis (triméthylsilyl) amide de potassium, hydrure de sodium, sur le précurseur de carbène de formule **4b**

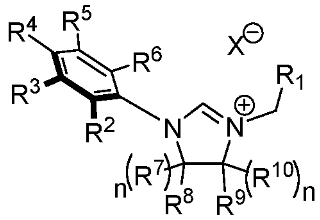

**4b**

où:

$R^1$ signifie hétéroaryle choisi dans le groupe consistant en furane, thiophène, benzothiophène, benzofurane;
$R^2$, $R^3$, $R^4$, $R^5$, $R^6$ signifient indépendamment un atome d'hydrogène, méthyle, isopropyle, un atome d'halogène;
$R^7$, $R^8$, $R^9$, $R^{10}$ signifient indépendamment un atome d'hydrogène ou méthyle;
n est égal à 0 ou 1
$X^-$ signifie un anion halogénure ou $BF_4^-$, $PF_6^-$, $ClO_4^-$.

**10.** Procédé de préparation d'un composé de formule **1a** ou **1b** selon l'une quelconque des revendications 6 à 9, **caractérisé en ce qu'**en tant que complexe alkylidène ruthénium est utilisé le composé de formule **2a**

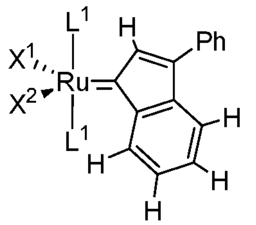

**2a**

où:
$X^1$, $X^2$, $L^1$ ont la même signification que dans la formule **2.**

**11.** Procédé de préparation du complexe ruthénium selon l'une quelconque des revendications 6 à 9, **caractérisé en ce qu'**en tant que complexe alkylidène ruthénium est utilisé le composé de formule **2b**

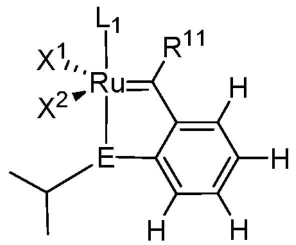

**2b**

où:

$X^1$, $X^2$, $L^1$, ont la même signification comme ci-dessus;
$R^{11}$ signifie un atome d'hydrogène;
E signifie en hétéroatome choisi parmi les atomes tels qu'atome d'oxygène, soufre, azote, phosphore.

**12.** L'utilisation du composé de formule **1a** ou **1b** selon les revendications 1 à 5 comme précatalyseur et/ou cataliseur dans les réactions de métathèse des oléfines, telles que les réactions de métathèse de fermeture de cycle (RCM), homométathèse, métathèse croisée (CM), éthénolyse, isomérisation, dans la réaction de métathèse diastéréosélective de réarrangement de cycle (DRRM), métathèse type "alcène-alcyne" (ène-yne) ou dans les réactions de polymérisation type ROMP, en particulier dans la réaction de métathèse de polymérisation par ouverture de cycle (ROMP) du dicyclopentadiène ou du norbornène.

**13.** L'utilisation selon la revendication 12, où le composé de formule **1a** ou **1b** est utilisé comme précatalyseur et / ou catalyseur dans le mélange réactionnel pendant la durée d'une minute à 24 heures, dans des solvants non polaires ou sans solvant.

**14.** L'utilisation d'un composé de formule **1a** ou **1b** selon l'une quelconque des revendications 1 à 5, comme substrat pour la synthèse d'autres composés complexes du ruthénium qui sont des précatalyseurs et / ou des catalyseurs pour la métathèse des oléfines.

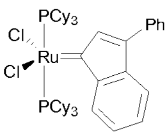

M1
(Ind-I)

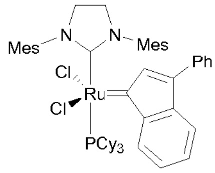

M2
(Ind-II)

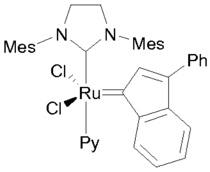

M31
(Ind-III)

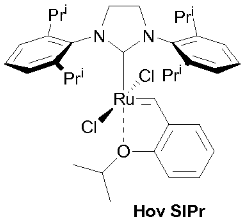

Hov SIPr

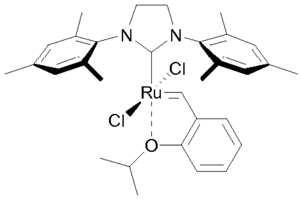

Hov SIMes
(Hov-II)

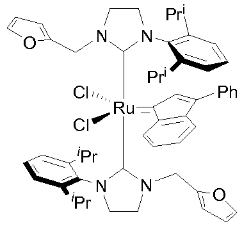

1aa

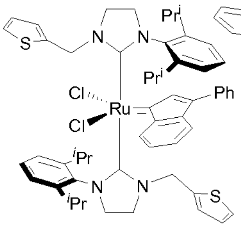

1ab

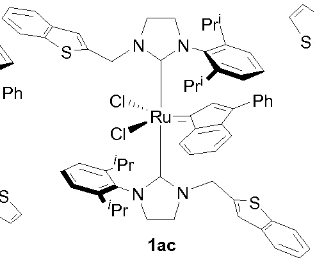

1ac

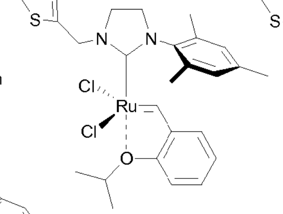

1ba

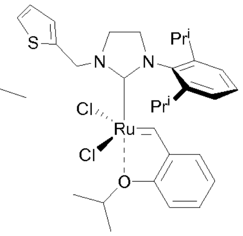

1bb

Fig. 1

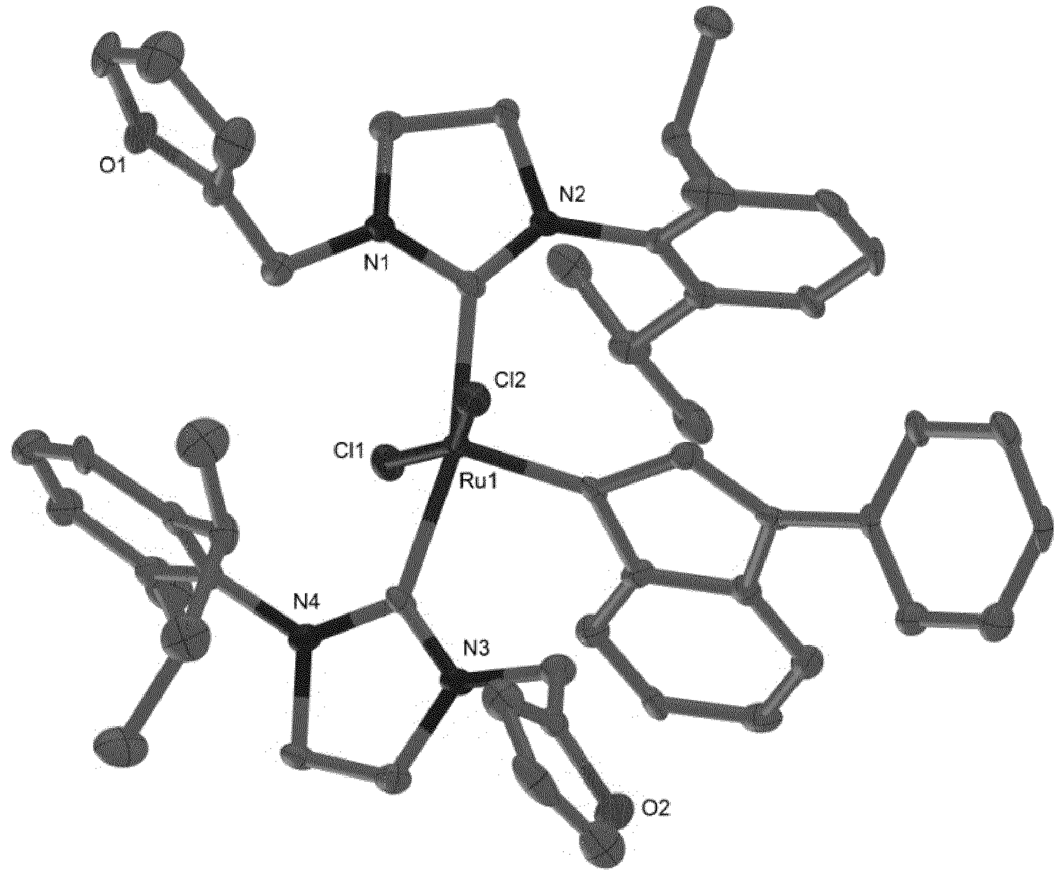

Fig. 2

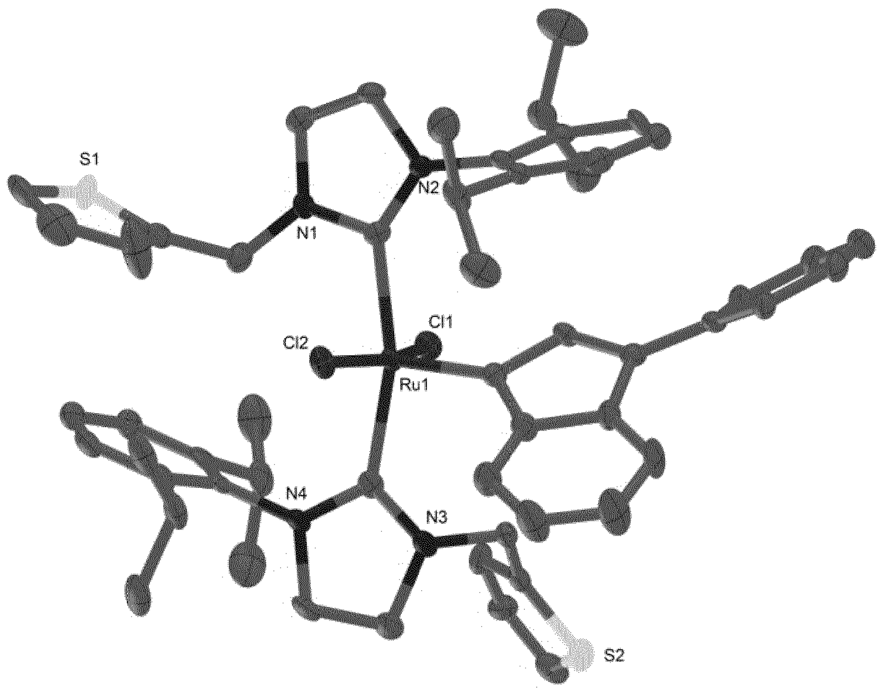


Fig. 3

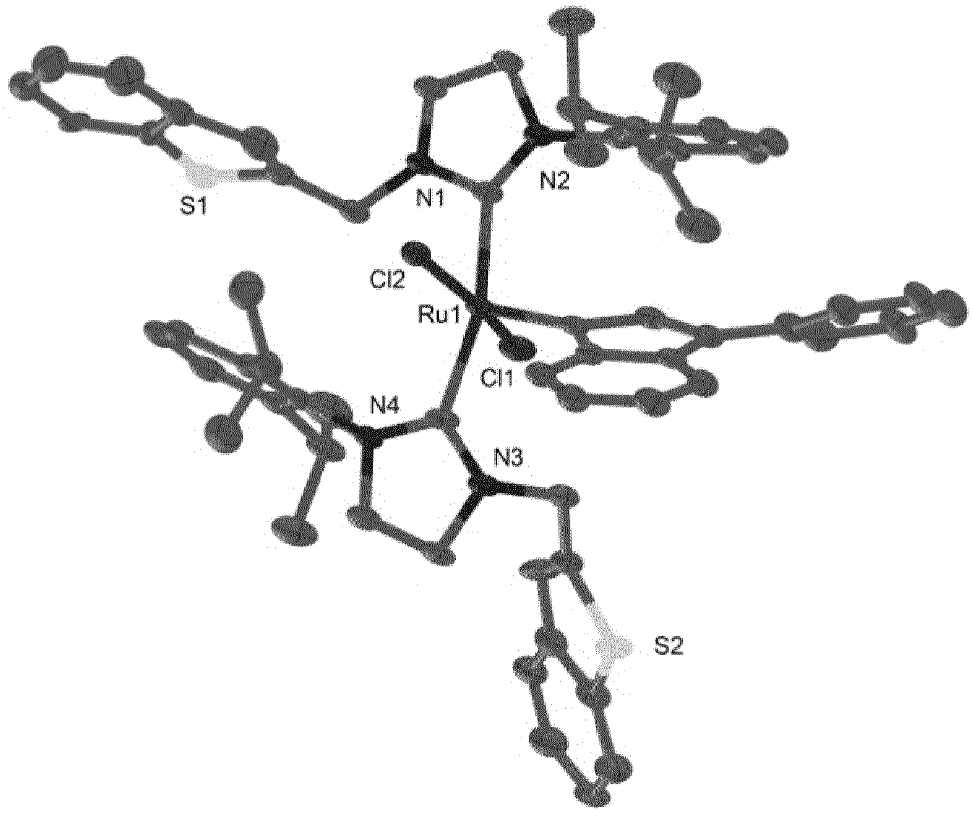


Fig. 4

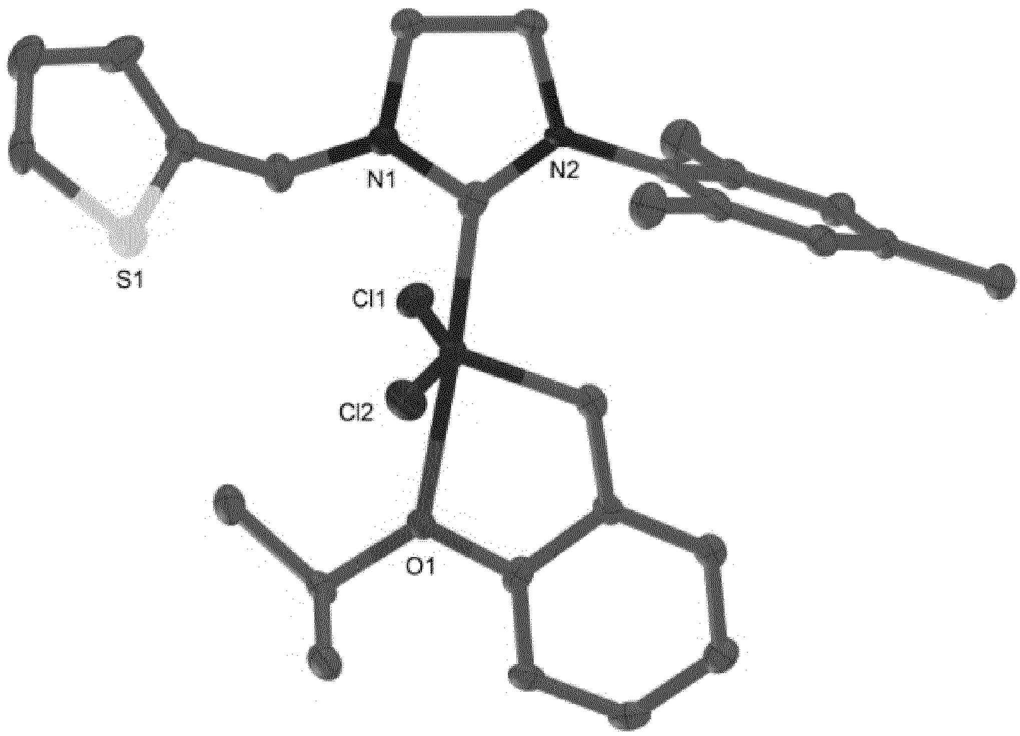


Fig. 5

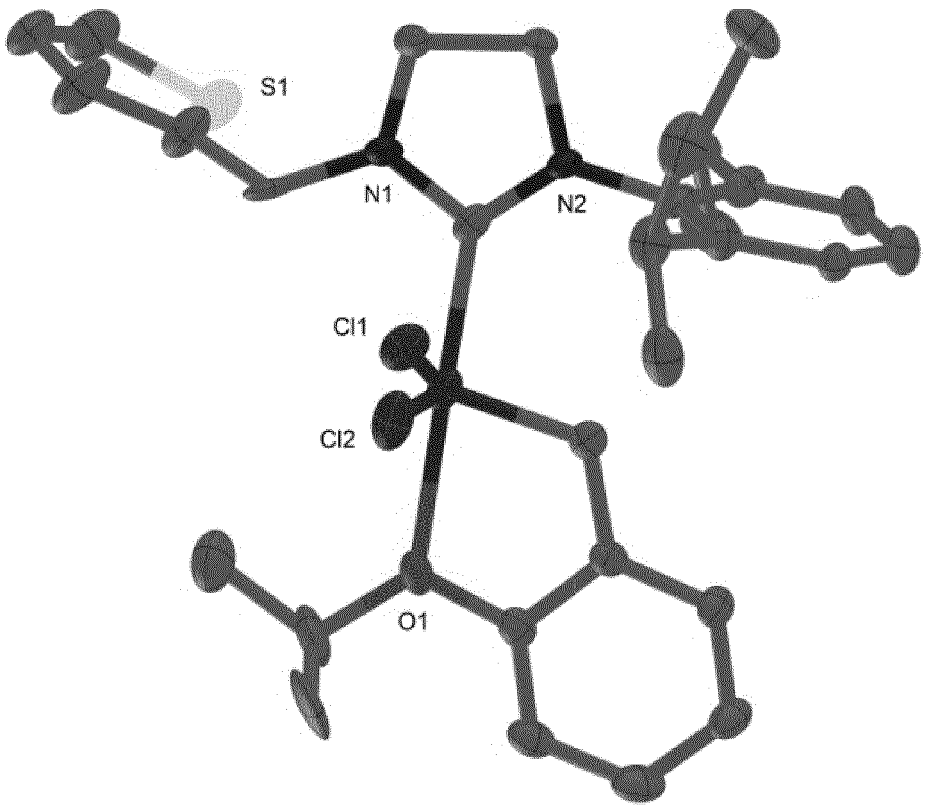


Fig. 6

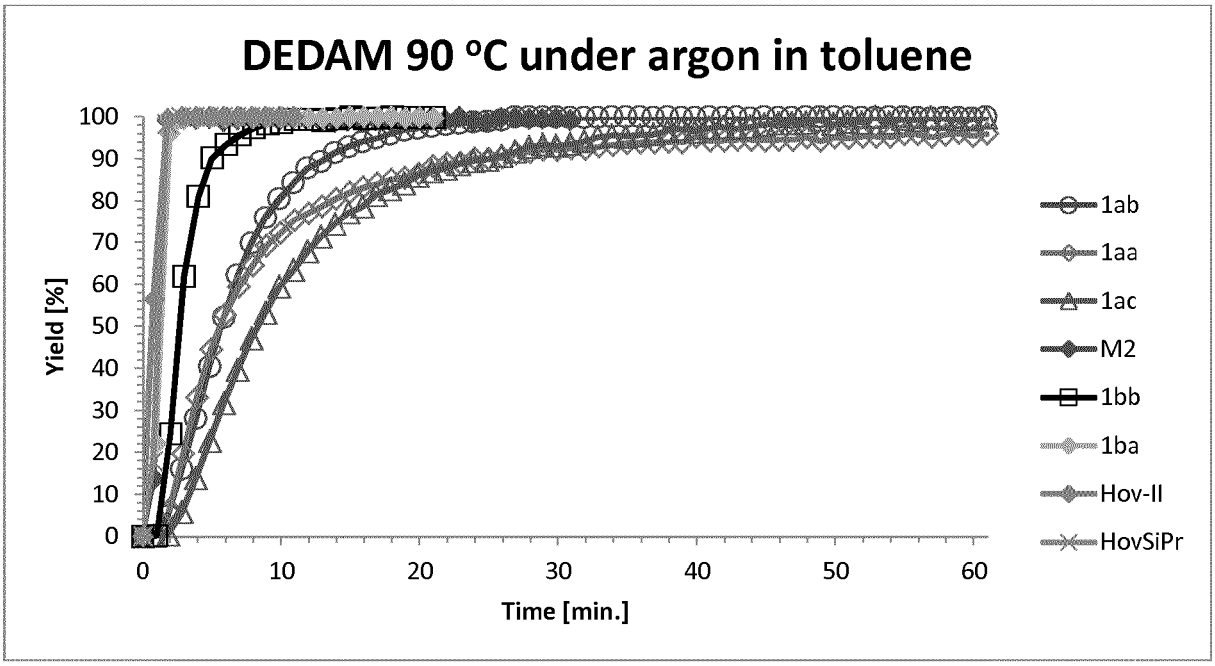
DEDAM 90 °C under argon in toluene
Yield [%]
100
90
80
70
60
50
40
30
20
10
0
0
10
20
30
40
50
60
Time [min.]
1ab
1aa
1ac
M2
1bb
1ba
Hov-II
HovSiPr

Fig. 7

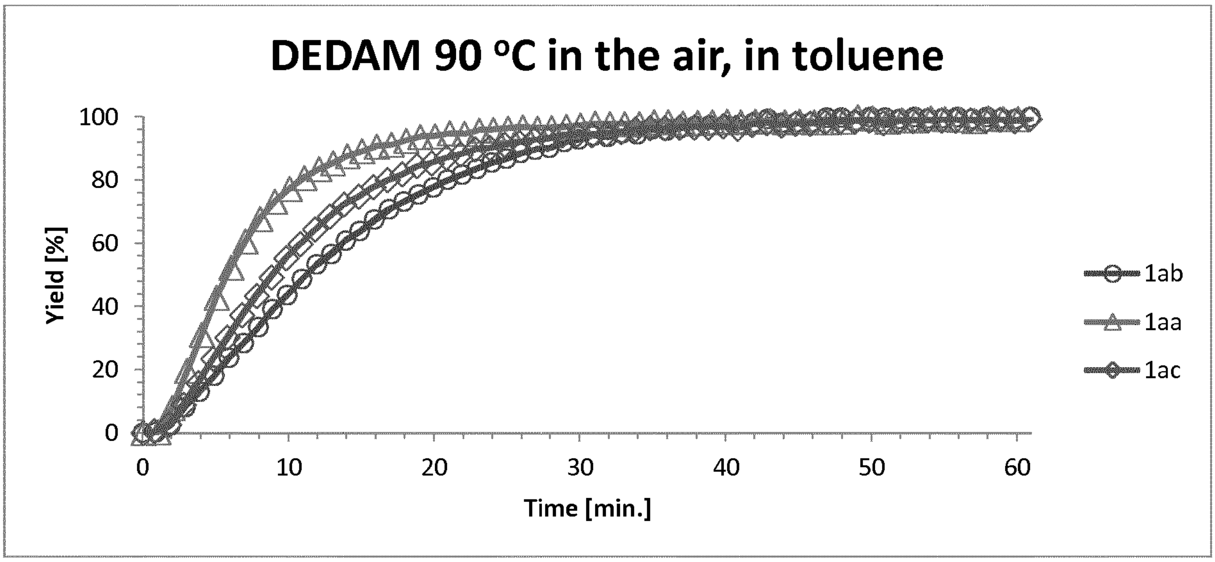
DEDAM 90 °C in the air, in toluene
Yield [%]
100
80
60
40
20
0
0
10
20
30
40
50
60
Time [min.]
1ab
1aa
1ac

Fig. 8

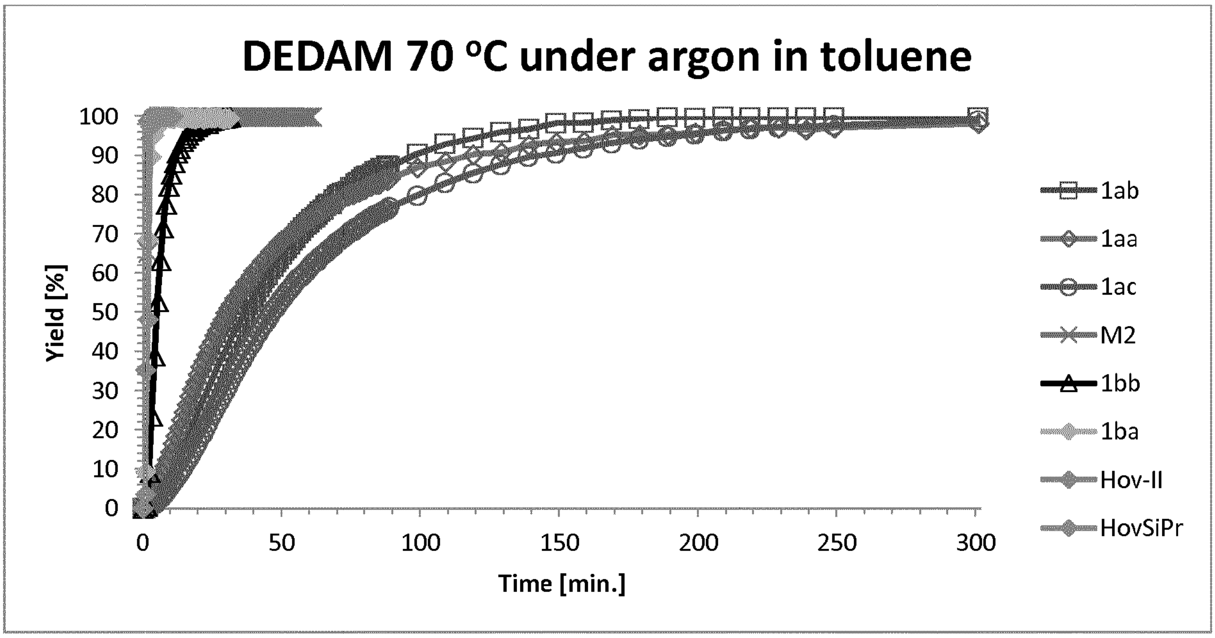
DEDAM 70 °C under argon in toluene
Yield [%]
100
90
80
70
60
50
40
30
20
10
0
0
50
100
150
200
250
300
Time [min.]
1ab
1aa
1ac
M2
1bb
1ba
Hov-II
HovSiPr

Fig. 9

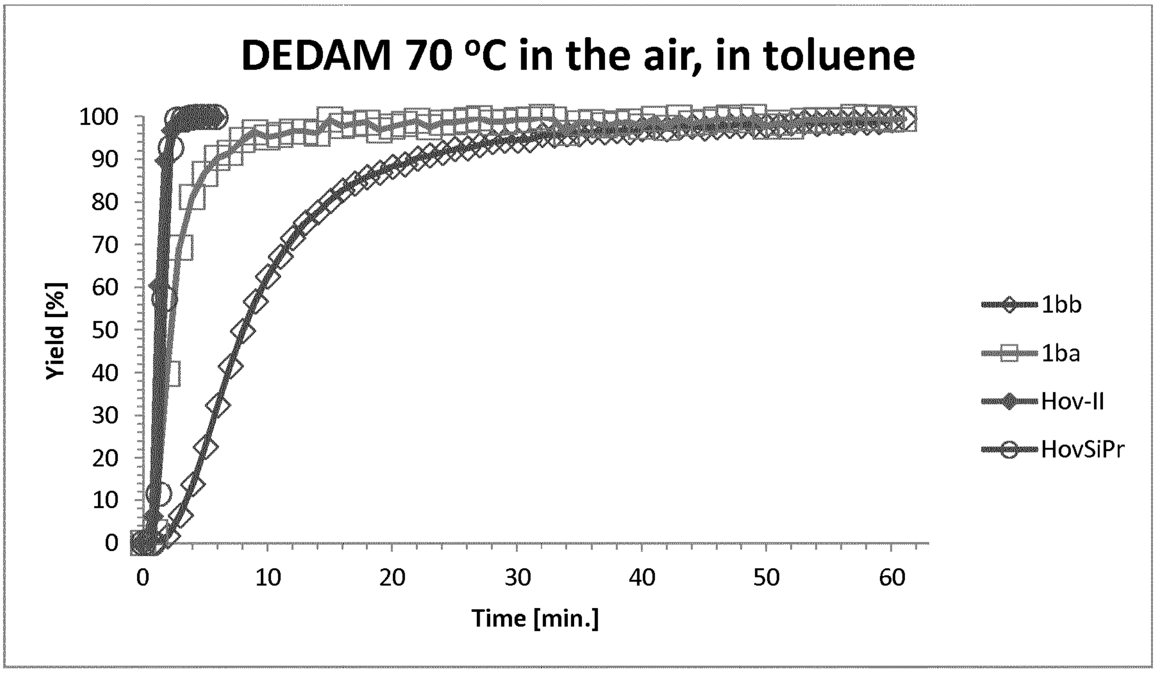
DEDAM 70 °C in the air, in toluene
Yield [%]
100
90
80
70
60
50
40
30
20
10
0
0
10
20
30
40
50
60
Time [min.]
1bb
1ba
Hov-II
HovSiPr

Fig. 10

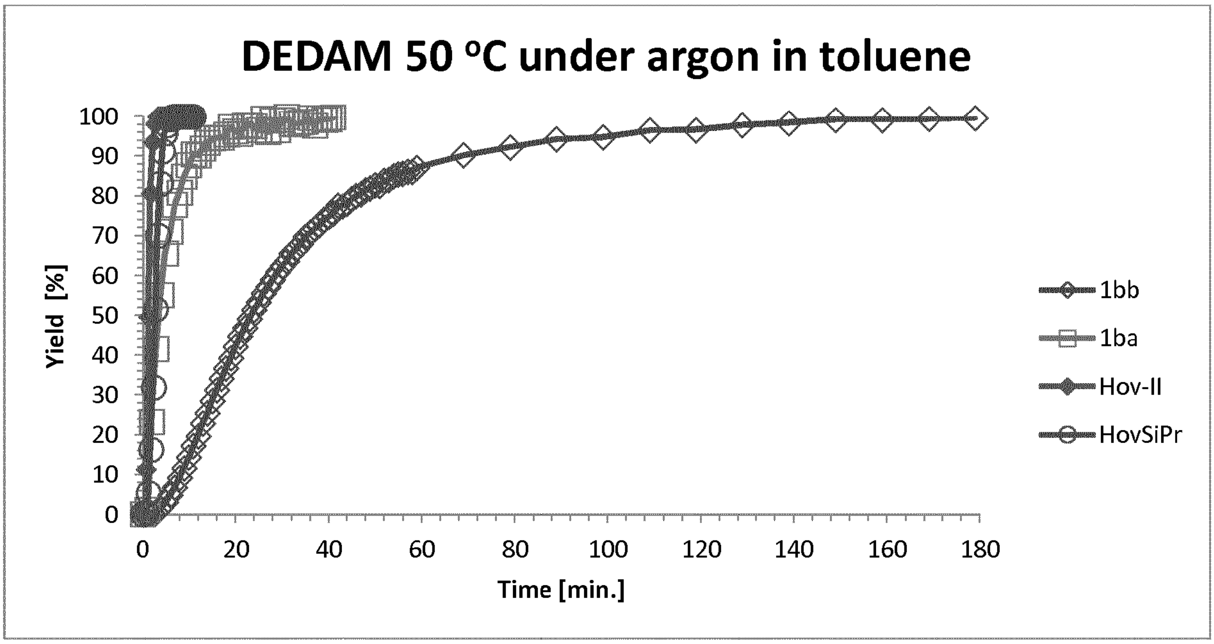
DEDAM 50 °C under argon in toluene
Yield [%]
0
10
20
30
40
50
60
70
80
90
100
0
20
40
60
80
100
120
140
160
180
Time [min.]
1bb
1ba
Hov-II
HovSiPr

Fig. 11

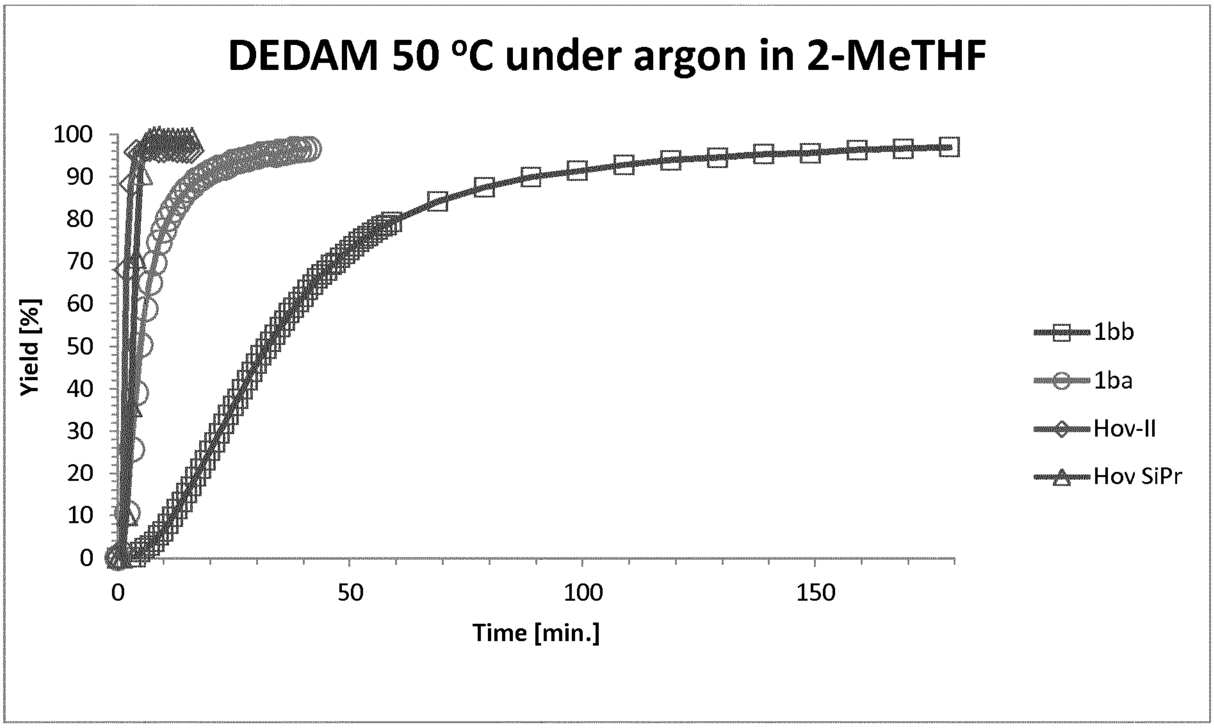
DEDAM 50 °C under argon in 2-MeTHF
Yield [%]
0
10
20
30
40
50
60
70
80
90
100
0
50
100
150
Time [min.]
1bb
1ba
Hov-II
Hov SiPr

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1087838 B1, Herrmann
- US 2013144437 A1


### Non-patent literature cited in the description


- **GRELA, K.** Olefin Metathesis: Theory and Practice. John Wiley & Sons, Inc, 2014, 608
- **GRELA et al.** *Chem. Rev.,* 2009, vol. 109, 3708-3742
- **GRUBBS et al.** *Chem. Rev.,* 2010, vol. 110, 1746-1787
- **LEMCOFF et al.** *Dalton Trans.,* 2012, vol. 41, 32-43
- **A. HOVEYDA.** *J. Org. Chem.,* 2014, vol. 79, 4763-4792
- **NOLAN et al.** *Chem. Commun.,* 2014, vol. 50, 10355-10375
- *Chem. Rev.,* 2010, vol. 110, 1746
- *Chem. Rev.,* 2009, vol. 109, 3708
- *Angew. Chem. Int. Ed.,* 1998, vol. 37, 2490-2493
- *Organometallics,* 2003, vol. 22, 1986-1988
- *Organometallics,* 2007, vol. 26, 1052-1056
- *Organometallics,* 2012, vol. 31, 2476-2481
- *J. Organomet. Chem.,* 2006, vol. 691, 5204-5210
- *Organometallics,* 2012, vol. 31, 580-587
- *Angew. Chem. Int. Ed.,* 2009, vol. 48, 5191-5194
- *Organometallics,* 2009, vol. 29, 250-256
- *Chem. Eur. J.,* 2010, vol. 16, 3983-3993
- *J. Organomet. Chem.,* 2010, vol. 695, 2418-2422
- *Organometallics,* 2010, vol. 29, 2761-2766
- *Organometallics,* 2010, vol. 29, 3007-3011
- *J. Organomet. Chem.,* 2012, vol. 710, 68-74
- *Organometallics,* 2006, vol. 25, 25-28
- *Adv. Synth. Catal.,* 2007, vol. 349, 1692-1700
- *Organometallics,* 2007, vol. 26, 2469-2472
- *Chem. Eur. J.,* 2008, vol. 14, 7545-75561
- *J. Am. Chem. Soc.,* 2011, vol. 133, 8525-8527
- *J. Am. Chem. Soc.,* 2011, vol. 133, 7490-7496
- Metathesis of renewable raw materials-influence of ligands in the indenylidene type catalysts on self-metathesis of methyl oleate and cross-metathesis of methyl oleate with (Z)-2-butene-1,4-diol acetate. *Green Chemistry,* 10 December 2013, vol. 16 (3), ISSN 1463-9262, 1579
- **ANNA KAJETANOWICZ et al.** *Supporting information,* 10 December 2013